(19) Europäisches Patentamt
European Patent Office
Office européen des brevets

(11) Numéro de publication : **0 382 628 B1**

(12) # FASCICULE DE BREVET EUROPEEN

(45) Date de publication du fascicule du brevet : **04.05.94 Bulletin 94/18**

(21) Numéro de dépôt : **90400324.1**

(22) Date de dépôt : **06.02.90**

(51) Int. Cl.$^5$ : **C07D 471/04,** A61K 31/395, A61K 31/47, A61K 31/435, A61K 31/34, A61K 31/38, A61K 31/415, C07D 401/12, C07D 405/12, C07D 217/04, C07D 409/12

(54) **Dérivés aminoalkoxyphényle, leur procédé de préparation ainsi que les compositions en contenant.**

Le dossier contient des informations techniques présentées postérieurement au dépôt de la demande et ne figurant pas dans le présent fascicule.

(30) Priorité : **07.02.89 FR 8901555**

(43) Date de publication de la demande : **16.08.90 Bulletin 90/33**

(45) Mention de la délivrance du brevet : **04.05.94 Bulletin 94/18**

(84) Etats contractants désignés : **AT BE CH DE DK ES FR GB GR IT LI LU NL SE**

(56) Documents cités :
**EP-A- 0 235 111**
**EP-A- 0 287 696**
**US-A- 4 379 167**
**Roche Lexikon d. Medizin, Urban + Schwarzenberg, München, Wien, Baltimore, 2ème ed., 1987, p. 190-191;**

(73) Titulaire : **ELF SANOFI**
**32-34, rue Marbeuf**
**F-75008 Paris (FR)**
(84) **CH DE DK ES FR GB GR IT LI LU NL SE AT**
Titulaire : **S.A. SANOFI PHARMA N.V.**
**Avenue de Béjar 1**
**B-1120 Bruxelles (BE)**
(84) **BE**

(72) Inventeur : **Gubin, Jean**
**Avenue des Citronniers, 24**
**B-1020 Bruxelles 2 (BE)**
Inventeur : **Chatelain, Pierre**
**Avenue du Haras 111**
**B-1150 Bruxelles (BE)**
Inventeur : **Luchetti, Jean**
**Rue des Combattants 23A**
**B-5860 Chastre (BE)**

(74) Mandataire : **Le Guen, Gérard et al**
**CABINET LAVOIX 2, place d'Estienne d'Orves**
**F-75441 Paris Cédex 09 (FR)**

**EP 0 382 628 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention se rapporte, d'une manière générale, à de nouveaux dérivés cycliques et plus particulièrement à de nouveaux dérivés aminoalkoxyphényle ainsi qu'à leur procédé de préparation.

US-4 379 167 décrit des 1-aryloxy-4-amino-2-butanole possédant des propriétés anti-arythmiques et antiadrénergiques β. Par ailleurs, EP-235 111 décrit des dérivés d'indolizine ayant une activité anti-calcique et β-bloquante.

L'invention a pour objet de nouveau aminoalkoxyphényle de formule générale :

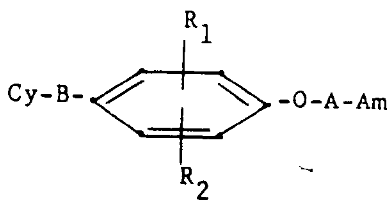

(1)

dans laquelle :

B représente un groupement -S-, -SO- ou $-SO_2-$,

$R_1$ et $R_2$, qui sont identiques ou différents, représentent chacun l'hydrogène, le radical méthyle ou éthyle ou un halogène tel que chlore, brome ou iode,

A représente un radical alkylène, linéaire ou ramifié, ayant de 2 à 5 atomes de carbone ou le radical hydroxy-2 propylène dans lequel l'hydroxy est éventuellement substitué par un radical alkyle inférieur,

Am représente un groupement :

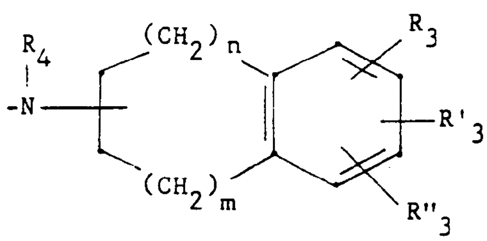

ou

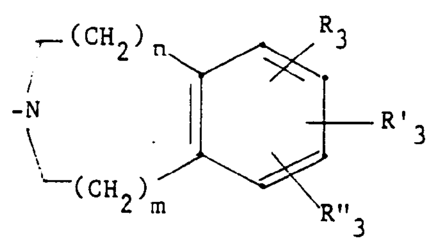

(D)

(E)

dans lequel : $R_3$, $R'_3$ et $R''_3$, qui sont identiques ou différents, représentent chacun l'hydrogène, un atome d'halogène tel que chlore ou brome, un groupement alkyle inférieur ou un groupement alkoxy inférieur,

$R_4$ représente l'hydrogène ou un radical alkyle,

n et m, identiques ou différents, représentent chacun 0, 1, 2 ou 3,

Cy représente un groupement de formule :

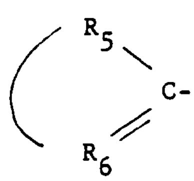

ou

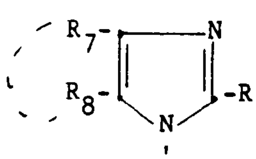

(F)

(G)

R représente l'hydrogène, un radical alkyle, un radical cycloalkyle, un radical benzyle ou un radical

phényle éventuellement substitué par un ou plusieurs substituants, qui peuvent être identiques ou différents, sélectionnés parmi des atomes d'halogène, par exemple fluor, chlore ou brome ou parmi des groupements alkyles inférieurs, alkoxy inférieurs ou nitro,

$R_5$ et $R_6$ sont pris ensemble, avec l'atome de carbone auquel ils sont attachés, pour former :

- un groupement carbocyclique mono- ou dicyclique éventuellement aromatique ayant de 5 à 10 atomes de carbone et éventuellement substitué par un groupement R en position α par rapport au groupement méthyne,
- un groupement hétérocyclique à 5 sommets éventuellement aromatique, les hétéroatomes ou hétérogroupes étant sélectionnés parmi des groupements O, S, N,

$$-\overset{|}{N}-R_9 \;;$$

O et N ; O et

$$-\overset{|}{N}-R_9 \;;$$

S et N ; S et

$$-\overset{|}{N}-R_9 \;;$$

N et N ; N et

$$-\overset{|}{N}-R_9 \;;$$

le groupement hétérocyclique étant éventuellement substitué par un groupement R en position α par rapport au groupement méthyne et éventuellement substitué par un ou deux groupements sélectionnés parmi des groupements alkyles inférieurs et phényle,

- un groupement hétérocyclique ayant de 6 à 10 sommets, mono- ou dicyclique et éventuellement aromatique, les hétéroatomes ou hétérogroupes étant sélectionnés parmi des groupements 0, S, N,

$$-\overset{|}{N}-R_9 \;;$$

O et N ; O et

$$-\overset{|}{N}-R_9 \;;$$

S et N ; S et

$$-\overset{|}{N}-R_9 \;;$$

N et N ; N et

$$-\overset{|}{N}-R_9 \;;$$

le groupement hétérocyclique étant éventuellement substitué par un groupement R en position α par rapport au groupement méthyne,

$R_7$ et $R_6$, qui sont identiques ou différents, représentent chacun l'hydrogène, un radical alkyle inférieur ou un radical phényle ou lorsqu'ils sont pris ensemble, avec les atomes de carbone auxquels ils sont attachés, représentent un carbocycle à 6 sommets éventuellement aromatique,

$R_9$ représente l'hydrogène, un groupement alkyle inférieur, phényle, benzyle ou halogénobenzyle.

Dans le présent contexte, aussi bien dans la description que dans les revendications, les termes ci-dessous comportent les significations suivantes :

"alkyle" désigne les restes d'hydrocarbures, aliphatiques saturés linéaires ou ramifiés, ayant jusqu'à 8 atomes de carbone tels que méthyle, éthyle, n-propyle, isopropyle, n-butyle, isobutyle, tertiobutyle, n-pentyle, néopentyle, n-hexyle, n-heptyle ou n-octyle.

"alkyle inférieur" désigne les restes d'hydrocarbures saturés, linéaires ou ramifiés, ayant jusqu'à 4 atomes de carbone tel que méthyle, éthyle, n-propyle, isopropyle, n-butyle, isobutyle, tertiobutyle ou méthyl-1 propyle,

"alkoxy inférieur" désigne un groupement hydroxy substitué par un groupement alkyle inférieur tel que défini

ci-dessus,
"cycloalkyle" désigne un groupement alicyclique ayant de 3 à 6 atomes de carbone, tels que cyclopropyle ou cyclohexyle.

Ainsi, en tenant compte des significations suivantes :

| | |
|---|---|
| R | représente, en particulier, un radical méthyle, éthyle, n-propyle, isopropyle, n-butyle, isobutyle, tertiobutyle, méthyl-1 propyle, n-pentyle, néopentyle, phényle, monofluoro-, monochloro-, ou monobromophényle, difluoro-, dichloro- ou dibromophényle, monométhyl- ou diméthylphényle, monométhoxy- ou diméthoxyphényle, un radical méthylphényle substitué par un atome d'halogène ou un radical cyclopropyle ou cyclohexyle, |
| $R_3$, $R'_3$ et $R''_3$ | représentent en particulier le radical méthyle ou méthoxy ou un atome de chlore, |
| $R_4$ | représente en particulier un radical méthyle, éthyle, n-propyle, isopropyle, n-butyle, isobutyle, tertiobutyle, n-pentyle, néopentyle, n-hexyle, n-heptyle ou n-octyle, |
| A | représente, en particulier, une chaine éthylène-1,2, propylène-1,3, méthyl-2 propylène-1,3, tétraméthylène-1,4 ou pentaméthylène-1,5, |
| Cy | représente, en particulier, un groupement phényle, cyclohexényle, indényle, naphtyle, dihydronaphtyle, pyridyle, dihydropyridyle, furyle, dihydrofuryle, thiényle, dihydrothiényle, pyrrolyle, dihydropyrrolyle, pyrazolyle, imidazolyle, pyrimidyle, pyrazinyle, pyridazynyle, oxazolyle, isoxazolyle, thiazolyle, benzofuryle, benzothiényle, indolyle, benzimidazolyle, benzoxazolyle, quinoléinyle, benzisoxazolyle, cinnolinyle, quinoxalinyle, quinazolinyle, indolizinyle, thiénopyridyle, tétrahydrothiénopyridyle, pyrrolopyridyle, pyrazolopyridyle, pyrrolopyridazinyle, imidazopyridyle. |

Une classe particulière de composés de formule (1) sont ceux dans laquelle Cy représente un groupement indolizinyle, benzofuryle, benzothiényle, indolyle, oxazolyle, pyrazolyle, phényle , pyrazolo[1,5-a] pyridyle ou imidazo[1,2-a]pyridyle.

Une autre classe de composés de l'invention peut être représentée par la formule (I) dans laquelle $R_1$ et $R_2$ représentent chacun l'hydrogène.

Comme composés particulièrement intéressants de formule (1), on peut également citer ceux dans lesquels $R_3$, $R'_3$ et $R''_3$ représentent hydrogène ou méthoxy.

D'autres composés intéressants de formule (1) sont ceux dans lesquels R représente le groupement isopropyle ou cyclopropyle.

L'invention se rapporte également aux sels pharmaceutiquement acceptables des composes de formule (1) formés à partir d'un acide organique ou inorganique.

Comme exemples de sels organiques de ce genre, on peut citer les oxalate, maléate, fumarate, méthanesulfonate, benzoate, ascorbate, pamoate, succinate, hexamate, bisméthylènesalicylate, éthanedisulfonate, acétate, propionate, tartrate, salicylate, citrate, gluconate, lactate, malate, cinnamate, mandélate, citraconate, aspartate, palmitate, stéarate, itaconate, glycolate, p-aminobenzoate, glutamate, benzènesulfonate et théophylline acétate ainsi que les sels formés à partir d'un acide aminé tel que le sel de lysine ou d'histidine.

Comme exemples de sels inorganiques de ce genre, on peut citer les chlorhydrate, bromhydrate, sulfate, sulfamate, phosphate et nitrate.

Les composés de formule (1) peuvent exister dans certains cas sous la forme d'isomères optiques notamment en raison du carbone asymétrique présent lorsque A représente une chaîne hydroxy-2 propylène.

L'invention se rapporte à la fois à l'ensemble des isomères des composés de formule (1), isomères considérés sous forme dextrogyre ou lévogyre ou, sous forme de mélange, par exemple sous forme de mélange racémique.

On a trouvé que les composés de l'invention possèdent de remarquables propriétés pharmacologiques notamment des propriétés inhibitrices de la translocation calcique ainsi que des propriétés bradycardisantes, hypotensives et antiadrénergiques.

De ce point de vue, les composés préférés de l'invention sont ceux dans lesquels B représente un groupement $-SO_2-$.

Ces propriétés sont capables de rendre les composés en question très utiles dans le traitement de certains syndromes pathologiques du système cardiovasculaire en particulier dans le traitement de l'angine de poitrine, de l'hypertension, de l'arythmie, de l'insuffisance circulatoire cérébrale.

Dans le domaine antitumoral, les composés de l'invention pourront être utiles comme potentialisateurs d'anticancéreux.

Selon la voie d'administration choisie, la posologie journalière pour un être humain pesant 60 kg se situera entre 2 et 50 mg de principe actif.

De même, les composés de l'invention pourront être utilisés seuls ou en association avec un agent antiinflammatoire, pour réduire et/ou contrôler la pression intraoculaire excessive. A cet effet, les composés de l'invention pourront être utilisés pour le traitement d'affections pathologiques occulaires en particulier dans le traitement du glaucome.

Généralement, on administrera, à chaque oeil, de 5ng à 0,5mg de principe actif selon l'invention, la fréquence journalière d'administration dépendant de la gravité de l'affection à traiter.

En conséquence, l'invention se rapporte également à des compositions pharmaceutiques ou vétérinaires contenant, comme principe actif au moins un composé de formule (1) ou un sel pharmaceutiquement acceptable de ce dérivé en association avec un véhicule pharmaceutique ou un excipient approprié.

Les composés de l'invention peuvent être obtenus comme suit :

I. - Les composés de formule I dans laquelle B représente un groupement -S- ou $-SO_2-$ et A représente un radical alkylène peuvent être préparés, selon l'invention, en condensant, en présence d'un accepteur d'acide et dans un solvant polaire tel que le diméthylsulfoxyde ou un alcool par exemple le butanol, une cétone telle que la méthyl-éthyl-cétone, ou un solvant non polaire tel qu'un hydrocarbure aromatique, par exemple le benzène, le toluène ou un xylène, un dérivé alkoxy-4 phényle de formule générale :

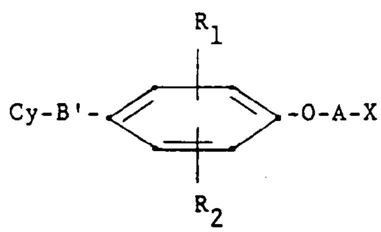

(2)

dans laquelle B' représente un groupement -S- ou $-SO_2-$, Cy, $R_1$ et $R_2$ ont la même signification que précédemment, A représente un radical alkylène tel que défini dans la formule (1) et X représente un atome d'halogène, de préférence le brome, ou un groupement alkylsulfonyloxy ayant de 1 à 4 atomes de carbone tel que par exemple méthanesulfonyloxy ou arylsulfonyloxy ayant de 6 à 10 atomes de carbone tel que benzènesulfonyloxy ou p-toluènesulfonyloxy, avec une amine de formule générale :

H-Am (3)

dans laquelle Am a la même signification que précédemment pour former le dérivé souhaité de formule (1) sous forme de base libre.

Généralement, la condensation en question est effectuée à une température comprise entre la température ambiante et la température de reflux du milieu, l'accepteur d'acide pouvant être par exemple un carbonate ou hydroxyde de métal alcalin ou un excès d'amine de formule (3).

Les composés de formule (2) en question peuvent être obtenus :

a) Lorsque X est un halogène, par condensation d'un dérivé hydroxy-4 phényle de formule générale :

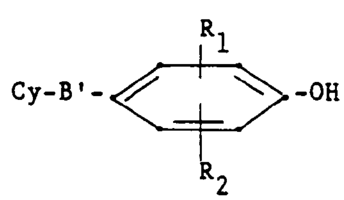

(4)

dans laquelle Cy, B', $R_1$ et $R_2$ ont la même signification que precedemment, avec un dihalogénoalcane de formule générale :

Hal-A-Hal (5)

dans laquelle A représente un radical alkylène tel que défini dans la formule (1) et Hal représente un atome d'halogène de préférence le brome et ce, au reflux, dans un solvant tel que la méthyl-éthyl-cétone ou le N,N-diméthylformamide et en présence d'un agent basique tel qu'un carbonate de métal alcalin par exemple le carbonate de potassium, un hydrure de métal alcalin tel que l'hydrure de sodium, un hydroxyde de métal alcalin par exemple l'hydroxyde de sodium ou de potassium ou un alcoolate de métal alcalin par exemple le méthylate ou l'éthylate de sodium,

b) lorsque X représente un groupement alkylsulfonyloxy ou arylsulfonyloxy, par condensation d'un halogénure de formule générale :

Hal-W

dans laquelle W représente un radical alkylsulfonyle ayant de 1 à 4 atomes de carbone, par exemple mé-

thanesulfonyle ou arylsulfonyle ayant de 6 à 10 atomes de carbone, par exemple benzènesulfonyle ou p-toluènesulfonyle, dans un solvant accepteur d'acide, par exemple la pyridine, avec un dérivé hydroxy alkoxy de formule générale :

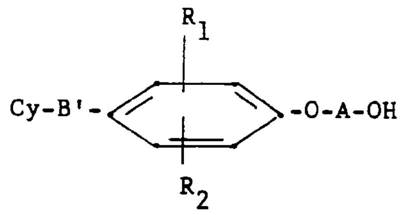

(6)

dans laquelle Cy, B', $R_1$ et $R_2$ ont la même signification que précédemment et A représente un radical alkylène tel que défini dans la formule (1).

Quant aux composés de formule (6), ceux-ci peuvent être préparés en condensant dans un solvant approprié tel que le N,N-diméthylformamide et en présence d'un agent basique tel qu'un carbonate de métal alcalin par exemple le carbonate de potassium, un hydroxyde de métal alcalin tel que l'hydroxyde de sodium ou de potassium, un hydrure de métal alcalin tel que l'hydrure de sodium ou un alcoolate de métal alcalin par exemple le méthylate ou l'éthylate de sodium, un dérivé d'indolizine de formule (4) ci-dessus, avec un alcool halogéné de formule générale :

Hal-A-OH (7)

dans laquelle A représente un radical alkylène tel que défini dans la formule (1) et Hal a la même signification que précédemment.

Les amines de formule (3) sont des composés connus ayant été décrits dans les demandes de brevets européens No. 219.813 et 227.986 ou peuvent être préparées selon les méthodes y décrites.

Certains composés de formule (4) sont des composés connus par exemple ceux dans lesquels Cy représente un groupement benzofuryle ou benzothiényle et B' représente un groupement $-SO_2-$ (brevet US N° 4.117.128) ou dans lesquels Cy représente un groupement indolizinyl-1 (demande EP N° 235.111).

Généralement, les autres composés de formule (4) peuvent être préparés en adaptant au composé désiré la méthode décrite dans le susdit brevet US ou les méthodes décrites ci-dessous.

Dans la plupart des cas, les composés de formule (4) peuvent être obtenus à partir d'un groupement benzènesulfonyle ou phénylthio, ce groupement étant O-protégé en position 4.

On fixe le groupement en question au carbocycle ou hétérocycle approprié en utilisant une réaction de Friedel-Crafts et on déprotège l'oxygène en position 4 du groupement benzènesulfonyle ou phénylthio au moyen de procédés classiques pour régénérer le groupement hydroxyle.

On a repris ci-dessous des exemples de procédés utilisés couramment pour préparer des dérivés de formule (4) :

a) Composés de formule (4) dans laquelle Cy représente un groupement (F)

1) Les composés de formule (4) dans laquelle Cy représente un groupement R-2 indolizinyl-3 peuvent être préparés en faisant réagir un dérivé d'indolizine de formule générale :

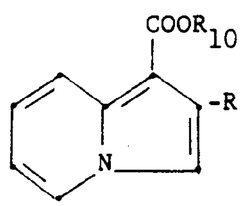

(8)

dans laquelle R a la même signification que précédemment et $R_{10}$ représente un radical alkyle inférieur de préférence éthyle, avec un halogénure de formule générale :

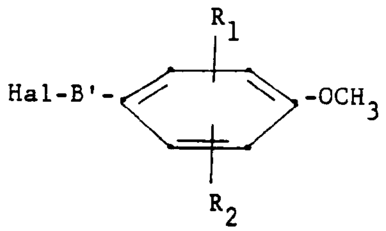

(9)

dans laquelle B', $R_1$, $R_2$ et Hal ont la même signification que précédemment et en présence d'un catalyseur de Friedel-Crafts tel que le chlorure d'aluminium, ce qui fournit un composé de formule générale :

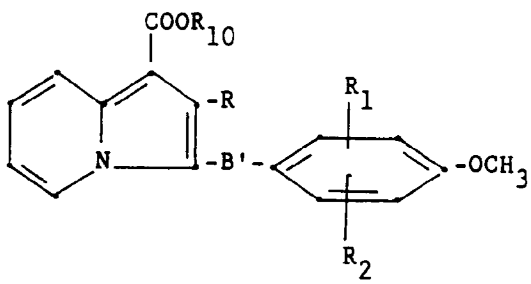

(10)

dans laquelle B', R, $R_1$, $R_2$ et $R_{10}$ ont la même signification que précédemment.

On déméthyle ensuite le composé de formule (10) au moyen d'un mélange éthanethiol/chlorure d'aluminium, pour obtenir le dérivé méthoxy-4 phényle de formule générale :

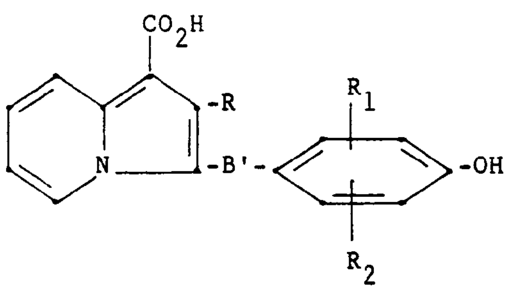

(11)

dans laquelle B', R, $R_1$ et $R_2$ ont la même signification que précédemment, dérivé que l'on chauffe aux environs de 200°C ce qui fournit le composé souhaité de formule (4).

Les composés de formule (8) sont soit des composés connus ayant été publiés dans J. Chem. Soc. 1962 pp. 2627-2629 soit des composés qui peuvent être préparés selon la méthode y décrite.

2) Les composés de formule (4) dans laquelle Cy représente un groupement R-2 imidazo[1,2-a]pyridyl-3 peuvent être obtenus en faisant réagir une R-2 imidazo[1,2-a] pyridine avec un halogénure de formule (9), en présence d'un catalyseur de Friedel-Crafts tel que le chlorure d'aluminium, ce qui fournit un composé de formule générale :

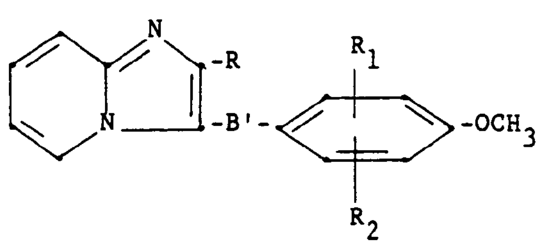

(12)

dans laquelle B', R, $R_1$ et $R_2$ ont la même signification que précédemment.

On déméthyle ensuite le composé de formule (12) en utilisant un agent approprié par exemple l'acide

bromhydrique ou un mélange éthanethiol/chlorure d'aluminium pour donner le composé désiré de formule (4).

Certaines aryl-2 imidazo[1,2-a] pyridines sont connues d'après J. Med. Chem. 8, p. 305 (1965). Les autres R-2 imidazo[1,2-a] pyridines peuvent être obtenues selon la méthode décrite dans la susdite référence ou en utilisant des procédés classiques.

D'une autre manière, les composés de formule (12) peuvent être obtenus à partir d'une R-2 halo-3 imidazo[1,2-a] pyridine et d'un sel de métal alcalin d'un dérivé méthoxy-4 de formule (15).

3) Les composés de formule (4) dans laquelle Cy représente un groupement pyridyle ou R-3 pyridyle-4 peuvent être obtenus en déméthylant avec un agent approprié tel que l'acide bromhydrique aqueux, un dérivé méthoxy-4 phényle de formule générale :

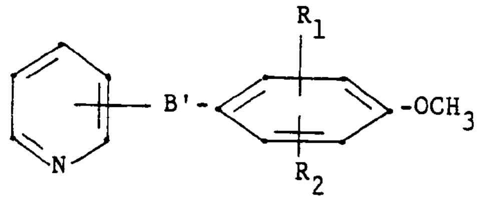

ou

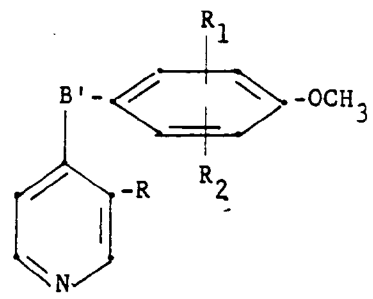

(13) (13')

dans laquelle B', $R_1$ et $R_2$ ont la même signification que précédemment et R a la même valeur que ci-dessus à l'exception d'hydrogène, pour fournir les composés désirés de formule (4).

Les composés de formule (13) et (13') dans lesquels B' représente un groupement $-SO_2-$, peuvent être préparés en oxydant un dérivé sulfure de formule générale :

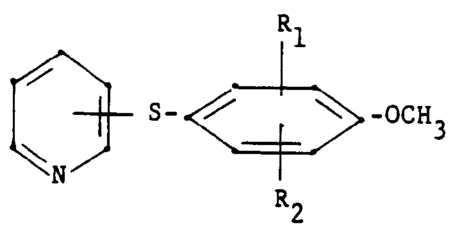

ou

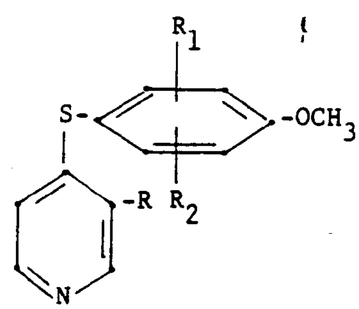

(14) (14')

dans laquelle $R_1$ et $R_2$ ont la même signification que précédemment et R a la même signification que dans la formule (13) ou (13').

Certains composés de formule (14) sont des composés connus ayant été décrits dans le brevet U.S. No. 4.128.552. Les autres composés de formule (14) peuvent être obtenus, selon la méthode décrite dans le susdit brevet U.S. Les composés de formule (14') quant à eux peuvent être préparés à partir de R-3 pyridine dans laquelle R est autre qu'hydrogène, par oxydation avec le peroxyde d'hydrogène dans l'acide acétique, ce qui fournit le dérivé R-3 pyridine-N-oxyde correspondant que l'on fait réagir avec un mélange acide nitrique/acide sulfurique pour donner le dérivé R-3 nitro-4 pyridine-N-oxyde correspondant.

On fait alors réagir ce dérivé nitro d'abord avec le bromure d'acétyle ensuite avec la poudre de fer dans l'acide acétique pour donner le dérivé R-3 bromo-4 pyridine correspondant qui lorsqu'on le traite avec un dérivé thiophénol de formule générale :

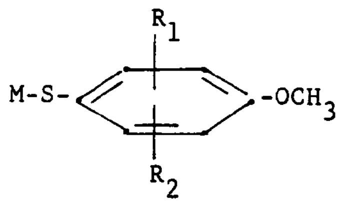

(15)

dans laquelle $R_1$ et $R_2$ ont la même signification que précédemment et M représente un atome de métal alcalin tel que sodium, fournit le dérivé désiré de formule (14').

4) Les composés de formule (4) dans laquelle Cy représente un groupement R-2 quinoléinyl-3 peuvent être préparés en faisant réagir une α-halocétone de formule générale :

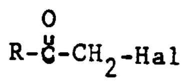

(16)

dans laquelle R et Hal ont la même signification que précédemment, avec un dérivé métallique de formule générale :

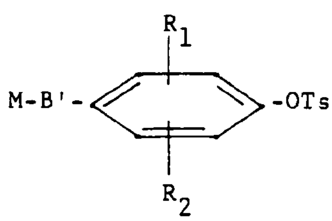

(17)

dans laquelle M, B', $R_1$ et $R_2$ ont la même signification que précédemment et Ts représente un groupement p-toluènesulfonyle, ce qui fournit une cétone de formule générale :

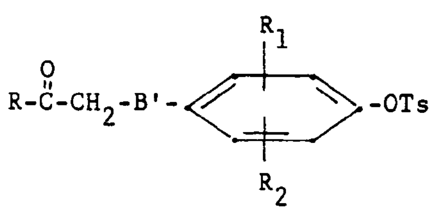

(18)

dans laquelle B', R, $R_1$, $R_2$ et Ts ont la même signification que précédemment.

Cette cétone de formule (18), lorsqu'on la traite avec l'amino-2 benzaldéhyde [Helv. Chem. Act. vol. XVIII, p. 1235 (1935)] fournit le dérivé méthoxy-4 phényle de formule générale :

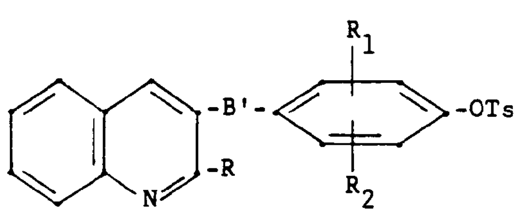

(19)

dans laquelle B', R, $R_1$, $R_2$ et Ts ont la même signification que précédemment, dérivé que l'on hydrolyse, par la suite, en milieu basique, pér exemple dans un hydroxyde de métal alcalin aqueux, pour donner le composé désiré de formule (4).

5) Les composés de formule (4) dans laquelle Cy représente un groupement R-3 cinnolinyl-4 ou R-4 cinnolinyl-3 peuvent être préparés en faisant réagir une R-3 halogéno-4 cinnoline (J. Chem. Soc. 1953, p.

609), avec un dérivé thiophénol de formule générale :

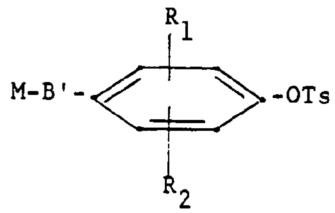

(20)

dans laquelle M, $R_1$, $R_2$ et Ts ont la même signification que précédemment et B' représente un groupement -S-, ce qui fournit le dérivé tosyloxy-4 phényle de formule générale :

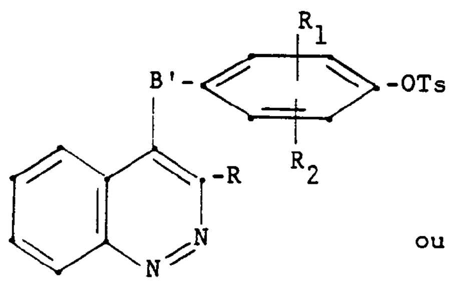

(21)

ou

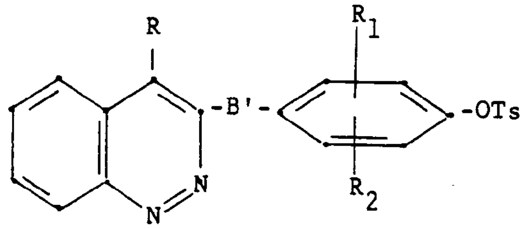

(21')

dans laquelle R, $R_1$, $R_2$ et Ts ont la même signification que précédemment et B' représente un groupement -S-.

On hydrolyse alors le dérivé tosyloxy-4 phényle de formule (21) ou (21') en milieu basique par exemple en milieu hydroxyde de métal alcalin aqueux pour donner le composé désiré de formule (4) dans laquelle B' représente un groupement -S-.

On peut également utiliser des composés de formule (20) dans laquelle -OTs est remplacé par $-OCH_3$. Dans ce cas, on déméthyle le composé correspondant de formule (21) ou (21') au moyen, par exemple d'acide bromhydrique.

Le sulfure de formule (21) ou (21') lorsqu'il est oxydé avec un agent approprié tel que le peroxyde d'hydrogène dans l'acide acétique ou le permanganate de potassium, produit le composé de formule (21) ou (21') dans laquelle B' représente un groupement $-SO_2-$, ce composé après hydrogénation sur catalyseur tel que le charbon palladié ou le noir de platine produisant les composés désirés de formule (4) dans laquelle B' représente un groupement $-SO_2-$.

D'une autre manière, les composés de formule (4) en question dans laquelle B' représente un groupement $-SO_2-$ peuvent être obtenus à partir d'une R-3 halogéno-4 cinnoline ou une R-4 halogéno-3 cinnoline en faisant réagir ce composé avec un dérivé benzènesulfonyle de formule (20) dans laquelle B' représente un groupement $-SO_2-$ pour obtenir un composé de formule (21) ou (21') dans laquelle B' représente un groupement $-SO_2-$ que l'on détosyle tel que décrit précédemment, ce qui fournit le composé désiré de formule (4).

6) Les composés de formule (4) dans laquelle Cy représente un groupement R-6 pyrrolo[1,2-b] pyridazinyl-5 peuvent être préparés en faisant réagir une halogénométhyl-3 pyridazine avec un dérivé métallique de formule (17) pour obtenir un dérivé de pyridazine de formule générale :

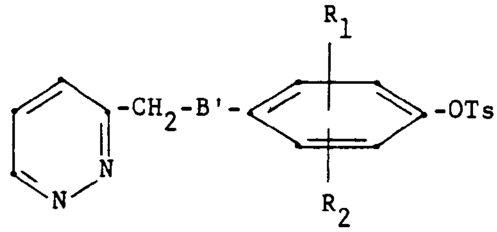

(22)

dans laquelle B', $R_1$, $R_2$ et Ts ont la même signification que precedemment, dérivé que l'on fait réagir, par la suite, avec une α-halocétone de formule (16) en présence d'une base non nucléophile telle que le diaza-1,8 bicyclo[5,4,0] undécène-7, ce qui fournit le dérivé de pyrrolo[1,2-b] pyridazine de formule générale :

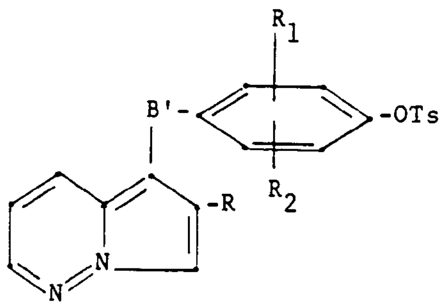

(23)

dans laquelle B', R, $R_1$, $R_2$ et Ts ont la même signification que précédemment.

On hydrolyse alors le dérivé tosyle de formule (23) en milieu basique, par exemple en milieu hydroxyde de métal alcalin aqueux, pour donner le composé désiré de formule (4).

La chlorométhyl-3 pyridazine est un composé connu ayant été publié dans Khim. Geterot. Sikl. Soedin. 3, pp 412-414 (1970).

7) Les composés de formule (4) dans laquelle Cy représente un groupement R-2 pyrazolo[1,5-a] pyridyl-1 peuvent être préparés selon la méthode décrite dans la demande de brevet européen No. 121.197, en traitant une R-2 pyrazolo[1,5-a]pyridine avec un halogénure de formule (9) en présence d'un catalyseur de Friedel-Crafts tel que par exemple le chlorure d'aluminium ce qui fournit le dérivé méthoxy-4 phényle de formule générale :

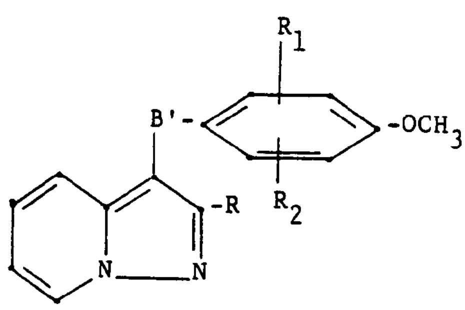

(24)

dans laquelle B', R, $R_1$ et $R_2$ ont la même signification que précédemment.

On déméthyle ensuite le dérivé depyrazolopyridine de formule (24) en utilisant par exemple le chlorhydrate de pyridine à 200-220°C pour donner le composé désiré de formule (4).

8) Les composés de formule (4) dans laquelle Cy représente un groupement phényle peuvent être préparés en faisant réagir le benzène avec un halogénure de formule (9) en présence d'un catalyseur de Friedel-Crafts tel que le chlorure d'aluminium, pour donner le composé désiré de formule (4).

9) Les composés de formule (4) dans laquelle Cy représente un groupement R-2 phényle ou R-1 naphtyl-2 peuvent être préparés en traitant un halogénure de formule générale :

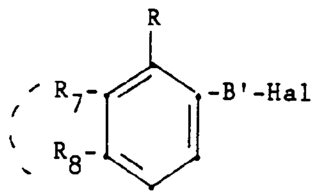

(25)

dans laquelle B', R et Hal ont la même signification que précédemment et $R_7$ et $R_8$ représentent chacun l'hydrogène ou sont pris avec les atomes de carbone auxquels ils sont attachés pour former un groupement phényle, avec un dérivé méthoxyphényle de formule générale :

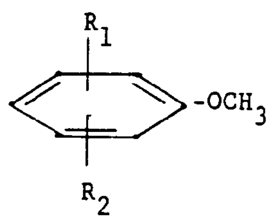

(26)

dans laquelle $R_1$ et $R_2$ ont la même signification que précédemment, en présence d'un catalyseur de Friedel-Crafts tel que le chlorure d'aluminium, pour donner les composés de formule générale :

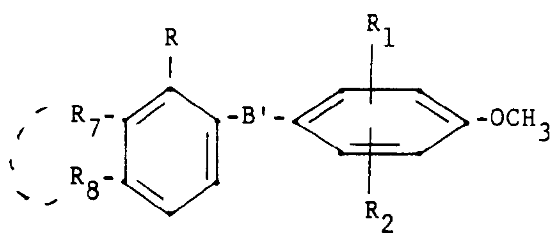

(27)

dans laquelle B', R, $R_1$ et $R_2$ ont la même signification que précédemment et $R_7$ et $R_8$ ont la même signification que dans la formule (25).

On déméthyle alors les composés de formule (27) en utilisant par exemple l'acide iodhydrique aqueux, ce qui fournit le composé désiré de formule (4).

Certains composés de formule (25) sont des composés connus ayant été décrits dans C.A. 81, 63285g, ou peuvent être obtenus selon des procédés connus.

D'une autre manière, on peut préparer les composés de formule (27) dans laquelle $R_7$ et $R_8$ représentent chacun l'hydrogène et B' représente un groupement $-SO_2-$ en traitant le dérivé de métal alcalin d'un R-2 benzènesulfonate, avec un dérivé phényle de formule (26) en présence d'acide méthanesulfonique/pentoxyde de phosphore, selon la méthode décrite dans Communications, Avril 1984, p. 323.

Selon un autre procédé, les composés de formule (4) dans laquelle Cy représente un groupement naphtyl-2 et B' représente un groupement $-SO_2-$ peuvent être obtenus en faisant réagir un halogénosulfonyl-2 naphtalène avec un dérivé $R_1R_2$ phénol.

On réarrange alors ce dérivé sulfonate en présence de chlorure d'aluminium pour obtenir un complexe que l'on traite par un acide tel que l'acide chlorhydrique pour obtenir le composé désiré de formule (4).

10) Les composés de formule (4) dans laquelle Cy représente un groupement R-2 dihydro-4,5 furannyl-3 éventuellement mono- ou di-substitué peuvent être préparés en chauffant un dérivé cétonique de formule (18) avec un dihalogéno-1,2 éthane de formule générale :

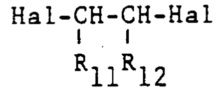

(28)

dans laquelle $R_{11}$ et $R_{12}$, qui sont identiques ou différents, représentent chacun l'hydrogène, un radical alkyle inférieur ou un radical phényle, en présence d'un agent basique tel qu'un carbonate de métal alcalin, pour obtenir un dérivé de cyclopropane de formule générale :

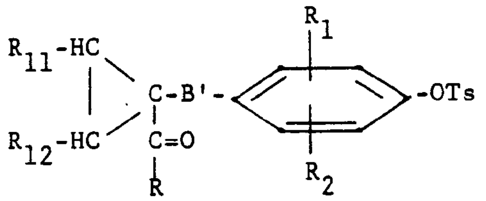

(29)

dans laquelle B', R, $R_1$, $R_2$, $R_{11}$, $R_{12}$ et Ts ont la même signification que précédemment.

On chauffe ensuite entre 100 et 130°C le dérivé de cyclopropane de formule (29) en présence d'un catalyseur de transfert de phase tel que par exemple la triphénylphosphine ou le chlorure de tricaprylylméthyl ammonium, ce qui fournit le dérivé tosyloxy-4 phényle de formule générale :

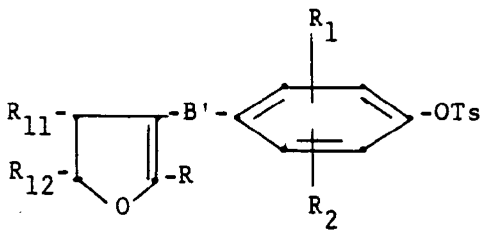

(30)

dans laquelle B', R, $R_1$, $R_2$, $R_{11}$ , $R_{12}$ et Ts ont la même signification que précédemment, dérivé tosyloxy-4 phényle que l'on détosyle par traitement au moyen d'un agent basique tel qu'un hydroxyde de métal alcalin, pour donner le composé désiré de formule (4).

11) Les composés de formule (4) dans laquelle Cy représente un groupement R-2 furannyl-3 mono- ou di-substitué peuvent être obtenus en oxydant par exemple avec l'oxyde de manganèse, un dérivé dihydro-4,5 furanne de formule (30) pour former un dérivé furanne de formule générale :

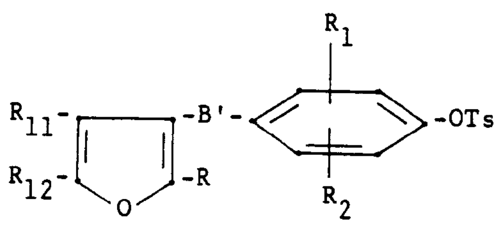

(31)

dans laquelle B', R, $R_1$, $R_2$, $R_{11}$, $R_{12}$ et Ts ont la même signification que précédemment, dérivé de furanne que l'on traite, par la suite, avec un agent basique tel qu'un hydroxyde de métal alcalin, pour obtenir le composé désiré de formule (4).

12) Les composés de formule (4) dans laquelle Cy représente un groupement R-2 furannyl-3 ou R-2 thiényl-3 ou R-2 pyrrolyl-3 peuvent être préparés en faisant réagir un composé de formule générale :

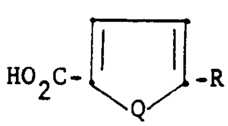

(32)

dans laquelle R a la même signification que précédemment et Q représente -O-, -S- ou

$$-\overset{|}{N}-R_9,$$

avec un halogénure de formule (9) et en présence d'un catalyseur de Friedel-Crafts tel que le chlorure

d'aluminium pour obtenir un dérivé méthoxy-4 de formule générale :

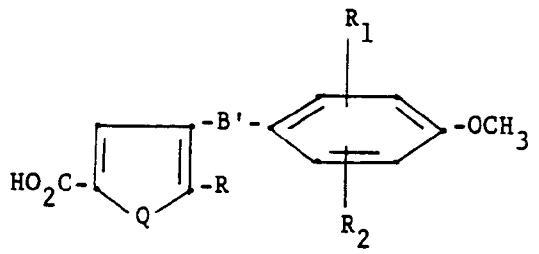

(33)

dans laquelle B', R, $R_2$, $R_2$ et Q ont la même signification que précédemment, que l'on décarboxyle par la suite par chauffage puis déméthyle avec un agent approprié tel que le chlorhydrate de pyridine ou l'acide bromhydrique, pour fournir le composé désiré de formule (4). D'une autre manière, les composés de formule (4) dans laquelle Cy représente un groupement R-2 furannyl-3 éventuellement substitué peuvent être préparés par oxydation, par exemple au moyen d'oxyde de manganèse, d'un dérivé sulfure de formule (30) pour obtenir un dérivé R-2 (tosyloxy-4 benzènesulfonyl)-3 furanne éventuellement substitué que l'on traite ensuite par un milieu basique par exemple un hydroxyde de métal alcalin, pour obtenir le composé désiré de formule (4).

13) Les composés de formule (4) dans laquelle Cy représente un groupement R-1 imidazolyl-2 ou R-1 benzimidazolyl-2 peuvent être obtenus en faisant réagir un R-1 imidazole ou R-1 benzimidazole avec un halogénure de formule (9) en présence d'un catalyseur de Friedel-Crafts tel que le chlorure d'aluminium pour former un composé de formule générale :

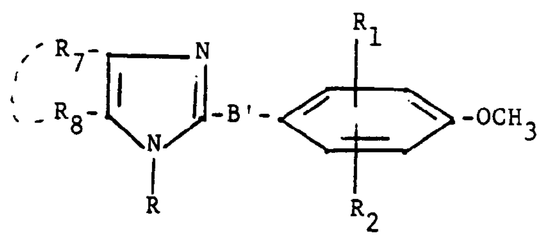

(34)

dans laquelle B', R, $R_1$ et $R_2$ ont la même signification que précédemment, $R_7$ et $R_8$ représentent chacun l'hydrogène ou sont pris ensemble avec les atomes de carbone auxquels ils sont attachés pour former un groupement phényle, composé que l'on déméthyle ensuite en utilisant un mélange éthanethiol/chlorure d'aluminium en présence d'hydrure de sodium pour obtenir le composé désiré de formule (4).

On peut également utiliser des composés de formule (34) dans laquelle le groupement $-OCH_3$ est remplacé par un groupement -O Benzyle. Dans ce cas, on débenzyle les composés de formule (34) en question en utilisant l'hydrogène et un catalyseur approprié par exemple le charbon palladié pour former le composé désiré de formule (4).

Lorsque R représente l'hydrogène, l'imidazole ou le benzimidazole est protégé en position 1 avec un groupement N-protecteur approprié par exemple un groupement benzyle que l'on élimine par la suite, si nécessaire, en utilisant des procédés classiques.

14) Les composés de formule (4) dans laquelle Cy représente un dérivé R-5 isoxazolyl-4 éventuellement substitué peuvent être préparés en faisant réagir un dérivé isoxazole de formule générale :

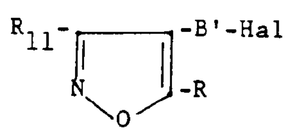

(35)

dans laquelle B', R, $R_{11}$ et Hal ont la même signification que précédemment, avec un dérivé méthoxy-4 de formule (26) en présence d'un catalyseur de Friedel-Crafts tel que le chlorure d'aluminium pour obtenir les composés de formule générale :

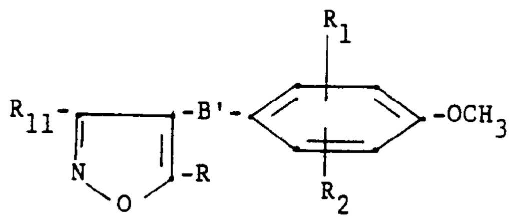 :

(36)

dans laquelle B', R, $R_1$, $R_2$ et $R_{11}$ ont la même signification que précédemment, composés que l'on déméthyle en utilisant par exemple le chlorure d'aluminium, pour former le composé désiré de formule (4).

Certains composés de formule (35) sont des composés connus ayant été décrits dans Gazz. Chim. Ital. 76, 30 (1946) tandis que les autres composés de formule (35) peuvent être préparés selon la méthode y décrite ou selon des méthodes classiques.

D'une autre manière, les composés de formule (36) dans laquelle $R_{11}$ représente l'hydrogène et B' représente un groupement $-SO_2-$ peuvent être obtenus selon la méthode décrite dans J. Hetero. Chem. 23, 1363 (1986) en faisant réagir un (méthoxy-4 benzènesulfonyl)-1 N,N-diméthylamino-2 éthène avec l'hydroxylamine.

De même, on peut utiliser des composés de formule (36) dans laquelle B' représente un groupement $-SO_2-$, $R_{11}$ est différent de l'hydrogène et dans laquelle $-OCH_3$ est remplacé par -O Tosyle, pour obtenir les composés correspondants de formule (4). Ces dérivés R-5 (O-Tosyl-4)-4 benzènesulfonyl-isoxazole substitués en position 3 peuvent être préparés selon la méthode décrite dans Gazz. Chim. Ital. 98, 656 (1968) c'est-à-dire en faisant réagir une benzènesulfonyl-cétone et un dérivé d'acide hydroxamique.

15) Les composés de formule (4) dans laquelle Cy représente un groupement R-5 pyrazolyl-4 peuvent être préparés en faisant réagir un composé de formule générale :

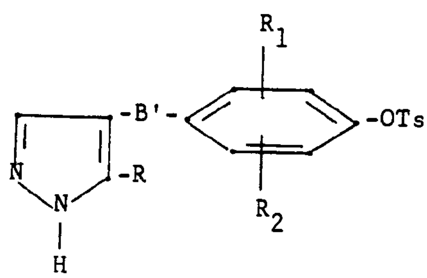

(37)

:

dans laquelle B', R, $R_1$, $R_2$ et Ts ont la même signification que précédemment, avec l'hydrazine, pour obtenir le composé désiré de formule (4).

Les composés de formule (37) peuvent être obtenus selon la méthode décrite dans J. Hetero. Chem. 23, 1963 (1986) c'est-à-dire à partir d'un dérivé N,N-diméthylaminoéthène et d'hydrazine.

D'une autre manière, les composés de formule (4) dans laquelle Cy représente un groupement R-5 pyrazolyl-4 peuvent être obtenus directement à partir d'un composé de formule générale :

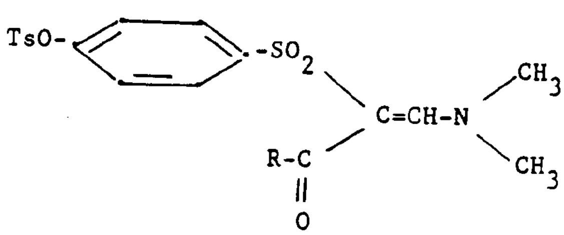

(38)

dans laquelle R et Ts ont la même signification que précédemment, et d'hydrazine en excès.

Les composés de formule (38) peuvent être préparés selon la méthode décrite dans J. Hetero. Chem. 23, 1363 (1986) citée précédemment.

16) Les composés de formule (4) dans laquelle Cy représente un dérivé $R_9$-1 R-2 indolyl-3 ou $R_9$-1 R-3 indolyl-2 peuvent être préparés :

a) lorsque $R_9$ représente l'hydrogène, en faisant réagir le p-méthoxythiophénol substitué par des grou-

pements $R_1$ et $R_2$, avec un R-2 indole ou R-3 indole en présence d'iode, pour obtenir un dérivé d'indole de formule générale :

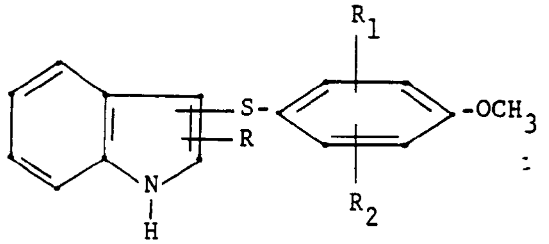

(39)

dans laquelle R, $R_1$ et $R_2$ ont la même signification que précédemment, dérivé d'indole que l'on peut alors oxyder avec l'acide chloro-3 perbenzoïque, pour former des dérivés sulfonyles de formule générale :

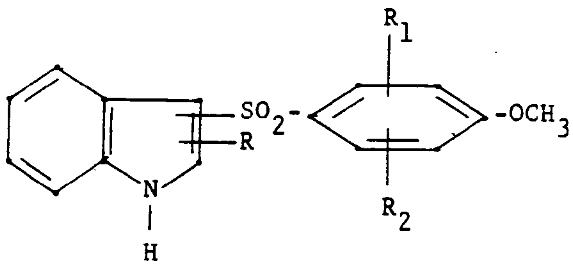

(40)

dans laquelle R, $R_1$ et $R_2$ ont la même signification que précédemment.

On peut alors déméthyler les composés de formule (39) et (40) en utilisant le mercapto-2 éthanol en présence d'hydrure de sodium, ce qui fournit les composés désirés de formule (4).

b) lorsque $R_9$ est différent d'hydrogène, en traitant un composé de formule (39) ou (40) avec un iodure de formule $R_9$-I dans laquelle $R_9$ est différent d'hydrogène et en déméthylant le dérivé substitué en position 1 ainsi obtenu, au moyen de mercapto-2 éthanol en présence d'hydrure de sodium, pour obtenir les composés souhaités de formule (4).

17) Les composés de formule (4) dans laquelle Cy représente un groupement R-2 $R_9$-5 tétrahydro-4,5,6,7 thiéno[3,2-c]pyridyl3 et B' représente un groupement $-SO_2-$ peuvent être obtenus en faisant réagir une R-2 $R_9$-5 tétrahydro-4,5,6,7 thiéno[3,2-c]pyridine dans laquelle $R_9$ est autre qu'hydrogène, avec un composé de formule générale :

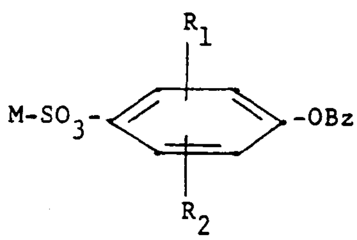

(41)

dans laquelle $R_1$, $R_2$, M et Bz ont la même signification que précédemment, en présence d'acide méthanesulfonique/pentoxyde de phosphore, pour former une tétrahydrothiénopyridine de formule générale :

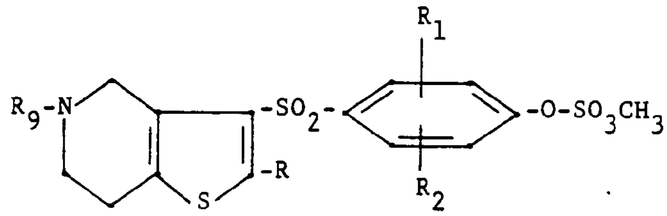

(42)

dans laquelle R, $R_1$ et $R_2$ ont la même signification que précédemment et $R_9$ a la même signification que précédemment a l'exception d'hydrogène.

On hydrolyse alors les composés de formule (42) en présence d'un agent basique tel qu'un hydroxyde de métal alcalin pour obtenir les composés désirés de formule (4) dans laquelle $R_9$ est autre qu'hydrogène.

Les R-2 $R_9$-5 tétrahydro-4,5,6,7 thiéno[3,2-c]pyridine de départ sont des composés connus ayant été décrits dans Heterocycles, 22, 1235 (1984) ou peuvent être préparés selon la méthode y décrite.

18) Les composés de formule (4) dans laquelle Cy représente un groupement R-2 thiéno[3,2-c]pyridyl-3 peuvent être préparés en hydrolysant un composé de formule (42) dans laquelle $R_9$ représente un radical benzyle ou halogénobenzyle puis en faisant réagir le dérivé hydroxy-4 benzènesulfonyle ainsi obtenu avec le charbon palladié dans le diphényléther pour obtenir le composé désiré de formule (4).

19) Les composés de formule (4) dans laquelle Cy représente un groupement R-5 thiazolyl-4 peuvent être préparés en déméthylant un composé de formule générale :

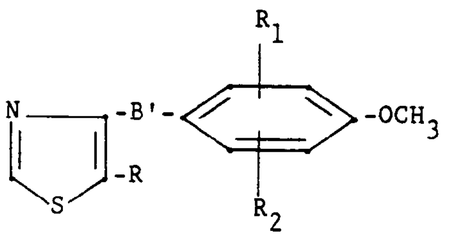

:

(43)

dans laquelle B', R, $R_1$ et $R_2$ ont la même signification que precedemment, en utilisant l'acide bromhydrique dans l'acide acétique, ce qui fournit les composés souhaités de formule (4).

Les composés de formule (43) peuvent être préparés selon la méthode décrite dans Tetrah. Lett. 1972, p. 2777, c'est-à-dire à partir d'un dérivé sulfonylméthylisonitrile et d'un dérivé d'acide thioglycolique.

20) Les composés de formule (4) dans laquelle Cy représente un groupement $R_9$-1 R-5 imidazolyl-4 peuvent être obtenus en déméthylant au moyen de mercapto-2 éthanol en présence d'hydrure de sodium, un composé de formule générale :

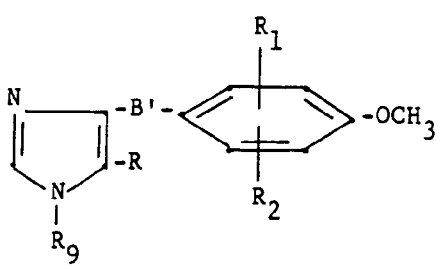

(44)

dans laquelle B', R, $R_1$, $R_2$ et $R_9$ ont la même signification que précédemment, ce qui fournit les composés désirés de formule (4).

Les composés de formule (44) peuvent être obtenus selon la méthode décrite dans Tetrahedron Lett. 23, pp. 2373-2374 (1972) c'est-à-dire à partir de sulfonylméthylisonitrile et d'un dérivé d'imidazole.

21) Les composés de formule (4) dans laquelle Cy représente un dérivé R-5 oxazolyl-4 éventuellement substitué peuvent être préparés en traitant un dérivé de benzènesulfonylméthyl formamide de formule générale :

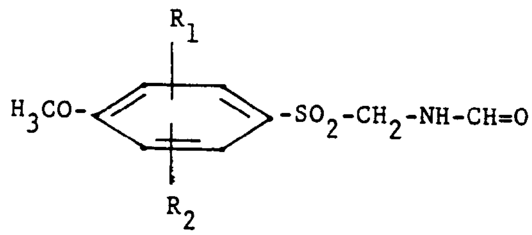

(45)

dans laquelle $R_1$ et $R_2$ ont la même signification que précédemment, avec l'oxychlorure de phosphore en présence d'un accepteur d'acide telle que la triéthylamine, pour obtenir un isonitrile de formule générale :

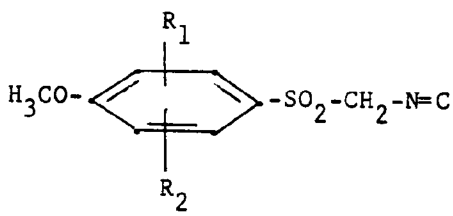

(46)

dans laquelle $R_1$ et $R_2$ ont la même signification que précédemment.

On fait alors réagir cet isonitrile avec un halogénure d'acyle de formule générale :

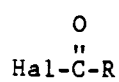

(47)

dans laquelle Hal et R ont la même signification que précédemment, ce qui fournit le dérivé d'isoxazole de formule générale :

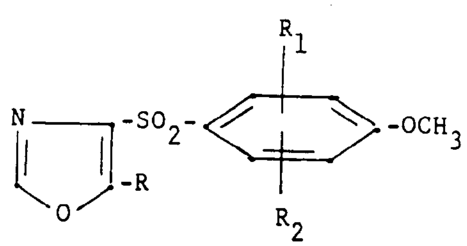

(48)

dans laquelle R, $R_1$ et $R_2$ ont la même signification que précédemment, dérivé que l'on déméthyle au reflux en présence de chlorure d'aluminium, ce qui fournit le composé désiré de formule (4).

22) Les composés de formule (4) dans laquelle B' représente un groupement $-SO_2-$ et Cy représente un groupement de formule (F) dans laquelle $R_5$ et $R_6$ sont pris ensemble avec l'atome de carbone auquel ils sont attachés pour former un groupement carbocyclique non aromatique mono- ou di-cyclique ayant de 5 à 10 atomes de carbone et éventuellement substitué par un groupement R en position $\alpha$ par rapport au groupement méthyne, par exemple un groupement R-3 indényl-2, R-2 cyclohexényl-1 ou R-1 dihydro-3,4 naphtyl-2, peuvent être préparés, selon la méthode décrite dans J. Org. Chem. vol. 35, No. 12, pp. 4217-4222 (1970), en chauffant un composé de formule générale :

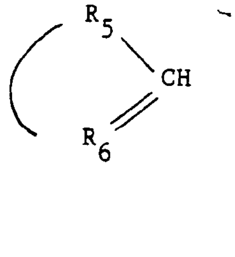

(49)

dans laquelle $R_5$ et $R_6$ sont pris ensemble avec l'atome de carbone auquel ils sont attachés pour former un groupement ayant de 5 à 10 atomes de carbone et éventuellement substitué par un groupement R en position α par rapport au groupement méthyne, avec un halogénure de tosyloxy-4 benzène substitué par des groupements $R_1$ et $R_2$, dans un solvant approprié tel que le benzène et en présence de chlorure cuivrique anhydre et de triéthylamine, pour obtenir un dérivé tosyloxy-4 phényle de formule générale :

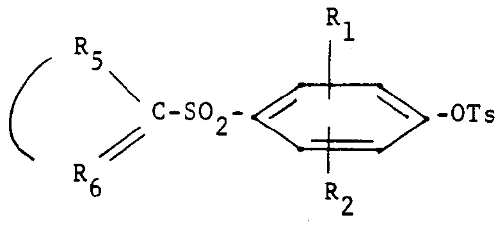

(50)

dans laquelle $R_1$, $R_2$ et Ts ont la même signification que précédemment et $R_5$ et $R_6$ ont la même signification que dans la formule (37), dérivé que l'on détosyle, par la suite, avec un agent approprié tel qu'un hydroxyde de métal alcalin, pour obtenir le composé désiré de formule (4).

Composés de formule (4) dans laquelle Cy représente un groupement (G)

Les composés de formule (4) dans laquelle Cy représente un groupement R-2 imidazolyl-1 ou R-2 benzimidazolyl-1 peuvent être obtenus en faisant réagir un R-2 imidazole ou un R-2 benzimidazole, avec un halogénure de formule (9) en présence d'un catalyseur de Friedel-Crafts tel que le chlorure d'aluminium, pour obtenir un composé de formule générale :

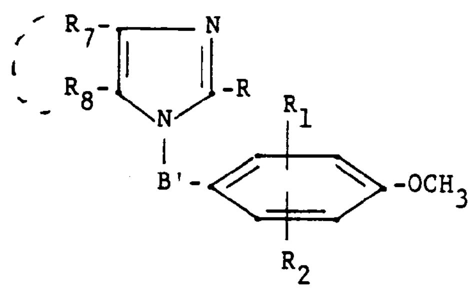

(51)

dans laquelle B', R, $R_1$ et $R_2$ ont la même signification que précédemment, que l'on déméthyle éventuellement en utilisant par exemple l'acide bromhydrique ou le chlorhydrate de pyridine, pour former le composé désiré de formule (4).

Selon une autre méthode, on peut également obtenir les composés de formule (1) dans laquelle B représente un groupement -S- ou $-SO_2-$ et A représente un radical alkylène, de préférence ceux dans lesquels A représente un radical propylène, en faisant réagir, en présence d'un agent basique tel qu'un carbonate de métal alcalin, par exemple le carbonate de potassium, un hydroxyde de métal alcalin tel que l'hydroxyde de sodium ou de potassium, un hydrure de métal alcalin tel que l'hydrure de sodium ou un alcoolate de métal alcalin, par exemple le méthylate ou l'éthylate de sodium, un dérivé hydroxy-4 phényle de formule (4) ci-dessus avec un composé de formule générale :

X-A-Am (52)

dans laquelle X a la même signification que précédemment et représente de préférence le chlore ou un radical benzènesulfonyloxy ou p-toluènesulfonyloxy, A représente un radical alkylène et Am a la même signification que précédemment, la réaction ayant lieu à une température comprise entre la température ambiante et la température de reflux du milieu ainsi que dans un solvant polaire tel que la méthyl éthyl cétone ou le diméthylsulfoxyde pour former le dérivé aminoalkoxyphényle désiré de formule (1) sous forme de base libre.

Lorsque $R_4$ représente l'hydrogène, l'atome d'azote est de préférence protégé par un groupement labile par exemple un groupement protecteur que l'on peut éliminer en milieu basique par exemple le groupement tertiobutoxycarbonyle (BOC).

Les composés de formule (52) sont des composés connus ou qui peuvent être obtenus selon des procédés connus.

Les composés de formule (1) dans laquelle Cy représente un groupement (G), A représente une chaîne alkylène et B représente un groupement -S- ou $-SO_2-$ peuvent également être préparés en faisant réagir un R-2 imidazole ou R-2 benzimidazole, avec un halogénure de formule générale :

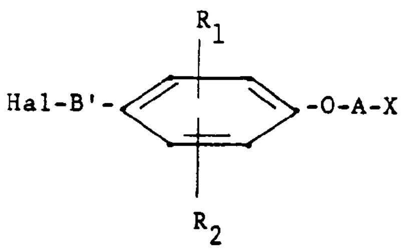

(53)

dans laquelle B', $R_1$, $R_2$, Hal et X ont la même signification que précédemment et A représente une chaîne alkylène, en présence d'un accepteur d'acide telle que la triéthylamine, pour obtenir un composé de formule générale :

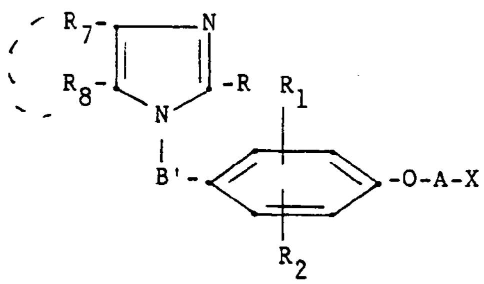

(54)

dans laquelle B', R, $R_1$, $R_2$ et X ont la même signification que précédemment, $R_7$ et $R_8$ représentent chacun l'hydrogène ou sont pris ensemble avec l'atome de carbone auquel ils sont attachés pour former un groupement phényle et A représente une chaîne alkylène, ce composé étant par la suite traité avec une amine de formule (3) pour obtenir le composé désiré de formule (1) sous forme de base libre.

De même, les composés de formule (1) dans laquelle Cy représente un groupement R-2 dihydro-4,5 furannyl-3 éventuellement mono- ou disubstitué, A représente une chaîne alkylène et B représente un groupement -S- ou $-SO_2-$, peuvent être préparés en hydrolysant un dérivé de cyclopropane de formule (29) en présence d'une solution aqueuse d'hydroxyde de métal alcalin pour obtenir un dérivé méthoxy-4 phényle de formule générale :

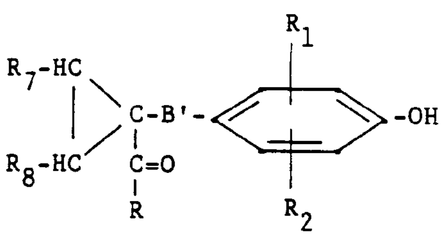

(55)

dans laquelle B', R, $R_1$, $R_2$, $R_7$ et $R_8$ ont la même signification que précédemment, dérivé que l'on fait alors réagir :

- avec un dihaloalkane de formule (5) et le produit résultant avec une amine de formule (3)
  ou
- avec un composé de formule générale (52), pour obtenir un dérivé aminoalkoxyphényle de formule générale :

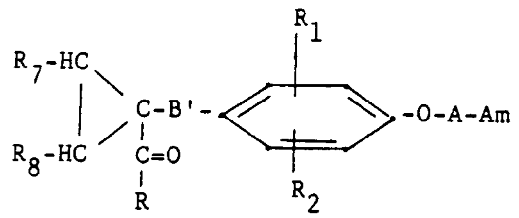

(56)

dans laquelle B', R, $R_1$, $R_2$, $R_7$, $R_8$ et Am ont la même signification que précédemment et A représente une chaîne alkylène.

On chauffe alors le dérivé de cyclopropane de formule (56) à une température comprise entre 100 et 130°C et en présence d'un catalyseur de transfert de phase tel que par exemple la triphénylphosphine ou le chlorure de tricaprylylammonium,pour obtenir le dérivé désiré de dihydro-2,3 furanne de formule (1) sous forme de base libre.

II. - Les composés de formule (1) dans laquelle B représente un groupement -SO- peuvent être obtenus en traitant, avec un agent oxydant,un sulfure de formule(1) dans laquelle B représente un groupement -S-, ce composé de formule (1) étant sous forme de base libre ou d'un sel de façon à obtenir le dérivé désiré de formule (1) sous forme de base libre ou de sel.

Lorsque le composé désiré est obtenu sous forme de sel, on peut régénérer la base libre par traitement avec un agent basique tel qu'un carbonate de métal alcalin par exemple le carbonate de potassium ou un bicarbonate de métal alcalin par exemple le bicarbonate de sodium.

Généralement, la réaction a lieu dans l'eau ou dans un solvant organique tel que le chlorure de méthylène et en présence d'un agent oxydant approprié tel que par exemple le périodate de sodium, le permanganate de potassium ou l'acide chloro-3 perbenzoïque.

Selon l'agent oxydant utilisé, on peut obtenir des mélanges de sulfoxydes ou de sulfones. Ces mélanges peuvent être séparés par des procédés conventionnels par exemple par chromatographie.

III. - Les composés de formule (1) dans laquelle B représente un groupement -S- ou -$SO_2$- et A représente une chaîne hydroxy-2 propylène éventuellement substituée peuvent être obtenus en faisant réagir au reflux un dérivé hydroxy-4 phényle de formule (4) avec une épihalohydrine telle que l'épichlorhydrine ou l'épibromhydrine sous forme dextrogyre, lévogyre ou sous forme de mélange de ces isomères par exemple sous forme racémique et en présence d'un agent basique tel qu'un carbonate de métal alcalin par exemple le carbonate de potassium, un hydroxyde de metal alcalin par exemple l'hydroxyde de sodium ou de potassium, un hydrure de métal alcalin tel que l'hydrure de sodium ou un alcoolate de métal alcalin par exemple le méthylate ou l'éthylate de sodium et dans un solvant polaire telle que la méthyl-éthyl-cétone pour donner les dérivés oxyranylméthoxy de formule générale :

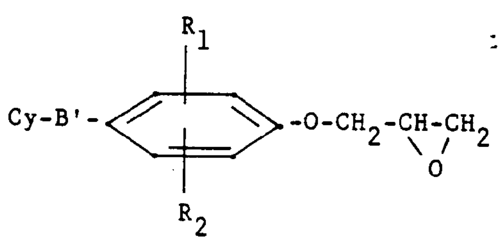

(57)

dans laquelle Cy, B', $R_1$ et $R_2$ ont la même signification que précédemment.

Les dérivés oxyranylméthoxy de formule (57) sont alors traités au reflux avec une amine de formule (3) et ce, dans un solvant polaire tel que la méthyl-éthyl-cétone ou dans un excès d'amine de formule (3) pour donner le dérivé désiré de formule (1) sous forme de base libre dans laquelle A représente une chaîne hydroxy-2 propylène, dérivé que l'on peut faire réagir, si on le désire, avec un halogénure d'alkyle inférieur en présence d'une base forte pour former les composés de formule (1) sous forme de base libre dans laquelle A représente une chaîne hydroxy-2 propylène dans laquelle l'hydroxy est substitué par un radical alkyle inférieur.

Dans certains cas, des produits secondaires peuvent se former parallèlement aux composés de formule (57) ci-dessus, par exemple des dérivés (halogéno-3 hydroxy-2 propoxy)-4 benzènesulfonyles.

Par réaction avec l'amine de formule (3), ces dérivés donneront naissance néanmoins aux composés désirés de formule (1) dans laquelle A représente une chaîne hydroxy-2 propylène.

Les composés de formule (1) ainsi obtenus sous forme de base libre peuvent ensuite être transformés en

sels pharmaceutiquement acceptables par réaction avec un acide organique ou inorganique approprié par exemple l'acide oxalique, maléique, fumarique, méthanesulfonique, benzoïque, ascorbique, pamoïque, succinique, hexamique, bisméthylènesalicylique, éthanedisulfonique, acétique, propionique, tartrique, salicylique, citrique, gluconique, lactique, malique, cinnamique, mandélique, citraconique, aspartique, palmitique, stéarique, itaconique, glycolique, p-aminobenzoïque, glutamique, benzènesulfonique, théophylinne acétique, ou avec la lysine ou l'histidine.

Comme il a été rapporté en détail par R. CHARLIER dans "Bruxelles Médical", No. 9, septembre 1969, pages 543-560, il est admis qu'une médication antiangineuse doit être capable notamment d'antagoniser les réactions cardiovasculaires de type adrénergique. A cet effet, on a proposé des agents aptes à bloquer les récepteurs α.

Cependant l'application clinique de tels composés au traitement de l'angor resta sans succès très probablement par le fait que les antagonistes des récepteurs α n'induisent qu'une neutralisation très partielle du système adrénergique, l'activité des récepteurs β étant respectée.

Or, les manifestations hémodynamiques les plus indésirables qui surviennent chez l'angineux de poitrine au cours de ses accès douloureux sont avant tout cardiaques et relèvent de ce fait des récepteurs β.

Parallèlement, on a proposé des médications antagonistes des récepteurs adrénergiques β. Ces composés, d'un intérêt clinique réel, diminuent les crises d'angor en réduisant le travail cardiaque par ralentissement de la fréquence du rythme. Cependant, il n'y a pas de chute des résistances artérielles périphériques qui s'élèvent au contraire par libération du tonus α.

Ces médications modifient toutefois certains paramètres hémodynamiques dans un sens qui, sur le plan fondamental, constitue une contrepartie défavorable à l'angineux de poitrine en particulier et au cardiaque en général.

Si l'on considère l'aspect antiadrénergique des β-bloquants, il devient évident que seules la tachycardie et l'augmentation de la puissance et de la vélocité de la contraction cardiaque sont susceptibles d'être neutralisées, l'hypertension artérielle relevant d'une stimulation des récepteurs α sur lesquels les antagonistes β n'ont pas d'action.

Or, si les perturbations cardiovasculaires entraînées par la stimulation des récepteurs β sont les plus préjudiciables à l'angineux, il n'en reste pas moins que l'hypertension artérielle joue également un rôle qui n'est pas négligeable.

Au surplus, le blocage des récepteurs β comporte un risque privant l'insuffisant cardiaque d'un mécanisme compensateur qu'il met normalement en jeu pour limiter son insuffisance circulatoire.

Ce mécanisme réflexe dont la composante principale emprunte la voie du système β-adrénergique aboutit notamment à une augmentation de la puissance et de la vélocité de la contraction cardiaque. Par conséquent , si ce système est bloqué, l'insuffisant cardiaque voit s'aggraver sa défaillance fonctionnelle. Il est donc logique de considérer que l'emploi d'un β-bloquant dont l'action est <u>pure</u> et <u>complète</u> comportera toujours un risque cardiaque.

Il paraît donc souhaitable de ne pas rechercher des propriétés antagonistes α ou β complètes, étant donné les effets secondaires qu'elles peuvent entraîner en clinique. Il semble plus logique de viser à amortir plutôt qu'à supprimer les perturbations cardiovasculaires qui caractérisent l'hyperstimulation du système adrénergique dans sa totalité.

Les composés de l'invention répondent à cet objectif puisqu'ils présentent des propriétés antiadrénergiques de type α et β incomplètes. Ils peuvent donc être considérés, non comme des β-bloquants mais comme des adrénofreinateurs c'est-à-dire des antagonistes partiels des réactions adrénergiques α et β potentiellement dépourvues des désavantages énumérés ci-dessus pour les β-bloquants.

En outre, la composante inhibitrice calcique mise en évidence chez les composés de l'invention complétera d'une manière remarquable leur spectre pharmacologique cardiovasculaire.

On sait, en effet, que la translocation des ions calcium est une des composantes essentielles du potentiel d'action au niveau des cellules cardiaques et que, à ce titre, elle joue un rôle primordial dans la conduction électrique ainsi que dans ses troubles éventuels (arythmie). En outre, il est connu que les ions calcium sont impliqués dans le couplage excitation-contraction lequel contrôle, au niveau de la musculature lisse le degré de vasoconstriction et, par la même occasion, joue un rôle critique dans la crise d'angine de poitrine.

Les composés antagonistes du calcium agissent au niveau de la membrane cellulaire en empêchant sélectivement le calcium d'intervenir dans le processus de contraction au sein de la cellule artérielle.

Or, il apparaît de plus en plus évident, à l'heure actuelle, que les résultats cliniques apportés par l'association d'inhibiteurs calciques et d'inhibiteurs β-adrénergiques sont meilleurs que lorsque chaque inhibiteur est utilisé isolément (J.A.M.A. 1982, 247, pages 1911-1917).

Il semble, en outre, qu'il n'existe, à l'heure actuelle, aucun β-bloquant exerçant en plus une action inhibitrice appréciable au niveau de la translocation calcique.

De ce point de vue, les composés de l'invention présentant à la fois une composante anticalcique et une composante antiadrénergique α et β seront d'un intérêt primordial car susceptibles d'applications thérapeutiques plus étendues qu'un β-bloquant isolé ou qu'un inhibiteur calcique isolé. A titre d'exemple, on citera :

- le {[(diméthoxy-6,7 tétrahydro-1,2,3,4 N-isoquinoléinyl-2)-3 propoxy-4 benzènesulfonyl}-1 isopropyl-2 indolizine (Ex. 1)
- le {[(N-méthyl N-(diméthoxy-6,7 tétrahydro-1,2,3,4 naphtyl-1) amino-3 propoxy]-4 benzènesulfonyl}-1 isopropyl-2 indolizine (Ex. 13)

Cependant l'intérêt majeur de ces composés résidera dans le fait qu'ils pourront, grâce à leur composante anticalcique, être utilisés dans le traitement de l'angine au repos, syndrome provoqué par l'apparition d'un spasme au niveau des coronaires lequel est combattu actuellement par des composés tels que le diltiazem, le vérapamil ou la nifédipine.

Au surplus, des composés de l'invention ont montré, in vivo, un degré de métabolisation beaucoup moins rapide que celui des composés du brevet FR 2.594.438.

Les résultats de tests pharmacologiques effectués en vue de déterminer les propriétés cardiovasculaires des composés de l'invention sont répertoriés ci-dessous.

I. Propriétés inhibitrices calciques

Les propriétés inhibitrices de la translocation calcique au niveau membranaire présentées par les composés de l'invention ont été mises en évidence par mesure de leur action antagoniste vis-à-vis de la réponse contractile à la dépolarisation provoquée par le potassium sur l'aorte isolée de rat. Il est bien établi que la dépolarisation de la membrane d'un muscle lisse par le potassium rend cette dernière perméable au calcium extracellulaire et provoque la contraction musculaire.

Dès lors, la mesure de l'inhibition de la réponse contractile à la dépolarisation par le potassium ou la mesure d'un relâchement de la contraction tonique à la dépolarisation potassique peut constituer une évaluation de la puissance d'un composé en tant qu'inhibiteur de la perméabilité membranaire aux ions $Ca^{++}$.

La technique utilisée est la suivante :

Sur des rats mâles Wistar pesant environ 300g, on prélève l'aorte et on la coupe en bandelettes d'environ 40mm de longueur et 3mm de largeur.

On place ces fragments dans une cuve à organe isolé de 25ml contenant une solution de Krebs-bicarbonate modifiée (NaCl 112mM; KCl 5mM; $NaHCO_3$ 25mM; $KH_2PO_4$ 1mM; $MgSO_4$ 1,2mM; $CaCl_2$ 2,5 mM; glucose 11,5mM; eau distillée jusqu'à 1000ml) parcourue par un courant de carbogène et maintenue à 37°C. On relie la préparation à un microcapteur de force et on enregistre la réponse contractile après amplification sur un enregistreur.

On applique une tension de 2g à la préparation. On maintient celle-ci durant 60 minutes dans la solution de Krebs-bicarbonate modifiée puis on provoque les contractions en remplaçant la solution de Krebsbicarbonate par une solution de Krebs-potassique (NaCl 17mM; KCl 100mM; $NaHCO_3$ 25mM; $KH_2PO_4$ 1mM; $MgSO_4$ 1,2mM; $CaCl_2$ 2,5mM; glucose 11,5mM; eau distillée jusqu'à 1000ml). Lorsque la réponse contractile de la préparation est devenue reproductible, on introduit,dans le bain, une quantité donnée d'un composé de l'invention. Soixante minutes plus tard un nouveau spasme est provoqué par la dépolarisation potassique.

Les résultats obtenus sur l'aorte éprouvée sont alors exprimés en % de l'effet contracturant maximal avant l'incubation avec la substance à tester.

A titre d'exemples, les résultats suivants ont été obtenus, les composés de formule (1) étant sous la forme de base ou d'oxalate.

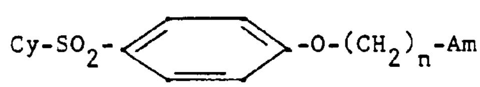

| Composé | Cy | n | Am | % de l'effet contracturant maximal | | | |
|---|---|---|---|---|---|---|---|
| | | | | $10^{-6}M$ | $10^{-7}M$ | $10^{-8}M$ | $10^{-9}$ |
| Ex. 1 | 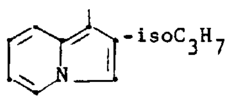 | 3 | 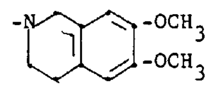 | 9,6 | 18,3 | 72,4 | 92,6 |
| Ex.7 | 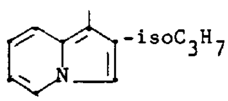 | 4 | 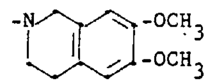 | 8,4 | 31,8 | 82,1 | - |
| Ex.13 | 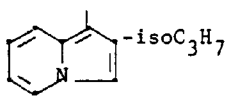 | 3 | 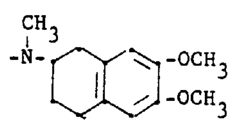 | 4,4 | 13,3 | 61,4 | 87,8 |

| Composé | Cy | n | Am | % de l'effet contractu-rant maximal | | | |
|---|---|---|---|---|---|---|---|
| | | | | $10^{-6}M$ | $10^{-7}M$ | $10^{-8}M$ | $10^{-9}M$ |
| Ex. 6 | 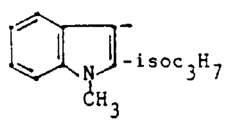 | 3 | 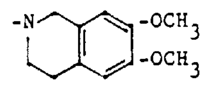 | 10,8 | 47,2 | 89,2 | - |
| Ex. 8 | 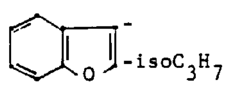 | 3 | 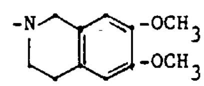 | 9,5 | 35,4 | 65,9 | 81,5 |
| Ex. 10 | 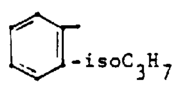 | 3 | 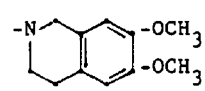 | 1,2 | 44,6 | 81,2 | - |
| Ex. 14 | 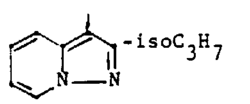 | 3 | 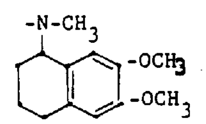 | 16,7 | 25,6 | 78,6 | - |
| Ex. 17 | 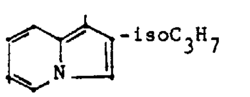 | 3 | 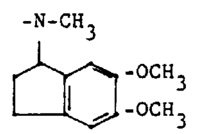 | 6,2 | 8,1 | 50 | 78,7 |
| Ex. 23 | 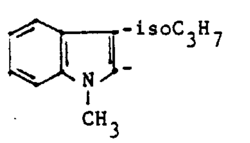 | | 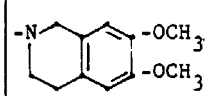 | 23,3 | 37 | 61,9 | 81,7 |

II. Propriétés antiadrénergiques

Le but de ce test est de déterminer la capacité des composés de l'invention de réduire l'augmentation de la pression sanguine induite par l'épinéphrine (effet anti-α) et l'accélération de la fréquence cardiaque induite par l'isoprénaline (effet anti-β) chez le chien préalablement anesthésié au pentobarbital et atropiné.

On détermine d'abord pour chaque chien la dose d'épinéphrine (entre 3 et 10 μg/kg) qui provoque une augmentation reproductible de la pression artérielle d'environ 133.10²Pa et la dose d'isoprénaline (1 à 2 μg/kg) qui provoque une augmentation reproductible de la fréquence cardiaque d'environ 70 battements/minute. On injecte alternativement toutes les dix minutes la dose d'épinéphrine et d'isoprénaline ainsi déterminée et après obtention de deux réponses de références successives, on administre une quantité du composé à étudier par

voie intraveineuse.

- Effet anti-α

On enregistre le pourcentage de réduction de l'hypertension provoquée par le composé à étudier comparativement à l'hypertension de référence obtenue précédemment (environ 100 mm Hg).

-Effet anti-β

On enregistre le pourcentage de réduction de l'accélération de la fréquence cardiaque provoquée par le composé à étudier comparativement à la tachycardie de référence mesurée précédemment (environ 70 battements).

Dans les deux cas, on a exprimé les résultats de la réduction de la pression artérielle ou de la fréquence cardiaque comme suit :

+ pour une réduction < 50%
++ pour une réduction ≧ 50%
+++ pour une réduction sub-totale (réduction presque complète)

On a enregistré les résultats suivants :

| Composés | Dose (mg/kg) | Effet anti-α | Effet anti-β |
|---|---|---|---|
| Ex. 1 | 0,127 | +++ | ++ |
| Ex. 2 | 0,64 | ++ | ++ |
| Ex. 3 | 1,2 | ++ | ++ |
| Ex. 5 | 2,6 | + | + |
| Ex. 6 | 0,33 | ++ | ++ |
| Ex. 7 | 0,66 | +++ | +++ |
| Ex. 8 | 0,63 | +++ | +++ |
| Ex. 9 | 1,3 | +++ | ++ |
| Ex. 10 | 0,6 | + | + |
| Ex. 12 | 1,2 | +++ | +++ |
| Ex. 13 | 0,34 | +++ | ++ |
| Ex. 14 | 1,2 | +++ | ++ |
| Ex. 17 | 0,11 | +++ | ++ |
| Ex. 18 | 2,8 | ++ | ++ |
| Ex. 23 | 0,068 | +++ | + |

III - Toxicité

La toxicité des composés de l'invention s'est révélée compatible avec leur emploi en thérapeutique.

Les compositions thérapeutiques selon l'invention peuvent être présentées sous toute forme convenant à l'administration en thérapie humaine ou vétérinaire. Pour ce qui concerne l'unité d'administration, celle-ci peut prendre la forme, par exemple, d'un comprimé, d'une dragée, d'une capsule, d'une gélule, d'une poudre, d'une suspension ou d'un sirop pour l'administration orale, d'un suppositoire pour l'administration rectale ou d'une solution ou suspension pour l'administration parentérale.

Les compositions thérapeutiques de l'invention pourront comprendre, par unité d'administration, par exemple de 50 à 500mg en poids d'ingrédient actif pour l'administration orale, de 50 à 200mg d'ingrédient actif pour l'administration rectale et de 50 à 150mg d'ingrédient actif pour l'administration parentérale.

Suivant la voie d'administration choisie, les compositions thérapeutiques ou vétérinaires de l'invention seront préparées en associant au moins un des composés de formule (1) ou un sel d'addition non toxique de ce composé avec un excipient approprié, ce dernier pouvant être constitué par exemple d'au moins un ingrédient sélectionné parmi les substances suivantes : lactose, amidons, talc, stéarate de magnésium, polyvinylpyrrolidone, acide alginique, silice colloidale, eau distillée, alcool benzylique ou agents édulcorants.

Les Exemples, non limitatifs suivants illustrent l'invention :

EXEMPLE 1

Préparation de l'oxalate acide de {[(diméthoxy-6,7 tétrahydro-1,2,3,4 N-isoquinoléinyl-2)-3 propoxy]-4 benzènesulfonyl}-1 isopropyl-2 indolizine (SR 33710A)

a) [(Bromo-3 propoxy)-4 benzènesulfonyl]-1 isopropyl-2 indolizine

On mélange 3,15g (0,01 mole) d'(hydroxy-4 benzènesulfonyl)-1 indolizine, 40,38g (0,2 mole; 20,3 ml) de dibromo-1,3 propane, 1,66g (0,012 mole) de carbonate de potassium et 20ml de N,N-diméthylformamide. On chauffe à 100°C et on suit la réaction par chromatographie sur couche mince (solvant : dichlorométhane/acétate d'éthyle 95/5). On laisse réagir durant 50 minutes, puis on élimine l'excès de dibromo-1,3 propane par évaporation sous pression réduite. On reprend dans l'acétate d'éthyle et on lave avec de l'hydroxyde de sodium dilué puis avec de l'eau. On sèche sur carbonate de potassium puis on filtre. On coule dans de l'eau, on extrait avec de l'acétate d'éthyle puis on lave avec de l'eau et avec une solution saturée de chlorure de sodium. On sèche sur sulfate de sodium et on concentre.

De cette manière, on obtient environ 4g de [(bromo-3 propoxy)-4 benzènesulfonyl]-1 isopropyl-2 indolizine sous forme brute que l'on recristallise dans un mélange acétate d'éthyle/hexane.

Rendement après recristallisation : 67%

P.F. : 135,4°C

De la même manière que précédemment, on a préparé la [(bromo-4 butoxy)-4 benzènesulfonyl]-1 isopropyl-2 indolizine

Rendement : 81,5%.

b) Oxalate acide de {[(diméthoxy-6,7 tétrahydro-1,2,3,4 N-isoquinoléinyl-2)-3 propoxy]-4 benzènesulfonyl}-1 isopropyl-2 indolizine

On mélange, à température ambiante, 1,1g (0,0025 mole) de [(bromo-3 propoxy)-4 benzènesulfonyl]-1 isopropyl-2 indolizine, 1,14g (0,005 mole) de chlorhydrate de diméthoxy-6,7 tétrahydro-1,2,3,4 isoquinoléine et 1,38g (0,010 mole) de carbonate de potassium dans 5ml de diméthylsulfoxyde. On agite le mélange pendant 22 heures, tout en suivant l'évolution de la réaction par chromatographie sur couche mince (solvant : méthanol), puis on verse le produit réactionnel dans l'eau.

On extrait avec du dichlorométhane et on lave avec une solution aqueuse saturée de chlorure de sodium. On sèche sur sulfate de sodium et on concentre pour obtenir environ 1,6g de produit brut. On purifie par chromatographie sur gel de silice en éluant avec un mélange acétate d'éthyle/méthanol 80/20.

On récupère ainsi 1,05g de {[(diméthoxy-6,7 tétrahydro-1,2,3,4 N-isoquinoléinyl-2)-3 propoxy]-4 benzènesulfonyl}-1 isopropyl-2 indolizine sous forme de base libre.

Rendement : 76,5%.

On fait alors réagir 1g (0,0018 mole) de la base ainsi obtenue, avec 0,164g (0,0018 mole) d'acide oxalique dans un mélange acétate d'éthyle/éther éthylique.

De cette manière, on récupère environ 0,95g d'oxalate acide de {[(diméthoxy-6,7 tétrahydro-1,2,3,4 N-isoquinoléinyl-2)-3 propoxy]-4 benzènesulfonyl}-1 isopropyl-2 indolizine que l'on peut recristalliser dans un mélange acétate d'éthyle/dichlorométhane par addition d'éther éthylique.
Rendement : 83%
P.F. : 120-122°C.

EXEMPLE 2

Préparation de l'oxalate acide d'éthyl-2 {[(diméthoxy-6,7 tétrahydro-1,2,3,4 N-isoquinoléinyl-2)-3 propoxy]-4 benzènesulfonyl}-3 benzothiophène (SR 33840A).

On agite pendant 3 jours un mélange de 4g (0,0126 mole) d'éthyl-2 (hydroxy-4 benzènesulfonyl)-3 benzothiophène et 5g (0,0189 mole) de (chloro-3 propyl)-2 diméthoxy-6,7 tétrahydro-1,2,3,4 isoquinoléine dans 60ml de diméthylsulfoxyde anhydre en présence de 6g (0,441 mole) de carbonate de potassium. Après réaction, on verse le mélange dans un grand volume d'eau et on extrait avec 3 fois 100ml de toluène. On lave à l'eau, sèche sur sulfate de sodium, filtre et évapore à sec sous vide. On agite dans de l'heptane et on recristallise, dans l'éthanol, le produit formé. On obtient ainsi 3,1g de produit dont on forme l'oxalate dans l'acétate d'éthyle à l'ébullition.

De cette manière, on recueille 3g d'oxalate acide d'éthyl-2 {[(diméthoxy-6,7 tétrahydro-1,2,3,4 N-isoquinoléinyl-2)-3 propoxy]-4 benzènesulfonyl}-3 benzothiophène après recristallisation dans l'éthanol.
Rendement : 37%
P.F. : 171°C

EXEMPLE 3

Préparation de l'oxalate acide de {[(diméthoxy-6,7 tétrahydro-1,2,3,4 N-isoquinoléinyl-2)-3 propoxy]-4 benzènesulfonyl}-4 isopropyl-5 oxazole (SR 33868 A)

a) N-(Méthoxy-4-benzènesulfonylmethyl)formamide.

On chauffe pendant 2h à 90-95°C, une suspension formée de 38,85g (0,2 mole) de méthoxy-4 benzènesulfinate de sodium, 46,6ml (0,59 mole) d'une solution de formaldéhyde à 34-37%, 90,7g (2,0 moles) de formamide, 32,5g (0,707 mole) d'acide formique et 100ml d'eau.
Pendant la réaction le benzènesulfinate se dissout graduellement. On laisse refroidir à l'air puis dans un bain de glace et on abandonne le mélange au congélateur durant environ 10 heures. On filtre le produit qui a cristallisé, on le lave 3 fois avec 30 à 35ml d'eau glacée puis on le sèche à 70°C sous vide.

De cette manière, on obtient 18,3g de N-(méthoxy-4 benzènesulfonylméthyl)formamide ce qui représente un rendement de 39,9%.
P.F. : 105-107°C.
Le filtrat fournit encore 1g de produit désiré soit 2,2% (P.F. : 105-107°C)
Rendement total : 19,3g soit 42,1%.
Pureté : 98,95%

b) N-(Méthoxy-4-benzènesulfonyl)méthylisonitrile .

A un mélange de 16,05g (0,07 mole) de N-(méthoxy-4 benzènesulfonylméthyl)formamide, 35ml de diméthoxy-1,2 éthane, 14ml d'éther isopropylique et 35g (0,35 mole) de triéthylamine, refroidit à -10°C, on ajoute, goutte à goutte, à une température comprise entre -10 et 0°C, une solution de 11,71g (0,077 mole) d'oxychlorure de phosphore dans 8,5ml de diméthoxy-1,2 éthane. On agite encore 0,5 heure à environ 0°C puis on ajoute, goutte à goutte, 210ml d'eau glacée en maintenant la température à 0°C. Les sels de triéthylamine se dissolvent et ensuite un précipité brun-orange se forme. On agite encore 0,5 heure à 0°C puis on filtre et lave avec 35ml d'eau glacée.
On obtient le N - (méthoxy - 4 benzènesulfonyl) méthylisonitrile sous forme brute que l'on recristallise dans 50ml de méthanol pour donner 10,3g soit un rendement de 69,6%.
P.F. : 99,5-101°C.

c) Isopropyl-5 (méthoxy-4 benzènesulfonyl)-4 oxazole.

A une température comprise entre 15 et 20°C, on ajoute rapidement 1,1g (0,0103 mole) de chlorure d'iso-

butyryle dans 10ml de diméthoxy-1,2 éthane à un mélange de 2,1g (0,01 mole) de (méthoxy-4 benzènesulfonyl) méthylisonitrile dans 10ml de diméthoxy-1,2 éthane en présence de 0,65g (0,0116 mole) d'hydroxyde de potassium. On agite durant 3h à environ 20°C, on refroidit dans un bain de glace et on ajoute de l'eau. Une huile précipite qui se solidifie en présence d'un peu de méthanol. De cette manière, on obtient 0,45g d'isopropyl-5 (méthoxy-4 benzènesulfonyl)-4 oxazole que l'on recristallise dans 5ml d'isopropanol pour donner 0,4g soit un rendement de 14,2%.
P.F. : 98-100°C

d) (Hydroxy-4 benzènesulfonyl)-4 isopropyl-5 oxazole .

On chauffe à reflux, durant 6 heures, un mélange formé de 2,35g (0,0083 mole) d'isopropyl-5 (méthoxy-4 benzènesulfonyl)-4 oxazole et 4,4g (0,033 mole) de chlorure d'aluminium dans 58ml de dichloréthane.

On verse le mélange dans 250ml d'eau et de glace et on agite durant 0,5 heure. On décante la phase organique, lave jusqu'à neutralité avec 2 fois 50ml d'eau, sèche sur sulfate de sodium et évapore à sec sous vide. Un produit précipite dans la phase aqueuse, produit que l'on ajoute au résidu issu de la phase organique. On dissout l'ensemble dans du méthanol et on décolore avec 1g de charbon actif. On filtre et on évapore. On purifie alors le résidu par chromatographie d'élution sur silice avec du méthanol comme éluant.
Après recristallisation dans 40ml de dichloréthane, on obtient 0,8g d'(hydroxy-4 benzènesulfonyl)-4 isopropyl-5 oxazole ce qui représente un rendement de 36,4%.
P.F. : 196-198°C.

d)Oxalate acide de {[(diméthoxy-6,7 tétrahydro-1,2,3,4 N-isoquinoléinyl-2)-3 propoxy}-4 benzènesulfonyl]-4 isopropyl-5 oxazole.

A la température du bain de glace, on mélange 1,35g (0,005 mole) d'hydroxy-4 benzènesulfonyl)-4 isopropyl-5 oxazole et 1,83g (0,0068 mole) de (chloro-3 propyl)-2 diméthoxy-6,7 tétrahydro-1,2,3,4 isoquinoléine dans 25ml de diméthylsulfoxyde en présence de 3,45g (0,025 mole) de carbonate de potassium. On laisse revenir à température ambiante et on agite durant 4 jours. On verse dans 125ml d'eau glacée et on extrait, avec 25ml de dichloréthane, le produit qui a précipité. On lave à l'eau et on décolore avec 0,5g de charbon actif. On évapore à sec et on recristallise dans 12ml d'isopropanol. On obtient ainsi 1,3g de solide que l'on redissout dans 15ml de méthanol.
On ajoute alors 0,27g d'acide oxalique dissous dans 5ml de méthanol.
Après recristallisation dans 20ml de méthanol, on obtient 1,4g d'oxalate acide de {[(diméthoxy-6,7 tétrahydro-1,2,3,4 N-isoquinoléinyl-2)-3 propoxy]-4 benzènesulfonyl}-4 isopropyl-5 oxazole soit un rendement de 55,9%.
P.F. : 183-185°C.

EXEMPLE 4

Préparation du {[(diméthoxy-6,7 tétrahydro-1,2,3,4 N-isoquinoléinyl-2)-3 propoxy]-4 benzènesulfonyl}-4 éthyl-5 méthyl-1 pyrazole (SR 33857)

a) (Tosyloxy-4 benzènesulfonyl)-1 butanone-2.

On chauffe à reflux pendant 3 heures, une solution formée de 11,65g (0,035 mole) de tosyloxy-4 benzènesulfinate de sodium et 5,26g (0,035 mole) de bromo-1 butanone-2 à 90% dans 35ml d'éthanol. On laisse refroidir et on agite durant 2 heures dans un bain d'eau et de glace. On essore et on lave d'abord avec un peu d'éthanol ensuite 4 fois avec de l'eau.

De cette manière, on obtient 10,56g de (tosyloxy-4 benzènesulfonyl)-1 butanone-2 soit un rendement de 78,8%.
P.F. : 104-106°C.

En utilisant le même procédé que précédemment, on a préparé la (tosyloxy-4 benzènesulfonyl)-1 méthyl-3 butanone-2
P.F. : 156-157°C.

b) (N,N-Diméthylamino)-1 propionyl-1 (tosyloxy-4 benzènesulfonyl)-2 éthène

On chauffe à reflux durant 18 heures un mélange formé de 10,25g (0,027 mole) de (tosyloxy-4 benzènesulfonyl)-1 butanone-2 et 8,05g (0,067 mole) de diméthylformamide diméthylacétal dans 55ml de toluène. On

laisse refroidir, filtre un léger produit insoluble et évapore à sec sous vide à 55°C. On obtient ainsi un résidu huileux de 13,6g que l'on agite avec 30ml de méthanol pendant 2h pour donner 6,7g de produit cristallin.

De cette manière, on obtient le (N,N-diméthylamino)-1 propionyl-1 (tosyloxy-4 benzènesulfonyl)-2 éthène avec un rendement de 57,75%.

P.F. : 148-149,5°C.

En utilisant le même procédé que précédemment, on a préparé l'isobutyroyl-1 (tosyloxy-4 benzènesulfonyl)-1 N,N-diméthylamino-2 éthène.

Rendement : 65,5%

Pureté : 92,01%

P.F. : 115-116°C.

c) Ethyl-5 (hydroxy-4 benzènesulfonyl)-4 méthyl-1 pyrazole.

On chauffe à reflux pendant 20 heures, un mélange formé de 4,3g (0,01 mole) de (N,N-diméthylamino)-1 propionyl-1 (tosyloxy-4 benzènesulfonyl)-2 éthène dans 25ml de méthanol et 10ml d'eau en présence de 2,35g (0,05 mole) de méthylhydrazine. On laisse revenir à température ambiante en on refroidit durant 1h dans de la glace. On évapore à sec et on obtient ainsi 4,85g de résidu que l'on recristallise dans 250ml d'eau et décolore avec 0,8g de charbon actif.

On filtre et on laisse cristalliser pendant 2 heures dans de la glace.

On recristallise à nouveau dans 50ml de dichloréthane.

De cette manière, on obtient 1,15g d'éthyl-5 (hydroxy-4 benzènesulfonyl)-4 méthyl-1 pyrazole, ce qui représente un rendement de 43,2%.

P.F. : 188,5-190°C.

En utilisant le même procédé que précédemment, on a obtenu l'isopropyl-5 méthyl-1 (hydroxy-4 benzènesulfonyl)-4 pyrazole.

Rendement : 74,95%

P.F. : 209-210,5°C

Pureté : 100%

d) {[(Diméthoxy-6,7 tétrahydro-1,2,3,4 isoquinoléinyl-2)-3 propoxy]-4 benzènesulfonyl}-4 éthyl-5 méthyl-1 pyrazole.

Ce composé a été obtenu selon la méthode décrite à l'Exemple 2.

P.F. : 120,5-122°C.

De la même manière que précédemment, on a préparé le {[(diméthoxy-6,7 tétrahydro-1,2,3,4 N-isoquinoléinyl-2)-3 propoxy]-4 benzènesulfonyl}-4 isopropyl-5 méthyl-1 pyrazole. (SR 33849) (Exemple 5)

Rendement : 66,6%

P.F. : 90-92°C.

En utilisant les procédés exemplifiés ci-dessus, on a préparé les composés suivants :

Oxalate de {[(diméthoxy-6,7 tétrahydro-1,2,3,4 N-isoquinoléinyl-2)-3 propoxy]-4 benzènesulfonyl}-3 isopropyl-2 méthyl-1 indole (SR 33837A) (Exemple 6).

Rendement : 26,3%

Pureté : 98,6%

P.F. : 107°C.

Oxalate acide de {[(diméthoxy-6,7 tétrahydro-1,2,3,4 N-isoquinoléinyl-2)-4 butoxy]-4 benzènesulfonyl}-1 isopropyl-2 indolizine (SR 33717A) (Exemple 7)

P.F. : 176,2°C

Oxalate acide d'isopropyl-2 {[(diméthoxy-6,7 tétrahydro-1,2,3,4 N-isoquinoléinyl-2)-3 propoxy]-4 benzènesulfonyl}-3 benzofuranne (SR 33830A) (Exemple 8)

P.F. : 185°C

Oxalate acide d'éthyl-2 {[(diméthoxy-6,7 tétrahydro-1,2,3,4 N-isoquinoléinyl-2)-3 propoxy}-4 benzènesulfonyl]-3 benzofuranne (SR 33842A) (Exemple 9)

P.F. : 165°C

Oxalate de {[(diméthoxy-6,7 tétrahydro-1,2,3,4 N-isoquinoléinyl)-2 propoxy]-4 phényl} (isopropyl-2 phényl)sulfone (SR 33836A) (Exemple 10)

P.F. : environ 106°C

Oxalate acide de {[N-(pentahydro-5,6,7,8,9 benzocycloheptényl-9) N-méthylamino-3 propoxy]-4 benzènesulfonyl}-3 isopropyl-2 méthyl-1 indole (SR 33878A) (Exemple 11)

P.F. : environ 76°C.

Oxalate acide de {[N-méthyl N-(tétrahydro-1,2,3,4 naphtyl-1)amino-3 propoxy]-4 benzènesulfonyl}-3 isopropyl-2 méthyl-1 indole (SR 33874A) (Exemple 12)

P.F. : environ 70°C.

Oxalate acide de {[N-méthyl N-(diméthoxy-6,7 tétrahydro-1,2,3,4 naphtyl-2)amino-3 propoxy]-4 benzènesulfonyl}-1 isopropyl-2 indolizine (SR 33739A) (Exemple 13).

P.F. : 115°C.

Oxalate acide de {[N-méthyl N-(tétrahydro-1,2,3,4 naphtyl-1) amino-3 propoxy]-4 benzènesulfonyl}-3 isopropyl-2 pyrazolo[1,5-a] pyridine (SR 33894 A) (Exemple 14).

P.F. : 99,8°C.

Oxalate acide de méthyl-1 {[(diméthoxy-6,7 tétrahydro-1,2,3,4 N-isoquinoléinyl-2)-3 propoxy]-4 benzènesulfonyl}-2 isopropyl-3 indole (SR 33905 A) (Exemple 15).

P.F. : 116°C (pâteux).

Oxalate acide de méthyl-1 {[N-méthyl N-(tétrahydro-1,2,3,4 naphtyl-1) amino-3 propoxy]-4 benzènesulfonyl}-2 méthyl-3 indole (SR 33892 A) (Exemple 16)

P.F. : 101,4°C.

Isopropyl-2 {[N-méthyl N-(diméthoxy-5,6 indanyl-1) amino-3 propoxy]-4 benzènesulfonyl}-1 indolizine (SR 33887) (Example 17).

Huileux.

Oxalate acide de {[N-méthyl N-(tétrahydro-1,2,3,4 naphtyl-1) amino-3 propoxy]-4 benzènesulfonyl}-4 isopropyl-5 oxazole (SR 33883 A) (Exemple 18).

P.F. : 99,6°C (isopropanol).

Oxalate acide de {[N-méthyl N-(tétrahydro-1,2,3,4 naphtyl-1) amino-3 propoxy]-4 benzènesulfonyl}-4 isopropyl-5 méthyl-1 pyrazole (SR 33899 A) (Exemple 19).

P.F. : 150,7°C (isopropanol)

Oxalate acide de {[N-(pentahydro-5,6,7,8,9 benzocycloheptényl-9) N-méthyl-amino-3 propoxy]-4 benzènesulfonyl}-4 isopropyl-5 méthyl-1 pyrazole (SR 33888 A) (Exemple 20).

P.F. : 126,1°C (éthanol)

Oxalate acide d'isopropyl-2 {[N-méthyl N-(tétrahydro-1,2,3,4 naphtyl-1) amino-3 propoxy]-4 benzènesulfonyl}-3 benzofuranne (SR 33908 A) (Ex. 21)

P.F. : 113°C (méthyl éthyl cétone )

Oxalate acide de {[N-(pentahydro-5,6,7,8,9 benzocycloheptényl-9) N-méthyl-amino-3 propoxy]-4 benzènesulfonyl}-3 isopropyl-2 benzofuranne (Example 22). (SR 33913A)

P.F. : 161°C (acétate d'éthyl)

Fumarate acide de méthyl-1 {[(diméthoxy-6,7 tétrahydro-1,2,3,4 N-isoquinoléinyl-2)-3 propoxy]-4 benzènesulfonyl}-2 isopropyl-3 indole (SR 33905B) (Exemple 23)

P.F. : 117,4°C (éthanol/éther éthylique)

Oxalate acide de N-(pentahydro-5,6,7,8,9 benzocycloheptenyl-9) N-méthyl-amino-3 propoxy]-4 benzènesulfonyl}-3 éthyl-2 benzo[b]thiophène (SR 33909A) (Exemple 24)

P.F. : 150°C (méthyl éthyl cétone)

Oxalate acide de {[N-méthyl N-(tétrahydro-1,2,3,4 naphtyl-1) amino-3 propoxy]-4 benzènesulfonyl}-3 éthyl-2 benzo[b]thiophène (SR 33910A) (Exemple 25) P.F. : 167°C (méthyl éthyl cétone)

Chlorhydrate d'isopropyl-2 {[N-(méthoxy-7 tétrahydro-1,2,3,4 naphtyl-2) amino-3 propoxy]-4 benzènesulfonyl}-1 indolizine (Exemple 26 )

P.F. : 202°C (isopropanol)

Chlorhydrate de méthyl-1 phényl-2 {[(diméthoxy-6,7 tétrahydro-1,2,3,4 N-isoquinoléinyl-2)-3 propoxy]-4 benzènesulfonyl}-3 indole (Exemple 27)

P.F. : 152°C (acétate d'éthyle/éther éthylique)

Chlorhydrate de méthyl-1 {[(diméthoxy-6,7 tétrahydro-1,2,3,4 N-isoquinoléinyl-2)-3 propoxy]-4 benzènesulfonyl}-2 éthyl-3 indole (Exemple 27)

## Revendications

### Revendications pour les Etats contractants suivants : CH, DE, DK, FR, GB, IT, LI, LU, NL, SE, AT

**1.** Dérivés aminoalkoxyphényle correspondant à la formule générale :

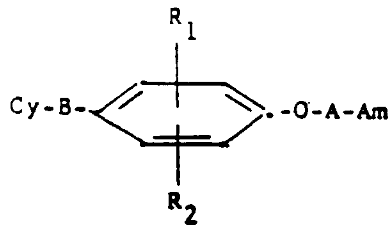

( 1 )

ainsi que leurs sels pharmaceutiquement acceptables dans laquelle :

B représente un groupement -S-, -SO- ou $-SO_2-$,

$R_1$ et $R_2$, qui sont identiques ou différents, représentent chacun l'hydrogène, le radical méthyle ou éthyle ou un halogène,

A représente un radical alkylène, linéaire ou ramifié en $C_2$-$C_5$ ou le radical hydroxy-2 propylène dans lequel l'hydroxy est éventuellement substitué par un radical alkyle en $C_1$-$C_4$,

Am représente un groupement :

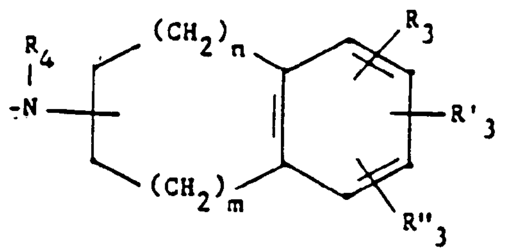

ou

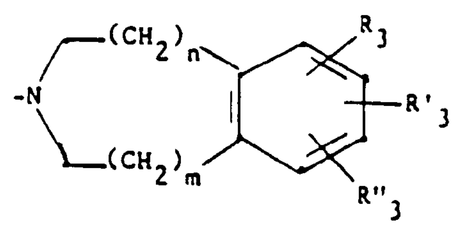

(D).

(E)

dans lequel : $R_3$, $R'_3$ et $R''_3$, qui sont identiques ou différents, représentent chacun l'hydrogène, un atome d'halogène, un groupement alkyle en $C_1$-$C_4$ ou un groupement alkoxy en $C_1$-$C_4$,

$R_4$ représente l'hydrogène ou un radical alkyle en $C_1$-$C_8$,

n et m, identiques ou différents, représentent chacun 0, 1, 2 ou 3,

Cy représente un groupement de formule :

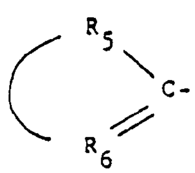

ou

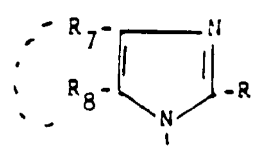

(F)

(G)

R représente l'hydrogène, un radical alkyle en $C_1$-$C_8$, un radical cycloalkyle en $C_3$-$C_6$, un radical benzyle ou un radical phényle éventuellement substitué par un ou plusieurs substituants, qui peuvent être identiques ou différents, choisis parmi des atomes d'halogène ou parmi des groupements alkyle en $C_1$-$C_4$, alkoxy en $C_1$-$C_4$ ou nitro,

$R_5$ et $R_6$ sont pris ensemble, avec l'atome de carbone auquel ils sont attachés, pour former :

- un groupement carbocyclique mono- ou di-cyclique éventuellement aromatique ayant de 5 à 10 atomes de carbone et éventuellement substitué par un groupement R en position α par rapport au groupement méthyne,
- un groupement hétérocyclique à 5 sommets éventuellement aromatique, les hétéroatomes ou hétérogroupes étant choisis parmi des groupements O, S, N,

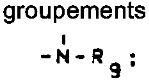 :

O et N; O et

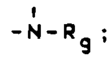

S et

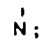

S et

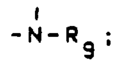

N et N; N et

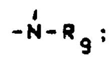

le groupement hétérocyclique étant éventuellement substitué par un groupement R tel que défini ci-dessus en position α par rapport au groupement méthyne et éventuellement substitué par un ou deux groupements choisis parmi des groupements alkyle en $C_1$-$C_4$ en phényle,

- un groupement hétérocyclique ayant de 6 à 10 sommets, mono ou di-cyclique et éventuellement aromatique, les hétéroatomes ou hétérogroupes étant choisis parmi des groupements O, S, N,

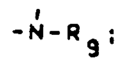

O et N; O et

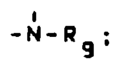

S et N; S et

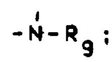

N et N; N et

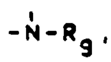

le groupement hétérocyclique étant éventuellement substitué par un groupement R tel que défini ci-dessus en position par rapport au groupement méthyne,

$R_7$ et $R_8$, qui sont identiques ou différents, représentent chacun l'hydrogène, un radical alkyle inférieur en $C_1$-$C_4$ ou un radical phényle ou lorsqu'ils sont pris ensemble, avec les atomes de carbone auxquels ils sont attachés, représentent un carbocycle à 6 sommets éventuellement aromatique,

$R_9$ représente l'hydrogène, un groupement alkyle en $C_1$-$C_4$, phényle, benzyle ou halogénobenzyle.

2. Dérivés aminoalkoxyphényle selon la revendication 1 dans laquelle Cy représente un groupement phényle, cyclohexényle, indényle, naphtyle, dihydronaphtyle, pyridyle, dihydropyridyle, furyle, dihydrofuryle, thiényle, dihydrothiényle, pyrrolyle, dihydropyrrolyle, pyrazolyle, imidazolyle, pyrimidyle, pyrazinyle, pyridazinyle, oxazolyle, isoxazolyle, thiazolyle, benzofuryle, benzothiényle, indolyle, benzimidazolyle, benzoxasolyle, quinoléinyle, benzisoxazolyle, cinnolinyle, quinoxalinyle, quinazolinyle, indolizinyle, thiénopyridyle, tétrahydrothiénopyridyle, pyrrolopyridyle, pyrazolopyridyle, pyrrolopyridazinyle, imidazopyridyle.

3. Dérivés aminoalkoxyphényle selon la revendication 1 dans laquelle Cy représente un groupement indolizinyle, benzofuryle, benzothiényle, indolyle, oxazolyle, pyrazolyle, phényle, pyrazolo[1,5-a]pyridyle ou imidazo[1,2-a]pyridyle.

4. Dérivés aminoalkoxyphényle selon la revendication 1 dans laquelle B représente un groupement $-SO_2-$.

5. Dérivés aminoalkoxyphényle selon la revendication 1 dans laquelle $R_1$ et $R_2$ représentent chacun l'hydrogène.

6. Dérivés aminoalkoxyphényle selon la revendication 1 dans laquelle $R_3$, et $R'_3$ et $R''_3$ représentent l'hydrogène ou méthoxy.

7. Dérivés aminoalkoxyphényle selon la revendication 1 dans laquelle R représente le groupement isopropyle ou cyclopropyle.

8. Dérivés aminoalkoxyphényle selon la revendication 1 dans laquelle le sel pharmaceutiquement acceptable est l'oxalate ou le chlorhydrate.

9. Dérivés aminoalkoxyphényle selon la revendication 1 choisis parmi les :
{[(diméthoxy-6,7 tétrahydro-1,2,3,4 N-isoquinoléinyl-2)-3 propoxy]-4 benzènesulfonyl}-1 isopropyl-2 indolizine,
ethyl-2 {[(diméthoxy-6,7 tétrahydro-1,2,3,4 N-isoquinoléinyl-2)-3 propoxy]-4 benzènesulfonyl}-3 benzothiophène,
{[(diméthoxy-6,7 tétrahydro-1,2,3,4 N-isoquinoléinyl-2)-3 propoxy]-4 benzènesulfonyl}-4 isopropyl-5 oxazole,
{[(diméthoxy-6,7 tétrahydro-1,2,3,4 N-isoquinoléinyl-2)-3 propoxy]-4 benzènesulfonyl}-4 éthyl-5 méthyl-1 pyrazole,
{[(diméthoxy-6,7 tétrahydro-1,2,3,4 N-isoquinoléinyl-2)-3 propoxy]-4 benzènesulfonyl}-4 isopropyl-5 méthyl-1 pyrazole,
{[(diméthoxy-6,7 tétrahydro-1,2,3,4 N-isoquinoléinyl-2)-3 propoxy]-4 benzènesulfonyl}-3 isopropyl-2 méthyl-1 indole,
{[(diméthoxy-6,7 tétrahydro-1,2,3,4 N-isoquinoléinyl-2)-4 butoxy]-4 benzènesulfonyl}-1 isopropyl-2 indolizine,
isopropyl-2 [(diméthoxy-6,7 tétrahydro-1,2,3,4 N-isoquinoléinyl-2)-3 propoxy]-4 benzènesulfonyl-3 benzofuranne,
ethyl-2 [(diméthoxy-6,7 tétrahydro-1,2,3,4 N-isoquinoléinyl-2)-3 propoxy]-4 benzènesulfonyl-3 benzofuranne,
[(diméthoxy-6,7 tétrahydro-1,2,3,4 N-isoquinoléinyl) -2 propoxy]-4 phényl (isopropyl-2 phényl)sulfone,
[N-(pentahydro-5,6,7,8,9 benzocycloheptényl-9) N-méthylamino-3 propoxy]-4 benzènesulfonyl-3 isopropyl-2 méthyl-1 indole,
[N-méthyl N-(tétrahydro-1,2,3,4 naphtyl-1)amino-3 propoxy]-4 benzènesulfonyl-3 isopropyl-2 méthyl-1 indole,
[N-méthyl N-(diméthoxy-6,7 tétrahydro-1,2,3,4 naphtyl -1)amino-3 propoxy]-4 benzènesulfonyl-1 isopropyl-2 indolizine,
[N-méthyl N-(tétrahydro-1,2,3,4 naphtyl-1)amino-3 propoxy]-4 benzènesulfonyl-3 isopropyl-2 pyrazolo[1,-5-a]pyridine
et leurs sels pharmaceutiquement acceptables.

10. Procédé de préparation de dérivés aminoalkoxyphényle selon la revendication 1 dans laquelle A est tel que défini dans la revendication 1 et B représente un groupement -S- ou $-SO_2-$, caractérisé en ce que l'on condense, en présence d'un accepteur d'acide, dans un solvant polaire ou non polaire et à une température comprise entre la température ambiante et la température de reflux, un dérivé alcoxy-4 phényle de formule générale :

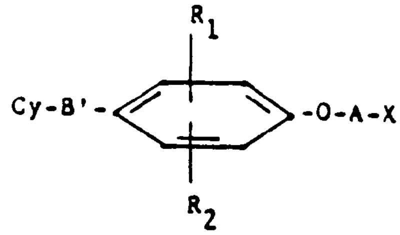

(2)

dans laquelle B' représente un groupement -S- ou $-SO_2-$; Cy, $R_1$ et $R_2$ ont la même signification que dans la revendication 1; A a la même signification que précédemment et X représente un atome d'halogène ou un groupement alkylsulfonyloxy ayant de 1 à 4 atomes de carbone ou arylsulfonyloxy ayant de 6 à 10 atomes de carbone, avec une amine de formule générale :

H - Am (3)

dans laquelle Am a la même signification que dans la revendication 1, pour former le dérivé désiré que l'on fait réagir, si on le désire, avec un acide organique ou inorganique pour former un sel pharmaceutiquement acceptable de ce dérivé.

**11.** Procédé de préparation de dérivés aminoalkoxyphényle selon la revendication 1 dans laquelle A est tel que défini dans la revendication 1 et B représente un groupement -S- ou $-SO_2-$, caractérisé en ce que l'on fait réagir, en présence d'un agent basique, un dérivé hydroxy-4 phényle de formule générale :

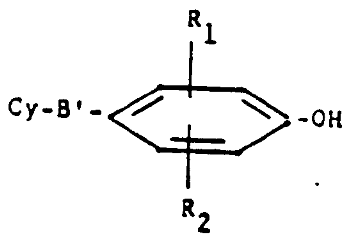

(4)

dans laquelle B' représente un groupement -S- ou $-SO_2-$ et Cy, $R_1$ et $R_2$ ont la même signification que dans la revendication 1 avec un composé de formule générale :

X - A - Am (52)

dans laquelle X représente un atome d'halogène ou un groupement alkylsulfonyloxy ayant de 1 à 4 atomes de carbone ou arylsulfonyloxy ayant de 6 à 10 atomes de carbone, A est tel que défini ci-dessus et Am a la même valeur que dans la revendication 1, la réaction ayant lieu au reflux et dans un solvant approprié pour obtenir le dérivé désiré que l'on peut, si on le désire, faire réagir avec un acide organique ou inorganique approprié pour former un sel pharmaceutiquement acceptable.

**12.** Procédé de préparation de dérivés aminoalkoxyphényle selon la revendication 1 dans laquelle A représente le radical hydroxy-2 propylène éventuellement substitué et 3 représente un groupement -S- ou $-SO_2-$, caractérisé en ce que l'on traite au reflux un dérivé oxyranylméthoxy de formule générale :

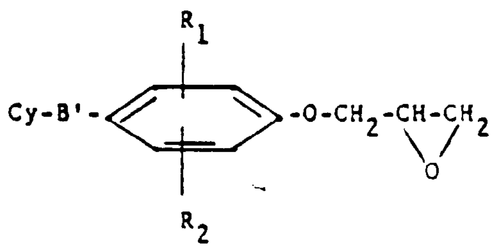

(57)

dans laquelle B' représente un groupement -S- ou $-SO_2-$ et Cy, $R_1$ et $R_2$ ont la même signification que dans la revendication 1, avec une amine de formule générale :

H - Am (3)

dans laquelle Am a la même signification que dans la revendication 1 et ce, dans un solvant polaire ou dans un excès de ladite amine pour donner :

- le dérivé désiré, sous forme de base libre, dans lequel A représente un radical hydroxy-2 propylène
- un dérivé aminoalkoxyphényle que l'on peut faire réagir avec un halogénure d'alkyle ayant de 1 à 4 atomes de carbone et en présence d'une base forte, ce qui fournit le dérivé désiré sous forme de base libre, dans lequel A représente un radical hydroxy-2 propylène dans lequel l'hydroxy est substitué par un alkyle ayant de 1 à 4 atomes de carbone,

  le dérivé aminoalkoxyphényle ainsi obtenu pouvant, si on le désiré, être mis en réaction avec un acide organique ou inorganique pour former un sel pharmaceutiquement acceptable.

**13.** Procédé de préparation de dérivés arninoalkoxyphényle selon la revendication 1 dans laquelle B représente un groupement -SO-, caractérisé en ce que l'on traite, avec un agent oxydant, un sulfure de formule générale :

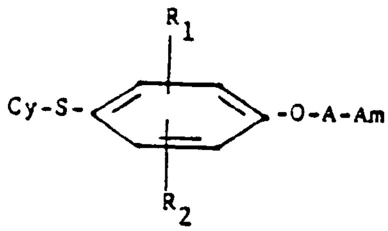

dans laquelle Cy, $R_1$, $R_2$, A et Am ont la même signification que dans la revendication 1, ce sulfure étant sous forme de base libre ou de sel, pour former le dérivé désiré sous forme de base libre ou de sel, et que l'on traite le sel ainsi obtenu avec un agent basique pour fournir le dérivé désiré sous forme de base libre, la base libre ainsi obtenue étant, si on le désire, mise en réaction avec un acide organique ou inorganique pour former un sel pharmaceutiquement acceptable de ce dérivé.

**14.** Procédé selon la revendication 13, caractérisé en ce que l'agent oxydant est le périodate de sodium, le permanganate de potassium ou l'acide chloro-3 perbenzoique.

**15.** Compositions pharmaceutiques ou vétérinaires contenant, comme principe actif, au moins un dérivé aminoalkoxyphényle selon l'une des revendications 1 à 9, en association avec un véhicule pharmaceutique ou un excipient approprié.

**16.** Compositions pharmaceutiques ou vétérinaires selon la revendication 15 pour le traitement de syndromes pathologiques du système cardiovasculaire, contenant de 50mg à 500mg de principe actif.

**17.** Utilisation d'au moins un dérivé aminoalkoxyphényle selon l'une quelconque des revendications 1 à 9 pour la fabrication d'un médicament.

**18.** Utilisation d'au moins un dérivé alkoxyphényle selon l'une quelconque des revendications 1 à 9 pour la fabrication d'un médicament destiné au traitement de syndromes pathologiques du systéme cardiovasculaire.

**19.** Utilisation d'au moins un dérivé aminoalkoxyphényle selon l'une quelconque des revendications 1 à 9, pour la fabrication d'un médicament destiné au traitement d'affections pathologiques occulaires.

**Revendications pour l'Etat contractant suivant : ES**

**1.** Procédé de préparation de dérivés aminoalkoxyphényle correspondant à la formule générale :

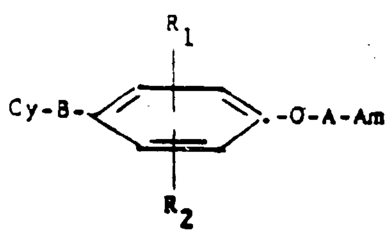

(1)

ainsi que leurs sels pharmaceutiquement acceptables dans laquelle :

B représente un groupement -S-, -SO- ou $-SO_2-$,

$R_1$ et $R_2$, qui sont identiques ou différents, représentent chacun l'hydrogène, le radical méthyle ou éthyle ou un halogène,

A représente un radical alkylène, linéaire ou ramifié en $C_2$-$C_5$ ou le radical hydroxy-2 propylène dans lequel l'hydroxy est éventuellement substitué par un radical alkyle en $C_1$-$C_4$,

Am représente un groupement :

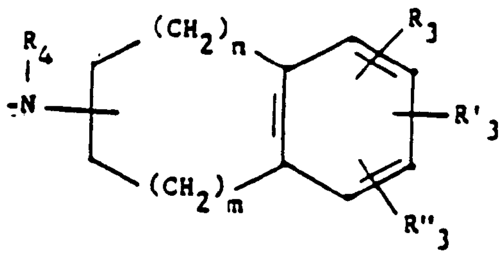

(D).

ou

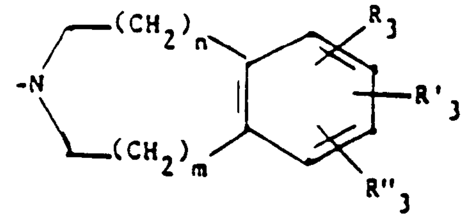

(E)

dans lequel : $R_3$, $R'_3$ et $R''_3$, qui sont identiques ou différents, représentent chacun l'hydrogène, un atome d'halogène, un groupement alkyle en $C_1$-$C_4$ ou un groupement alkoxy on $C_1$-$C_4$,

$R_4$ représente l'hydrogène ou un radical alkyle en $C_1$-$C_8$,

n et m, identiques ou différents, représentent chacun 0, 1, 2 ou 3,

Cy représente un groupement de formule :

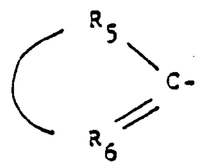

(F)

ou

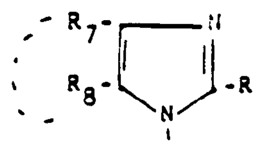

(G)

R représente l'hydrogène, un radical alkyle en $C_1$-$C_8$, un radical cycloalkyle en $C_3$-$C_6$, un radical benzyle ou un radical phényle éventuellement substitué par un ou plusieurs substituants, qui peuvent être identiques ou différents, choisis parmi des atomes d'halogène ou parmi des groupements alkyle en $C_1$-$C_4$, alkoxy en $C_1$-$C_4$ ou nitro,

$R_5$ et $R_6$ sont pris ensemble, avec l'atome de carbone auquel ils sont attachés, pour former :

- un groupement carbocyclique mono- ou di-cyclique éventuellement aromatique ayant de 5 à 10 atomes de carbone et éventuellement substitué par un groupement R en position α par rapport au groupement méthyne,
- un groupement hétérocyclique à 5 sommets éventuellement aromatique, les hétéroatomes ou hétérogroupes étant choisis parmi des groupements O, S, N,

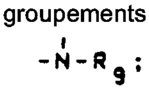

O et N; O et

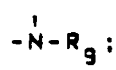

S et

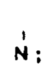

S et

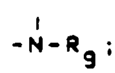

N et N; N et

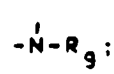

le groupement hétérocyclique étant éventuellement substitué par un groupement R tel que défini ci-

dessus en position α par rapport au groupement méthyne et éventuellement substitué par un ou deux groupements choisis parmi des groupements alkyle en $C_1$-$C_4$ en phényle,

- un groupement hétérocyclique ayant de 6 à 10 sommets, mono ou di-cyclique et éventuellement aromatique, les hétéroatomes ou hétérogroupes étant choisis parmi des groupements O, S, N,

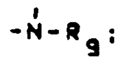

O et N; O et

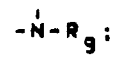

S et N; S et

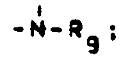

N et N; N et

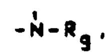

le groupement hétérocyclique étant éventuellement substitué par un groupement R tel que défini ci-dessus en position par rapport au groupement méthyne, $R_7$ et $R_8$, qui sont identiques ou différents, représentent chacun l'hydrogène, un radical alkyle inférieur en $C_1$-$C_4$ ou un radical phényle ou lorsqu'ils sont pris ensemble, avec les atomes de carbone auxquels ils sont attachés, représentent un carbocycle à 6 sommets éventuellement aromatique.

$R_9$ représente l'hydrogène, un groupement alkyle en $C_1$-$C_4$, phényle, benzyle ou halogénobenzyle, caractérisé en ce que

A/ Quand A est tel que défini précédemment et B représente un groupement -S- ou -$SO_2$-, soit l'on condense, en présence d'un accepteur d'acide, dans un solvant polaire ou non polaire et à une température comprise entre la température ambiante et la température de reflux, un dérivé alcoxy-4 phényle de formule générale :

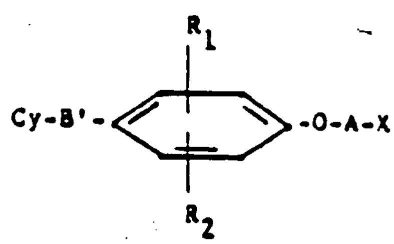

(2)

dans laquelle B' représente un groupement -S- ou -$SO_2$-; Cy, $R_1$ et $R_2$ ont la même signification que dans la revendication 1; A a la même signification que précédemment et X représente un atome d'halogène ou un groupement alkysulfonyloxy ayant de 1 à 4 atomes de carbone ou arylsulfonyloxy ayant de 6 à 10 atomes de carbone, avec une amine de formule générale :

H - Am (3)

dans laquelle Am a la même signification que précédemment,

- soit l'on fait réagir, en présence d'un agent basique, un dérivé hydroxy-4 phényle de formule générale :

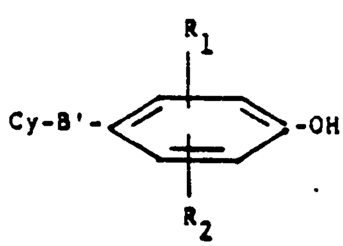

(4)

dans laquelle B' représente un groupement -S- ou $-SO_2-$ et Cy, $R_1$ et $R_2$ ont la même signification que précédemment avec un composé de formule générale :

X - A - Am (52)

dans laquelle X représente un atome d'halogène ou un groupement alkysulfonyloxy ayant de 1 à 4 atomes de carbone ou arylsulfonyloxy ayant de 6 à 10 atomes de carbone, A est tel que défini ci-dessus et Am a la même valeur que précédemment, la réaction ayant lieu au reflux et dans un solvant approprié.

B/ Quand A représente le radical hydroxy-2 propylène éventuellement substitué et B représente un groupement -S- ou $-SO_2-$, l'on traite au reflux un dérivé oxyranylméthoxy de formule générale :

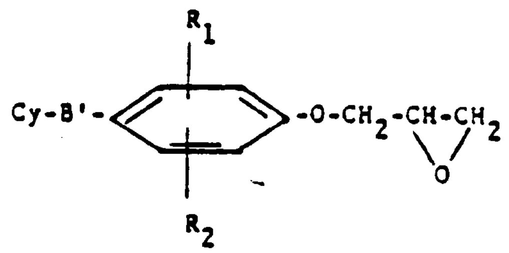

(57)

dans laquelle B' représente un groupement -S- ou $-SO_2-$ et Cy, $R_1$ et $R_2$ ont la même signification que précédemment, avec une amine de formule générale :

H - Am (3)

dans laquelle Am a la même signification que précédemment et ce, dans un solvant polaire ou dans un excès de ladite amine pour donner :

- le dérivé désiré, sous forme de base libre, dans lequel A représente un radical hydroxy-2 propylène
- un dérivé aminoalkoxyphényle que l'on peut faire réagir avec un halogénure d'alkyle ayant de 1 à 4 atomes de carbone et en présence d'une base forte, ce qui fournit le dérivé désiré sous forme de base libre, dans lequel A représente un radical hydroxy-2 propylène dans lequel l'hydroxy est substitué par un alkyle ayant de 1 à 4 atomes de carbone, et

C/ Quand B représente un groupement -SO-, l'on traite avec un agent oxydant, un sulfure de formule générale:

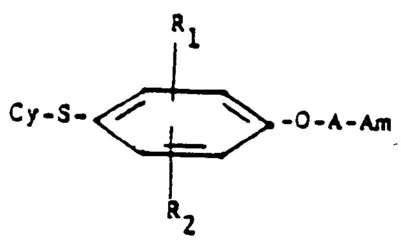

dans laquelle Cy, $R_1$,$R_2$, A et Am ont la même signification que précédemment, ce sulfure étant sous forme de base libre ou de sel pour former le dérivé désiré sous forme de base libre ou de sel, et que l'on traite le sel ainsi obtenu avec un agent basique pour fournir le dérivé désiré sous forme de base libre, le dérivé aminoalkoxyphényle ainsi obtenu étant, si on le désire, mis en réaction avec un acide organique ou inorganique pour former un sel pharmaceutiquement acceptable de ce dérivé.

**2.** Procédé selon la revendication 1, caractérisé en ce que l'agent oxydant est le périodate de sodium, le permanganate de potassium ou l'acide chloro-3 perbenzoique.

**3.** Procédé selon la revendication 1, caractérisé en ce que l'on prépare les dérivés aminoalkoxyphényle de formule générale (1) dans laquelle Cy représente un groupement phényle, cyclohexényle, indényle, naphtyle, dihydronaphtyle, pyridyle, dihydropyridyle, furyle, dihydrofuryle, thiényle, dihydrothiényle, pyrrolyle, dihydropyrrolyle, pyrazolyle, imidazolyle, pyrimidyle, pyrazinyle, pyridazinyle, oxazolyle, isoxazolyle, thiazolyle, benzofuryle, benzothiényle, indolyle, benzimidazolyle, benzoxasolyle, quinoléinyle, benzisoxazolyle, cinnolinyle, quinoxalynyle, quinazolinyle, indolizinyle, thiénopyridyle, tétrahydrothiénopyridyle, pyrrolopyridyle, pyrazolopyridyle, pyrrolopyridazinyle, imidazopyridyle.

**4.** Procédé selon la revendication 1, caractérisé en ce que l'on prépare des dérivés aminoalkoxyphényle de

formule générale (1) dans laquelle Cy représente un groupement indolizinyle, benzofuryle, benzothiényle, indolyle, oxazolyle, pyrazolyle, phényle, pyrazolo [1,5-a]pyridyle ou imidazo [1,2-a] pyridyle.

5. Procédé selon la revendication 1 caractérisé en ce que l'on prépare des dérivés aminoalkoxyphényle de formule générale (1) dans laquelle B représente un groupement $-SO_2-$.

6. Procédé selon la revendication 1, caractérisé en ce que l'on prépare des dérivés aminoalkoxyphényle de formule générale (1) dans laquelle $R_1$ et $R_2$ représentent chacun l'hydrogène.

7. Procédé selon la revendication 1 caractérisé en ce que l'on prépare des dérivés aminoalkoxyphényle de formule générale (1) dans laquelle $R_3$, $R'_3$ et $R''_3$ représentent l'hydrogène ou méthoxy.

8. Procédé selon la revendication 1 caractérisé en ce que l'on prépare des dérivés aminoalkoxyphényle de formule générale 1 dans laquelle R représente le groupement isopropyle ou cyclopropyle.

9. Procédé selon la revendication 1, caractérisé en ce que l'on prépare des dérivés aminoalkoxyphényle de formule générale (1) dans laquelle le sel pharmaceutiquement acceptable est l'oxalate ou le chlorhydrate.

10. Procédé selon la revendication 1 caractérisé en ce que l'on prépare des dérivés aminoalkoxyphényle choisis parmi les :
{[(diméthoxy-6¯7 tétrahydro-1,2,3,4 N-isoquinoléinyl-2)-3 propoxy]-4 benzènesulfonyl}-1 isopropyl-2 indolizine, ethyl-2 {[(diméthoxy-6,7 tétrahydro-1,2,3,4 N-isoquinoléinyl-2)-3 propoxy]-4 benzènesulfony}-3 benzothiophène.
{[(diméthoxy-6,7 tétrahydro-1,2,3,4 N-isoquinoléinyl-2)-3 propoxy]-4 benzènesulfonyl}-4 isopropyl-5 oxazole,
{[(diméthoxy-6,7 tétrahydro-1,2,3,4 N-isoquinoléinyl-2)-3 propoxy]-4 benzènesulfonyl}-4 éthyl-5 méthyl-1 pyrazole,
{[(diméthoxy-6,7 tétrahydro-1,2,3,4 N-isoquinoléinyl-2)-3 propoxy]-4 benzènesulfonyl}-4 isopropyl-5 méthyl-1 pyrazole,
{[(diméthoxy-6,7 tétrahydro-1,2,3,4, N-isoquinoléinyl-2)-3 propoxy]-4 benzènesulfonyl}-3 isopropyl-2 méthyl-1 indole,
{[(diméthoxy-6,7 tétrahydro-1,2,3,4 N-isoquinoléinyl-2)-4 butoxy]-4 benzènesulfonyl}-1 isopropyl-2 indolizine,
isopropyl-2 [(diméthyoxy-6,7 tétrahydro-1,2,3,4 N-isoquinoléinyl-2)-3 propoxy]-4 benzènesulfonyl-3 benzofuranne,
ethyl-2 [(diméthoxy-6,7 tétrahydro-1,2,3,4 N-isoquinoléinyl-2)-3 propoxy]-4 benzènesulfonyl-3 benzofuranne,
[(diméthoxy-6,7 tétrahydro-1,2,3,4 N-isoquinoléinyl) -2 propoxy]-4 phényl (isopropyl-2 phényl)sulfone.
[N-(pentahydro-5,6,7,8,9 benzocycloheptényl-9) N-méthylamino-3 propoxy]-4 benzènosulfonyl-3 isopropyl-2 méthyl-1 indole,
[N-méthyl N-(tétrahydro-1,2,3,4 naphtyl-1)amino-3 propoxy]-4 benzènesulfonyl-3 isopropyl-2 méthyl-1 indole,
[N-méthyl N-(diméthoxy-6,7 tétrahydro-1,2,3,4 naphtyl -1)amino-3 propoxy]-4 benzènesulfonyl-1 isopropyl-2 indolizine,
[N-méthyl N-(tétrahydro-1,2,3,4 naphtyl-1)amino-3 propoxy]-4 benzènesulfonyl-3 isopropyl-2 pyrazolo[1,-5-a]pyridine
et leurs sels pharmaceutiquement acceptables.

11. Procédé de préparation d'une composition pharmaceutique ou vétérinaire caractérisé en ce que l'on ajoute comme principe actif au moins un dérivé aminoalkoxyphényle préparé suivant l'une quelconque des revendications 1 à 10 à un véhicule pharmaceutique ou un récipient approprié.

12. Procédé de préparation d'une composition pharmaceutique ou vétérinaire selon la revendication 11 pour le traitement de syndromes pathologiques du système cardiovasculaire contenant de 50mg à 500mg de principe actif.

13. Procédé de préparation d'une composition pharmaceutique ou vétérinaire selon la revendication 11 pour le traitement d'affections pathologiques occulaires.

**Revendications pour l'Etat contractant suivant : GR**

1. Dérivés aminoalkoxyphényle correspondant à la formule générale :

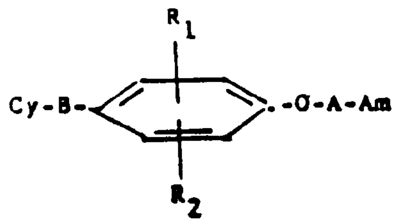

(I)

ainsi que leurs sels pharmaceutiquement acceptables dans laquelle :

B représente un groupement -S-, -SO- ou $-SO_2-$,

$R_1$ et $R_2$, qui sont identiques ou différents, représentent chacun l'hydrogène, le radical méthyle ou éthyle ou un halogène,

A représente un radical alkyléne, linéaire ou ramifié en $C_2$-$C_5$ ou le radical hydroxy-2 propylène dans lequel l'hydroxy est éventuellement substitué par un radical alkyle en $C_1$-$C_4$,

Am représente un groupement :

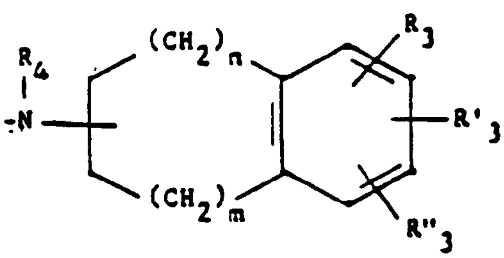

(D).

ou

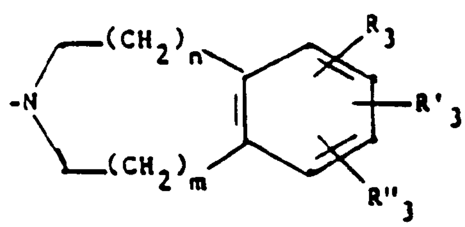

(E)

dans lequel : $R_3$, $R'_3$ et $R''_3$, qui sont identiques ou différents, représentent chacun l'hydrogène, un atome d'halogène, un groupement alkyle en $C_1$-$C_4$ ou un groupement alkoxy en $C_1$-$C_4$,

$R_4$ représente l'hydrogène ou un radical alkyle en $C_1$-$C_8$,

n et m, identiques ou différents, représentent chacun 0, 1, 2 ou 3,

Cy représente un groupement de formule :

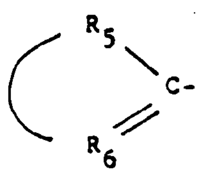

(F)

ou

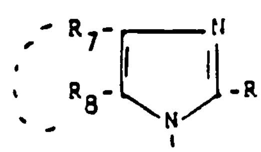

(G)

R représente l'hydrogène, un radical alkyle en $C_1$-$C_8$, un radical cycloalkyle en $C_3$-$C_6$, un radical benzyle ou un radical phényle éventuellement substitué par un ou plusieurs substituants, qui peuvent être identiques ou différents, choisis parmi des atomes d'halogène ou parmi des groupements alkyle en $C_1$-$C_4$, alkoxy en $C_1$-$C_4$ ou nitro,

$R_5$ et $R_6$ sont pris ensemble, avec l'atome de carbone auquel ils sont attachés, pour former :

- un groupement carbocyclique mono- ou di-cyclique éventuellement aromatique ayant de 5 à 10 atomes de carbone et éventuellement substitué par un groupement R en position $\alpha$ par rapport au groupement méthyne,
- un groupement hétérocyclique à 5 sommets éventuellement aromatique, les hétéroatomes ou hétérogroupes étant choisis parmi des groupements O, S, N,

$-\overset{|}{N}-R_9$ ;

O et N; O et

$-\overset{|}{N}-R_9$ ;

S et

$\overset{|}{N}$ ;

S et

$-\overset{|}{N}-R_9$ ;

N et N; N et

$-\overset{|}{N}-R_9$ ;

le groupement hétérocyclique étant éventuellement substitué par un groupement R tel que défini ci-dessus en position $\alpha$ par rapport au groupement méthyne et éventuellement substitué par un ou deux groupements choisi parmi des groupements alkyle en $C_1$-$C_4$ et phényle,

- un groupement hétérocyclique ayant de 6 à 10 sommets, mono ou di-cyclique et éventuellement aromatique, les hétéroatomes ou hétérogroupes étant choisis parmi des groupements O, S, N,

$-\overset{|}{N}-R_9$ ;

O et N; O et

$-\overset{|}{N}-R_9$ ;

S et N; S et

$-\overset{|}{N}-R_9$ ;

N et N; N et

$-\overset{|}{N}-R_9$ ,

le groupement hétérocyclique étant éventuellement substitué par un groupement R tel que défini ci-dessus en position par rapport au groupement méthyne,

$R_7$ et $R_8$ qui sont identiques ou différents, représentent chacun l'hydrogène, un radical alkyle inférieur en $C_1$-$C_4$ ou un radical phényle ou lorsqu'ils sont pris ensemble, avec les atomes de carbone auxquels ils sont attachés, représentent un carbocycle à 6 sommets éventuellement aromatique,

$R_9$ représente l'hydrogène, un groupement alkyle en $C_1$-$C_4$, phényle, benzyle ou halogénobenzyle.

**2.** Dérivés aminoalkoxyphényle selon la revendication 1 dans laquelle Cy représente un groupement phényle, cyclohexényle, indényle, naphtyle, dihydronaphtyle, pyridyle, dihydropyridyle, furyle, dihydrofuryle, thiényle, dihydrothiényle, pyrrolyle, dihydropyrrolyle, pyrazolyle, imidazolyle, pyrimidyle, pyrazinyle, pyridazinyle, oxazolyle, isoxazolyle, thiazolyle, benzofuryle, benzothiényle, indolyle, benzimidazolyle, benzoxasolyle, quinoléinyle, benzisoxazolyle, cinnolinyle,quinoxalinyle, quinazolinyle, indolizinyle, thiénopyridyle, tétrahydrothiénopyridyle, pyrrolopyridyle, pyrazolopyridyle, pyrrolopyridazinyle, imidazopyridyle.

**3.** Dérivés aminoalkoxyphényle selon la revendication 1 dans laquelle Cy représente un groupement indolizinyle, benzofuryle, benzothiényle, indolyle, oxazolyle, pyrazolyle, phényle, pyrazolo[1,5-a]pyridyle ou imidazo[1,2-a]pyridyle.

**4.** Dérivés aminoalkoxyphényle selon la revendication 1 dans laquelle B représente un groupement $-SO_2-$.

5. Dérivés aminoalkoxyphényle selon la revendication 1 dans laquelle $R_1$ et $R_2$ représentent chacun l'hydrogène.

6. Dérivés aminoalkoxyphényle selon la revendication dans laquelle $R_3$, $R'_3$ et $R''_3$ représentent l'hydrogène ou méthoxy.

7. Dérivés aminoalkoxyphényle selon la revendication 1 dans laquelle R représente le groupement isopropyle ou cyclopropyle.

8. Dérivés aminoalkoxyphényle selon la revendication 1 dans laquelle le sel pharmaceutiquement acceptable est l'oxalate ou le chlorhydrate.

9. Dérivés aminoalkoxyphényle selon la revendication 1 choisis parmi les :
{[(diméthoxy-6,7 tétrahydro-1 2,3,4 N-isoquinoléinyl-2)-3 propoxy]-4 benzènesulfonyl}-1 isopropyl-2 indolizine,
ethyl-2 {[(diméthoxy-6,7 tétrahydro-1,2,3,4 N-isoquinoléinyl-2)-3 propoxy]-4 benzénesulfonyl}-3 benzothiophène,
{[(diméthoxy-6,7 tétrahydro-1,2,3,4 N-isoquinoléinyl-2)-3 propoxy]-4 benzènesulfonyl}-4 isopropyl-5 oxazole,
{[(diméthoxy-6,7 tétrahydro-1,2,3,4 N-isoquinoléinyl-2)-3 propoxy]-4 benzènesulfonyl}-4 éthyl-5 méthyl-1 pyrazole,
{[(diméthoxy-6,7 tétrahydro-1,2,3,4 N-isoquinolèinyl-2)-3 propoxy]-4 benzènesulfonyl}-4 isopropyl-5 méthyl-1 pyrazole,
{[(diméthoxy-6,7 tétrahydro-1,2,3,4 N-isoquinoléinyl-2)-3 propoxy]-4 benzènesulfonyl}-3 isopropyl-2 méthyl-1 indole,
{[(diméthoxy-6,7 tétrahydro-1,2,3,4 N-isoquinoléinyl-2)-4 butoxy]-4 benzènesulfonyl}-1 isopropyl-2 indolizine,
isopropyl-2 [(diméthoxy-6,7 tétrahydro-1,2,3,4 N-isoquinoléinyl-2)-3 propoxy]-4 benzènesulfonyl-3 benzofuranne,
ethyl-2 [(diméthoxy-6,7 tétrahydro-1,2,3,4 N-isoquinoléinyl-2)-3 propoxy]-4 benzènesulfonyl-3 benzofuranne,
[(diméthoxy-6,7 tétrahydro-1,2,3,4 N-isoquinoléinyl) -2 propoxy]-4 phényl (isopropyl-2 phényl)sulfone,
[N-(pentahydro-5,6,7,8,9 benzocycloheptényl-9) N-méthylamino-3 propoxy]-4 benzènesulfonyl-3 isopropyl-2 méthyl-1 indole,
[N-méthyl N-(tétrahydro-1,2,3,4 naphtyl-1)amino-3 propoxy]-4 benzènesulfonyl-3 isopropyl-2 méthyl-1 indole,
[N-méthyl N-(diméthoxy-6,7 tétrahydro-1,2,3,4 naphtyl -1)amino-3 propoxy]-4 benzènesulfonyl-1 isopropyl-2 indolizine,
[N-méthyl N-(tétrahydro-1,2,3,4 naphtyl-1)amino-3 propoxy]-4 benzènesulfonyl-3 isopropyl-2 pyrazolo[1,-5-a]pyridine
et leurs sels pharmaceutiquement acceptables.

10. Procédé de préparation de dérivés aminoalkoxyphényle selon la revendication 1 dans laquelle A est tel que défini dans la revendication 1 et B représente un groupement -S- ou $-SO_2-$, caractérisé en ce que l'on condense, en présence d'un accepteur d'acide, dans un solvant polaire ou non polaire et à une température comprise entre la température ambiante et la température de reflux, un dérivé alcoxy-4 phényle de formule générale :

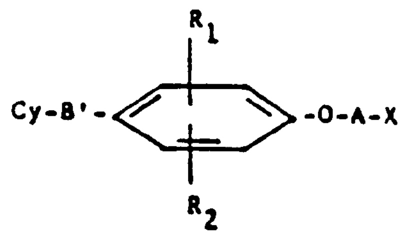

(2)

dans laquelle B' représente un groupement -S- ou $-SO_2-$; Cy, $R_1$ et $R_2$ ont la même signification que dans

la revendication 1; A a la même signification que précédemment et X représente un atome d'halogène ou un groupement alkylsulfonyloxy ayant de 1 à 4 atomes de carbone ou arylsulfonyloxy ayant de 6 à 10 atomes de carbone, avec une amine de formule générale :

H - Am (3)

dans laquelle Am a la même signification que dans la revendication 1, pour former le dérivé désiré que l'on fait réagir, si on le désire, avec un acide organique ou inorganique pour former un sel pharmaceutiquement acceptable de ce dérivé.

**11.** Procédé de préparation de dérivés aminoalkoxyphényle selon la revendication 1 dans laquelle A est tel que défini dans la revendication 1 et B représente un groupement -S- ou $-SO_2-$, caractérisé en ce que l'on fait réagir, en présence d'un agent basique, un dérivé hydroxy-4 phényle de formule générale :

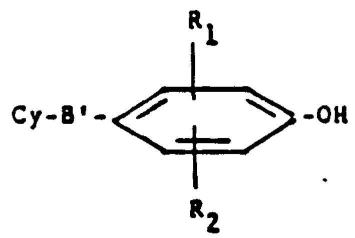

(4)

dans laquelle B' représente un groupement -S- ou $-SO_2-$ et Cy, $R_1$ et $R_2$ ont la même signification que dans la revendication 1 avec un composé de formule générale :

X - A - Am (52)

dans laquelle X représente un atome d'halogène ou un groupement alkylsulfonyloxy ayant de 1 à 4 atomes de carbone ou arylsulfonyloxy ayant de 6 à 10 atomes de carbone. A est tel que défini ci-dessus et Am a la même valeur que dans la revendication 1, la réaction ayant lieu au reflux et dans un solvant approprié pour obtenir le dérivé désiré que l'on peut, si on le désire, faire réagir avec un acide organique ou inorganique approprié pour former un sel pharmaceutiquement acceptable.

**12.** Procédé de préparation de dérivés cycloaminoalkoxyphényle selon la revendication 1 dans laquelle A représente le radical hydroxy-2 propylène éventuellement substitué et B représente un groupement -S- ou $-SO_2-$, caractérisé en ce que l'on traite au reflux un dérivé oxyranylméthoxy de formule générale :

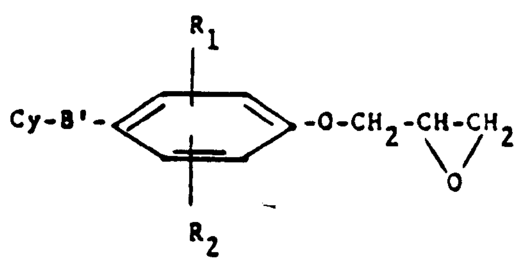

(57)

dans laquelle B' représente un groupement -S- ou $-SO_2-$ et Cy, $R_1$ et $R_2$ ont la même signification que dans la revendication 1, avec une amine de formule générale :

H - Am (3)

dans laquelle Am a la même signification que dans la revendication 1 et ce, dans un solvant polaire ou dans un excès de ladite amine pour donner :

- le dérivé désiré, sous forme de base libre, dans lequel A représente un radical hydroxy-2 propylène
- un dérivé aminoalkoxyphényle que l'on peut faire réagir avec un halogénure d'alkyle ayant de 1 à 4 atomes de carbone et en présence d'une base forte, ce qui fournit le dérivé désiré sous forme de base libre, dans lequel A représente un radical hydroxy-2 propylène dans lequel l'hydroxy est substitué par un alkyle ayant de 1 à 4 atomes de carbone,

  le dérivé aminoalkoxyphényle ainsi obtenu pouvant, si on le désiré, être mis en réaction avec un acide organique ou inorganique pour former un sel pharmaceutiquement acceptable.

**13.** Procédé de préparation de dérivés aminoalkoxyphényle selon la revendication 1 dans laquelle B représente un groupement -SO-, caractérisé en ce que l'on traite, avec un agent oxydant, un sulfure de formule générale :

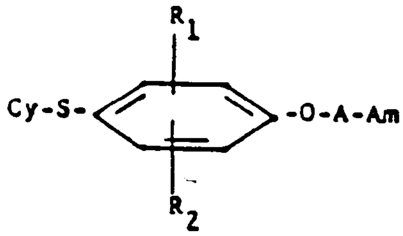

dans laquelle Cy, $R_1$, $R_2$, A et Am ont la même signification que dans la revendication 1, ce sulfure étant sous forme de base libre ou de sel, pour former le dérivé désiré sous forme de base libre ou de sel, et que l'on traite le sel ainsi obtenu avec un agent basique pour fournir le dérivé désiré sous forme de base libre, la base libre ainsi obtenue étant, si on le désire, mise en réaction avec un acide organique ou inorganique pour former un sel pharmaceutiquement acceptable de ce dérivé.

**14.** Procédé selon la revendication 13, caractérisé en ce que l'agent oxydant est le périodate de sodium, le permanganate de potassium ou l'acide chloro-3 perbenzoique.

**15.** Procédé de préparation de compositions pharmaceutiques ou vétérinaires caractérisé en ce que l'on additionne, au moins un dérivé aminoalkoxyphényle selon l'une des revendications 1 à 9, avec un véhicule pharmaceutique ou un excipient approprié.

**16.** Procédé de préparation de compositions pharmaceutiques ou vétérinaires selon la revendication 15 pour le traitement de syndromes pathologiques du système cardiovasculaire, contenant de 50mg à 500mg de principe actif.

**17.** Procédé de préparation de compositions pharmaceutiques ou vétérinaires selon la revendication15 pour la fabrication d'un médicament destiné au traitement d'affections pathologiques occulaires.

## Patentansprüche

## Patentansprüche für folgende Vertragsstaaten : CH, DE, DK, FR, GB, IT, LI, LU, NL, SE, AT

**1.** Aminoalkoxyphenylderivate der allgemeinen Formel

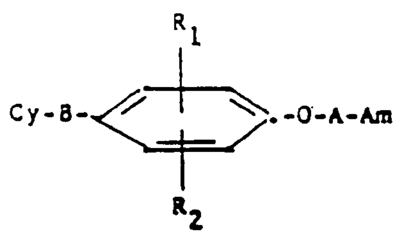

(1)

sowie deren pharmazeutisch annehmbaren Salze,
worin bedeuten:
B eine Gruppe -S-, -SO- oder $-SO_2-$,
$R_1$ und $R_2$, welche gleich oder verschieden sind, jeweils Wasserstoff, einen Methyl- oder Ethylrest oder ein Halogen,
A einen linearen oder verzweigten $C_2$-$C_5$-Alkylenrest oder den 2-Hydroxypropylenrest, worin das Hydroxy gegebenenfalls durch einen $C_1$-$C_4$-Alkylrest substituiert ist.
Am eine Gruppe:

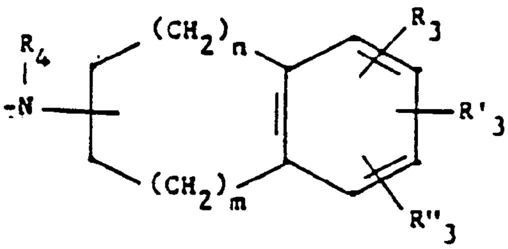

oder

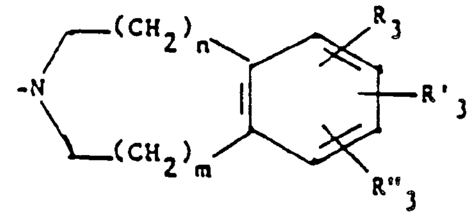

(D) (E)

worin $R_3$, $R'_3$ und $R''_3$, welche gleich oder verschieden sind, jeweils Wasserstoff, ein Halogenatom, eine $C_1$-$C_4$-Alkylgruppe oder eine $C_1$-$C_4$-Alkoxygruppe,

$R_4$ Wasserstoff oder einen $C_1$-$C_8$-Alkylrest,

n und m, welche gleich oder verschieden sind, jeweils 0, 1, 2 oder 3 bedeuten,

Cy eine Gruppe der Formel:

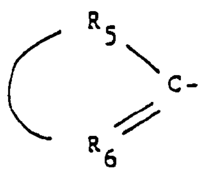

(F)

oder

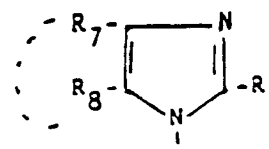

(G)

worin bedeuten:

R Wasserstoff, einen $C_1$-$C_8$-Alkylrest, einen $C_3$-$C_6$-Cycloalkylrest, einen Benzylrest oder Phenylrest, welcher gegebenenfalls durch einen oder mehrere Substituenten, welche gleich oder verschieden sein können, gewählt aus Halogenatomen oder $C_1$-$C_4$-Alkylgruppen, $C_1$-$C_4$-Alkoxygruppen oder Nitro, substituiert ist,

$R_5$ und $R_6$ sind mit dem Kohlenstoffatom, an welches sie gebunden sind, verknüpft zur Bildung:

- einer mono- oder dicyclischen, gegebenenfalls aromatischen, carbocyclischen Gruppe mit 5 bis 10 Kohlenstoffatomen, welche gegebenenfalls durch eine R-Gruppe in α-Stellung zur Methingruppe substituiert ist,
- einer gegebenenfalls aromatischen, heterocyclischen, 5-gliedrigen Gruppe, wobei die Heteroatome oder Heterogruppen aus den Gruppen O, S, N,

$-\overset{|}{N}-R_9$;

O und N; O und

$-\overset{|}{N}-R_9$;

S und

$\overset{|}{N}$;

S und

$-\overset{|}{N}-R_9$;

N und N; N und

$-\overset{|}{N}-R_9$;

gewählt sind, die heterocyclische Gruppe gegebenenfalls durch eine R-Gruppe,welche wie obenste

hend definiert ist, in α-Stellung zur Methingruppe und gegebenenfalls durch eine oder zwei aus $C_1$-$C_4$-Alkylgruppen und Phenyl gewählte Gruppen substituiert ist,

- einer gegebenenfalls aromatischen, mono- oder dicyclischen, 6- bis 10-gliedrigen heterocyclischen Gruppe, wobei die Heteroatome oder Heterogruppen aus den Gruppen O, S, N,

$$-\overset{|}{N}-R_9;$$

und N; O und

$$\cdot\overset{|}{N}-R_9;$$

S und N; S und

$$-\overset{|}{N}-R_9;$$

N und N; N und

$$-\overset{|}{N}-R_9,$$

gewählt sind, die heterocyclische Gruppe gegebenenfalls durch eine R-Gruppe, welche wie obenstehend definiert ist, in α-Stellung zur Methingruppe substituiert ist,

$R_7$ und $R_8$, welche gleich oder verschieden sind, jeweils Wasserstoff, einen $C_1$-$C_4$-Niederalkylrest oder einen Phenylrest, oder sie bilden, wenn sie mit den Kohlenstoffatomen, an welche sie gebunden sind, verknüpft sind, einen gegebenenfalls aromatischen, 6-gliedrigen Carbocyclus,

$R_9$ Wasserstoff, eine $C_1$-$C_4$-Alkylgruppe, Phenyl, Benzyl oder Halogenbenzyl.

2. Aminoalkoxyphenylderivate nach Anspruch 1, worin Cy eine Phenyl-. Cyclohexenyl-, Indenyl-, Naphthyl-, Dihydronaphthyl-, Pyridyl-, Dihydropyridyl-, Furyl-, Dihydrofuryl-, Thienyl-, Dihydrothienyl-, Pyrrolyl-, Dihydropyrrolyl-, Pyrazolyl-, Imidazolyl-, Pyrimidyl-, Pyrazinyl-, Pyridazinyl-, Oxazolyl-, Isoxazolyl-, Thiazolyl-, Benzofuryl-, Benzothienyl-, Indolyl-, Benzimidazolyl-, Benzoxazolyl-, Chinolinyl-, Benzisoxazolyl-, Cinnolinyl-, Chinoxalinyl-, Chinazolinyl-, Indolizinyl-, Thienopyridyl-, Tetrahydrothienopyridyl-, Pyrrolopyridyl-, Pyrazolopyridyl-, Pyrrolopyridazinyl- oder Imidazolpyridyl-Gruppe bedeutet.

3. Aminoalkoxyphenylderivate nach Anspruch 1, worin Cy eine Indolizinyl-, Benzofuryl-, Benzothienyl-, Indolyl-, Oxazolyl-, Pyrazolyl-, Phenyl-, Pyrazolo[1,5-a]pyridyl- oder Imidazo[1,2-a]pyridyl-Gruppe deutet.

4. Aminoalkoxyphenylderivate nach Anspruch 1, worin B die Gruppe -$SO_2$-bedeutet.

5. Aminoalkoxyphenylderivate nach Anspruch 1, worin $R_1$ und $R_2$ jeweils Wasserstoff bedeuten.

6. Aminoalkoxyphenylderivate nach Anspruch 1, worin $R_3$, $R'_3$ und $R''_3$ Wasserstoff oder Methoxy bedeuten.

7. Aminoalkoxyphenylderivate nach Anspruch 1, worin R eine Isopropyl- oder Cyclopropyl-Gruppe bedeutet.

8. Aminoalkoxyphenylderivate nach Anspruch 1, worin das pharmazeutisch annehmbare Salz das Oxalat oder das Chlorhydrat ist.

9. Aminoalkoxyphenylderivate nach Anspruch 1, gewählt aus:
1-{4-[3-(6,7-Dimethoxy-1,2,3,4-tetrahydro-2-N-isochinolinyl)-propoxy]-ben zolsulfonyl}-2-isopropyl-indolizin,
2-Ethyl-3-{4-[3-{6,7-dimethoxy-1,2,3,4-tetrahydro-2-N-isochinolinyl)-propoxy]-benzolsulfonyl}-benzothiophen,
4-{4-[3-(6,7-Dimethoxy-1,2,3,4-tetrahydro-2-N-isochinolinyl)-propoxy]-benzolsulfonyl}-5-isopropyl-oxazol,
4-{4-[3-(6,7-Dimethoxy-1,2,3,4-tetrahydro-2-N-isochinolinyl)-propoxy]-benzolsulfonyl}-5-ethyl-1-methyl-pyrazol,
4-{4-[3-(6,7-Dimethoxy-1,2,3,4-tetrahydro-2-N-isochinolinyl)-propoxy]-benzolsulfonyl}-5-isopropyl-1-methyl-pyrazol,
3-{4-[3-(6,7-Dimethoxy-1,2,3,4-tetrahydro-2-N-isochinolinyl)-propoxy]-benzolsulfonyl}-2-isopropyl-1-methyl-indol,
1-{4-[4-(6,7-Dimethoxy-1,2,3,4-tetrahydro-2-N-isochinolinyl)-butoxy]-benzolsulfonyl}-2-isopropyl-indolizin,
2-Isopropyl-3-{4-[3-(6,7-dimethoxy-1,2,3,4-tetrahydro-2-N-isochinolinyl)-propoxy]-benzolsulfonyl}-benzo-

furan,
2-Ethyl-3-{4-[3-(6,7-dimethoxy-1,2,3,4-tetrahydro-2-N-isochinolinyl)-propoxy]-benzolsulfonyl}-benzofuran,
{4-[2-(6,7-Dimethoxy-1,2,3,4-tetrahydro-N-isochinolinyl)-propoxy]-phenyl}-(2-isopropyl-phenyl)-sulfon,
3-{4-[N-(5,6,7,8,9-Pentahydro-9-benzocycloheptenyl)-3-N-methylamino-propoxy]-benzolsulfonyl}-2-isopropyl-1-methyl-indol,
3-{4-[N-Methyl-N-(1,2,3,4-tetrahydro-1-naphthyl)-3-amino-propoxy]benzolsulfonyl}-2-isopropyl-1-methyl-indol,
1-{4-[N-Methyl-N-(6,7-dimethoxy-1,2,3,4-tetrahydro-1-naphthyl)-3-aminopropoxy]-benzolsulfonyl}-2-isopropyl-indolizin,
3-{4-[N-Methyl-N-(1,2,3,4-tetrahydro-1-naphthyl)-3-amino-propoxy]-benzolsulfonyl}-2-isopropyl-pyrazolo[1,5-a]-pyridin,
und deren pharmazeutisch annehmbaren Salzen.

**10.** Verfahren zur Herstellung der Aminoalkoxyphenylderivate nach Anspruch 1, worin A wie in Anspruch 1 definiert ist und B eine Gruppe -S- oder -$SO_2$- bedeutet, **dadurch gekennzeichnet,** daß man in Gegenwart eines Säureakzeptors in einem polaren oder nichtpolaren Lösungsmittel bei einer Temperatur, welche zwischen Umgebungstemperatur und der Rückflußtemperatur liegt, ein 4-Alkoxyphenylderivat der allgemeinen Formel:

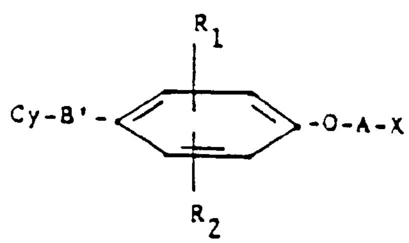

(2)

worin B' eine Gruppe -S- oder -$SO_2$- bedeutet; Cy, $R_1$ und $R_2$ die in Anspruch 1 angegebenen Bedeutungen haben; A die vorangehende Bedeutung hat, und X ein Halogenatom oder eine Alkylsulfonyloxygruppe mit 1 bis 4 Kohlenstoffatomen oder eine Arylsulfonyloxygruppe mit 6 bis 10 Kohlenstoffatomen bedeutet, mit einem Amin der allgemeinen Formel:

H - Am (3)

worin Am die in Anspruch 1 angegebene Bedeutung hat, kondensiert zur Bildung des erwünschten Derivats, welches, falls erwünscht, mit einer organischen oder anorganischen Säure zur Bildung eines pharmazeutisch annehmbaren Salzes des Derivats umgesetzt wird.

**11.** Verfahren zur Herstellung der Aminoalkoxyphenylderivate nach Anspruch 1, worin A wie in Anspruch 1 definiert ist und B eine Gruppe -S- oder -$SO_2$- bedeutet, **dadurch gekennzeichnet,** daß man in Gegenwart eines basischen Mittels ein 4-Hydroxyphenyl derivat der allgemeinen Formel:

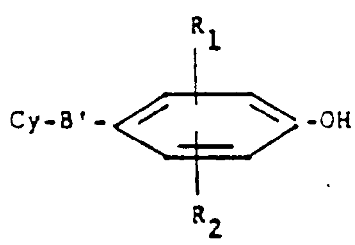

(4)

worin B' eine Gruppe -S- oder -$SO_2$- bedeutet und Cy, $R_1$ und $R_2$ die in Anspruch 1 angegebenen Bedeutungen haben, mit einer Verbindung der allgemeinen Formel:

X - A - Am (52)

worin X ein Halogenatom oder eine Alkylsulfonyloxygruppe mit 1 bis 4 Kohlenstoffatomen oder Arylsulfonyloxygruppe mit 6 bis 10 Kohlenstoffatomen bedeutet, A wie obenstehend definiert ist und Am die in Anspruch 1 angegebene Bedeutung hat, umsetzt, wobei die Umsetzung unter Rückfluß und in einem geeigneten Lösungsmittel stattfindet, um das erwünschte Derivat zu erhalten, welches, falls erwünscht, mit einer geeigneten organischen oder anorganischen Säure zur Bildung eines pharmazeutisch annehmbaren Salzes umgesetzt werden kann.

**12.** Verfahren zur Herstellung der Aminoalkoxyphenylderivate nach Anspruch 1, worin A einen gegebenenfalls substituierten 2-Hydroxypropylenrest bedeutet und B eine Gruppe -S- oder $-SO_2-$ bedeutet, **dadurch gekennzeichnet,** daß man unter Rückfluß ein Oxiranylmethoxyderivat der allgemeinen Formel:

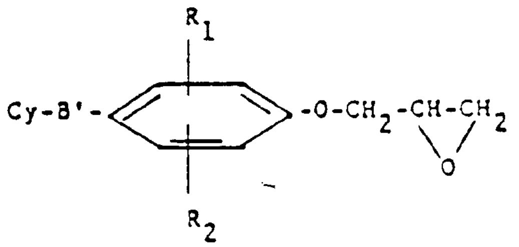
(57)

worin B' eine Gruppe -S- oder $-SO_2-$ bedeutet und Cy, $R_1$ und $R_2$ die in Anspruch 1 angegebenen Bedeutungen haben, mit einem Amin der allgemeinen Formel:

H - Am (3)

worin Am die in Anspruch 1 angegebene Bedeutung hat, behandelt, nämlich in einem polaren Lösungsmittel oder in einem Überschuß des Amins, zur Bereitstellung:

- des erwünschten Derivats in Form der freien Base, worin A einen 2-Hydroxypropylenrest bedeutet,
- eines Aminoalkoxyphenylderivats, welches mit einem Alkylhalogenid mit 1 bis 4 Kohlenstoffatomen in Gegenwart einer starken Base umgesetzt werden kann, wodurch das erwünschte Derivat in Form der freien Base erhalten wird, worin A einen 2-Hydroxypropylenrest bedeutet, wobei das Hydroxy durch ein Alkyl mit 1 bis 4 Kohlenstoffatomen substituiert ist,

  wobei das so erhaltene Aminoalkoxyphenylderivat, falls erwünscht, der Umsetzung mit einer organischen oder anorganischen Säure zur Bildung eines pharmazeutisch annehmbaren Salzes unterzogen werden kann.

**13.** Verfahren zur Herstellung der Aminoalkoxyphenylderivate nach Anspruch 1, worin B eine Gruppe -SO- bedeutet, **dadurch gekennzeichnet,** daß man ein Sulfid der allgemeinen Formel:

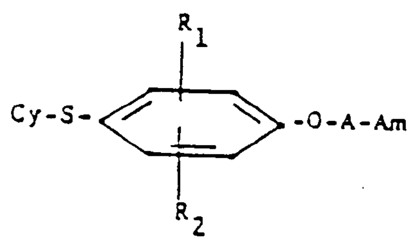

worin Cy, $R_1$, $R_2$, A und Am die in Anspruch 1 angegebenen Bedeutungen haben, mit einem Oxidationsmittel behandelt, wobei das Sulfid in Form der freien Base oder des Salzes vorliegt, um das erwünschte Derivat in Form der freien Base oder des Salzes zu bilden, und daß man das so erhaltene Salz mit einem basischen Mittel zur Bereitstellung des erwünschten Derivats in Form der freien Base behandelt, wobei die so erhaltene freie Base, falls erwünscht, der Umsetzung mit einer organischen oder anorganischen Säure zur Bildung eines pharmazeutisch annehmbaren Salzes des Derivats unterzogen wird.

**14.** Verfahren nach Anspruch 13, **dadurch gekennzeichnet,** daß das Oxidationsmittel Natriumperiodat, Kaliumpermanganat oder 3-Chlorperbenzoesäure ist.

**15.** Pharmazeutische oder tierärztliche Zusammensetzungen, enthaltend als Wirkstoff mindestens ein Aminoalkoxyphenylderivat nach einem der Ansprüche 1 bis 9, in Kombination mit einem pharmazeutischen Vehikel oder einem geeigneten Träger.

**16.** Pharmazeutische oder tierärztliche Zusammensetzungen nach Anspruch 15 zur Behandlung pathologischer Syndrome des kardiovaskulären Systems, enthaltend 50 mg bis 500 mg des Wirkstoffs.

**17.** Verwendung mindestens eines Aminoalkoxyphenylderivats nach einem der Ansprüche 1 bis 9 zur Herstellung eines Arzneimittels.

**18.** Verwendung mindestens eines Alkoxyphenylderivats nach einem der Ansprüche 1 bis 9 zur Herstellung eines Arzneimittels zur Behandlung pathologischer Syndrome des kardiovaskulären Systems.

**19.** Verwendung mindestens eines Aminoalkoxyphenylderivats nach einem der Ansprüche 1 bis 9 zur Herstellung eines Arzneimittels zur Behandlung pathologischer Okularaffektionen.

**Patenstansprüche für folgenden Vertragsstaat : ES**

**1.** Verfahren zur Herstellung von Aminoalkoxyphenylderivaten der allgemeinen Formel:

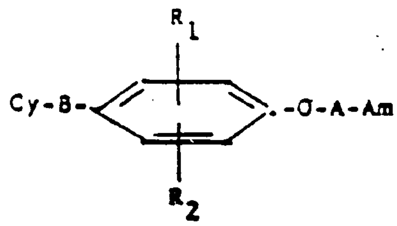

(1)

sowie deren pharmazeutisch annehmbaren Salze,
worin bedeuten:
B eine Gruppe -S-, -SO- oder $-SO_2-$,
$R_1$ und $R_2$, welche gleich oder verschieden sind, jeweils Wasserstoff, einen Methyl- oder Ethylrest oder ein Halogen,
A einen linearen oder verzweigten $C_2$-$C_5$-Alkylenrest oder den 2-Hydroxypropylenrest, worin das Hydroxy gegebenenfalls durch einen $C_1$-$C_4$-Alkylrest substituiert ist,
Am eine Gruppe:

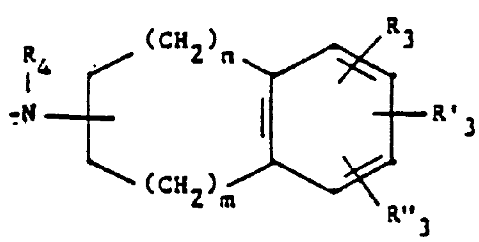

(D)

oder

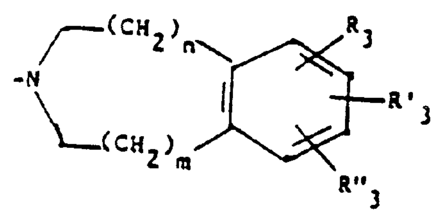

(E)

worin $R_3$, $R'_3$ und $R''_3$, welche gleich oder verschieden sind, jeweils Wasserstoff, ein Halogenatom, eine $C_1$-$C_4$-Alkylgruppe oder eine $C_1$-$C_4$-Alkoxygruppe,
$R_4$ Wasserstoff oder einen $C_1$-$C_8$-Alkylrest,
n und m, welche gleich oder verschieden sind, jeweils 0, 1, 2 oder 3 bedeuten,
Cy eine Gruppe der Formel:

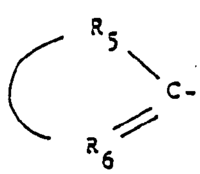

(F)

oder

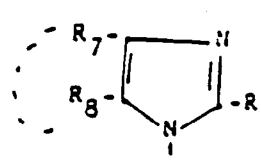

(G)

worin bedeuten:

R Wasserstoff, einen $C_1$-$C_8$-Alkylrest, einen $C_3$-$C_6$-Cycloalkylrest, einen Benzylrest oder Phenylrest, welcher gegebenenfalls durch einen oder mehrere Substituenten, welche gleich oder verschieden sein können, gewählt aus Halogenatomen oder $C_1$-$C_4$-Alkylgruppen, $C_1$-$C_4$-Alkoxygruppen oder Nitro, substituiert ist,

$R_5$ und $R_6$ sind mit dem Kohlenstoffatom, an welches sie gebunden sind, verknüpft zur Bildung:

- einer mono- oder dicyclischen, gegebenenfalls aromatischen, carbocyclischen Gruppe mit 5 bis 10 Kohlenstoffatomen, welche gegebenenfalls durch eine R-Gruppe in α-Stellung zur Methingruppe substituiert ist,
- einer gegebenenfalls aromatischen, heterocyclischen, 5-gliedrigen Gruppe, wobei die Heteroatome oder Heterogruppen aus den Gruppen O, S, N,

  $$-\overset{|}{N}-R_9;$$

  O und N; O und

  $$-\overset{|}{N}-R_9;$$

  und

  $$\overset{|}{N};$$

  S und

  $$-\overset{|}{N}-R_9;$$

  N und N; N und

  $$-\overset{|}{N}-R_9;$$

  gewählt sind, die heterocyclische Gruppe gegebenenfalls durch eine R-Gruppe, welche wie obenstehend definiert ist, in α-Stellung zur Methingruppe und gegebenenfalls durch eine oder zwei aus $C_1$-$C_4$-Alkylgruppen und Phenyl gewählten Gruppen substituiert ist,
- einer gegebenenfalls aromatischen, mono- oder dicyclischen, 6- bis 10-gliedrigen heterocyclischen Gruppe, wobei die Heteroatome oder Heterogruppen aus den Gruppen O, S, N,

  $$-\overset{|}{N}-R_9;$$

  O und N; O und

  $$-\overset{|}{N}-R_9;$$

  S und N; S und

  $$-\overset{|}{N}-R_9;$$

  N und N; N und

  $$-\overset{|}{N}-R_9,$$

  gewählt sind, die heterocyclische Gruppe gegebenenfalls durch eine R-Gruppe, welche wie obenstehend definiert ist, in α-Stellung zur Methingruppe substituiert ist.

$R_7$ und $R_8$, welche gleich oder verschieden sind, jeweils Wasserstoff, einer $C_1$-$C_4$-Niederalkylrest oder einen Phenylrest, oder sie bilden, wenn sie mit den Kohlenstoffatomen, an welche sie gebunden sind, verknüft sind, einen gegebenenfalls aromatischen, 6-gliedrigen Carbocyclus,

$R_9$ Wasserstoff, eine $C_1$-$C_4$-Alkylgruppe, Phenyl, Benzyl oder Halogenbenzyl,

**dadurch gekennzeichnet,** daß man

A/ wenn A wie voranstehend definiert ist und B eine Gruppe -S- oder -$SO_2$- bedeutet, in Gegenwart

eines Säureakzeptors in einem polaren oder nichtpolaren Lösungsmittel bei einer Temperatur, welche zwischen Umgebungstemperatur und der Rückflußtemperatur liegt, ein 4-Alkoxyphenylderivat der allgemeinen Formel:

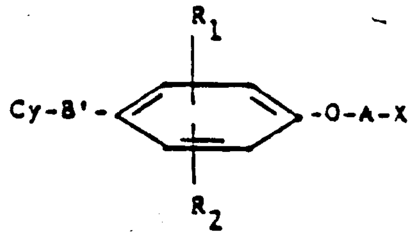

(2)

worin B' eine Gruppe -S- oder $-SO_2-$ bedeutet; Cy, $R_1$ und $R_2$ die in Anspruch 1 angegebenen Bedeutungen haben; A die vorangehende Bedeutung hat, und X ein Halogenatom oder eine Alkylsulfonyloxygruppe mit 1 bis 4 Kohlenstoffatomen oder eine Arylsulfonyloxygruppe mit 6 bis 10 Kohlenstoffatomen bedeutet, mit einem Amin der allgemeinen Formel:

H - Am (3)

worin Am die voranstehend angegebene Bedeutung hat, kondensiert,

- in Gegenwart eines basischen Mittels ein 4-Hydroxyphenylderivat der allgemeinen Formel:

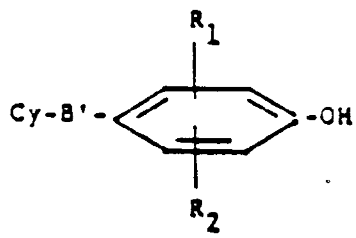

(4)

worin B' eine Gruppe -S- oder $-SO_2-$ bedeutet und Cy, $R_1$ und $R_2$ die voranstehenden Bedeutungen haben, mit einer Verbindung der allgemeinen Formel:

X - A - Am (52)

worin X ein Halogenatom oder eine Alkylsulfonyloxygruppe mit 1 bis 4 Kohlenstoffatomen oder Arylsulfonyloxygruppe mit 6 bis 10 Kohlenstoffatomen bedeutet, A wie obenstehend definiert ist und Am die voranstehend angegebene Bedeutung hat, umsetzt, wobei die Umsetzung unter Rückfluß und in einem geeigneten Lösungsmittel stattfindet;

B/ wenn A einen gegebenenfalls substituierten 2-Hydroxypropylenrest bedeutet und B eine Gruppe -S- oder $-SO_2-$ bedeutet, unter Rückfluß ein Oxiranylmethoxyderivat der allgemeinen Formel:

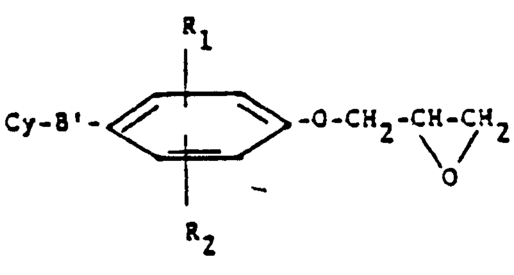

(57)

worin B' eine Gruppe -S- oder $-SO_2-$ bedeutet und Cy, $R_1$ und $R_2$ die voranstehend angegebenen Bedeutungen haben, mit einem Amin der allgemeinen Formel:

H - Am (3)

worin Am die voranstehend angegebene Bedeutung hat, behandelt, nämlich in einem polaren Lösungsmittel oder in einem Überschuß des Amins, zur Bereit stellung:

- des erwünschten Derivats in Form der freien Base, worin A einen 2-Hydroxypropylenrest bedeutet,
- eines Aminoalkoxyphenylderivats, welches mit einemAlkylhalogenid mit 1 bis 4 Kohlenstoffatomen in Gegenwart einer starken Base umgesetzt werden kann, wodurch das erwünschte Derivat in Form der freien Base erhalten wird, worin A einen 2-Hydroxypropylenrest bedeutet, wobei das Hydroxy durch ein Alkyl mit 1 bis 4 Kohlenstoffatomen substituiert ist; und

C/ wenn B eine Gruppe -SO- bedeutet, ein Sulfid der allgemeinen Formel:

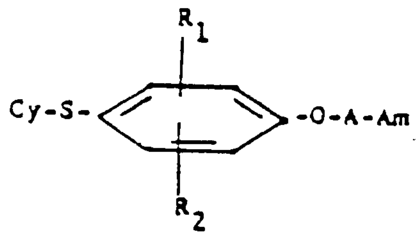

worin Cy, $R_1$, $R_2$, A und Am die voranstehend angegebenen Bedeutungen haben, mit einem Oxidationsmittel behandelt, wobei das Sulfid in Form der freien Base oder des Salzes vorliegt zur Bildung des erwünschten Derivats in Form der freien Base oder des Salzes, und daß man das so erhaltene Salz mit einem basischen Mittel behandelt zur Bereitstellung des erwünschten Derivats in Form der freien Base, wobei das so erhaltene Aminoalkoxyphenylderivat, falls erwünscht, der Umsetzung mit einer organischen oder anorganischen Säure zur Bildung eines pharmazeutisch annehmbaren Salzes des Derivats unterzogen wird.

2. Verfahren nach Anspruch 1, **dadurch gekennzeichnet,** daß das Oxidationsmittel Natriumperiodat, Kaliumpermanganat oder 3-Chlorperbenzoesäure ist.

3. Verfahren nach Anspruch 1, **dadurch gekennzeichnet,** daß man Aminoalkoxyphenylderivate der allgemeinen Formel (1) herstellt, worin Cy eine Phenyl-, Cyclohexenyl-, Indenyl-, Naphthyl-, Dihydronaphthyl-, Pyridyl-, Dihydropyridyl-, Furyl-, Dihydrofuryl-, Thienyl-, Dihydrothienyl-, Pyrrolyl-, Dihydropyrrolyl-, Pyrazolyl-, Imidazolyl-, Pyrimidyl-, Pyrazinyl-, Pyridazinyl-, Oxazolyl-, Isoxazolyl-, Thiazolyl-, Benzofuryl-, Benzothienyl-, Indolyl-, Benzimidazo lyl-, Benzoxazolyl-, Chinolinyl-, Benzisoxazolyl-, Cinnolinyl-, Chinoxalinyl-, Chinazolinyl-, Indolizinyl-, Thienopyridyl-, Tetrahydrothienopyridyl-, Pyrrolopyridyl-, Pyrazolopyridyl-, Pyrrolopyridazinyl- oder Imidazolpyridyl-Gruppe bedeutet.

4. Verfahren nach Anspruch 1, **dadurch gekennzeichnet,** daß man Aminoalkoxyphenylderivate der allgemeinen Formel (1) herstellt, worin Cy eine Indolizinyl-, Benzofuryl-, Benzothienyl-, Indolyl-, Oxazolyl-, Pyrazolyl-, Phenyl-, Pyrazolo[1,5-a]pyridyl- oder Imidazo[1,2-a]pyridyl-Gruppe deutet.

5. Verfahren nach Anspruch 1, **dadurch gekennzeichnet,** daß man Aminoalkoxyphenylderivate der allgemeinen Formel (1) herstellt, worin B die Gruppe $-SO_2-$ bedeutet.

6. Verfahren nach Anspruch 1, **dadurch gekennzeichnet,** daß man Aminoalkoxyphenylderivate der allgemeinen Formel (1) herstellt, worin $R_1$ und $R_2$ jeweils Wasserstoff bedeuten.

7. Verfahren nach Anspruch 1, **dadurch gekennzeichnet,** daß man Aminoalkoxyphenylderivate der allgemeinen Formel (1) herstellt, worin $R_3$, $R'_3$ und $R''_3$ Wasserstoff oder Methoxy bedeuten.

8. Verfahren nach Anspruch 1, **dadurch gekennzeichnet,** daß man Aminoalkoxyphenylderivate der allgemeinen Formel (1) herstellt, worin R eine Isopropyl- oder Cyclopropylgruppe bedeutet.

9. Verfahren nach Anspruch 1, **dadurch gekennzeichnet,** daß man Aminoalkoxyphenylderivate der allgemeinen Formel (1) herstellt, worin das pharmazeutisch annehmbare Salz das Oxalat oder das Chlorhydrat ist.

10. Verfahren nach Anspruch 1, **dadurch gekennzeichnet,** daß man Aminoalkoxyphenylderivate herstellt, welche gewählt werden aus:
1-{4-[3-(6,7-Dimethoxy-1,2,3,4-tetrahydro-2-N-isochinolinyl)-propoxy]-benzolsulfonyl}-2-isopropyl-indolizin,
2-Ethyl-3-{4-[3-{6,7-dimethoxy-1,2,3,4-tetrahydro-2-N-isochinolinyl)-propoxy]-benzolsulfonyl}-benzothiophen,
4-{4-[3-(6,7-Dimethoxy-1,2,3,4-tetrahydro-2-N-isochinolinyl)-propoxy]-benzolsulfonyl}-5-isopropyl-oxazol,

4-{4-[3-(6,7-Dimethoxy-1,2,3,4-tetrahydro-2-N-isochinolinyl)-propoxy]-benzolsulfonyl}-5-ethyl-1-methyl-pyrazol,
4-{4-[3-(6,7-Dimethoxy-1,3,4-tetrahydro-2-N-isochinolinyl)-propoxy]-benzolsulfonyl}-5-isopropyl-1-methyl-pyrazol,
3-{4-[3-(6,7-Dimethoxy-1,2,3,4-tetrahydro-2-N-isochinolinyl)-propoxy]-benzolsulfonyl}-2-isopropyl-1-methyl-indol, 1-{4-[4-(6,7-Dimethoxy-1,2,3,4-tetrahydro-2-N-isochinolinyl)-butoxy]-benzolsulfonyl}-2-isopropyl-indolizin,
2-Isopropy1-3-{4-[3-(6,7-dimethoxy-1,2,3,4-tetrahydro-2-N-isochinolinyl)-propoxy]-benzolsulfonyl}-benzofuran,
2-Ethyl-3-{4-[3-(6,7-dimethoxy-1,2,3,4-tetrahydro-2-N-isochinolinyl)-propoxy]-benzolsulfonyl}-benzofuran,
{4-[2-(6,7-Dimethoxy-1,2,3,4-tetrahydro-N-isochinolinyl)-propoxy]-phenyl}-(2-isopropyl-phenyl)-sulfon,
3-{4-[N-(5,6,7,8,9-Pentahydro-9-benzocycloheptenyl)-3-N-methylamino-propoxy]-benzolsulfonyl}-2-isopropyl-1-methyl-indol,
3-{4-[N-Methyl-N-(1,2,3,4-tetrahydro-1-naphthyl)-3-amino-propoxy]benzolsulfonyl}-2-isopropyl-1-methyl-indol,
1-{4-[N-Methyl-N-(6,7-dimethoxy-1,2,3,4-tetrahydro-1-naphthyl)-3-aminopropoxy]-benzolsulfonyl)-2-isopropyl-indolizin,
3-{4-[N-Methyl-N-(1,2,3,4-tetrahydro-1-naphthyl)-3-amino-propoxy]-benzolsulfonyl}-2-isopropyl-pyrazolo[1,5-a]-pyridin,
und deren pharmazeutisch annehmbaren Salzen.

**11.** Verfahren zur Herstellung einer pharmazeutischen oder tierärztlichen Zusammensetzung, **dadurch gekennzeichnet,** daß man als Wirkstoff mindestens ein nach einem der Ansprüche 1 bis 10 hergestelltes Aminoalkoxyphenylderivat mit einem pharmazeutischen Vehikel oder einem geeigneten Rezipienten bzw, Träger vermischt.

**12.** Verfahren zur Herstellung einer pharmazeutischen oder tierärztlichen Zusammensetzung nach Anspruch 11 zur Behandlung pathologischer Syndrome des kardiovaskulären Systems, enthaltend 50 mg bis 500 mg des Wirkstoffs.

**13.** Verfahren zur Herstellung einer pharmazeutischen oder tierärztlichen Zusammensetzung nach Anspruch 11 zur Behandlung pathologischer Okularaffektionen.

**Pantentansprüche für folgenden Vertragsstaat : GR**

**1.** Aminoalkoxyphenylderivate der allgemeinen Formel

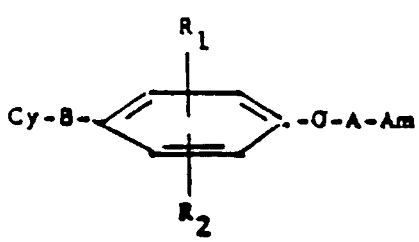

(1)

sowie deren pharmazeutisch annehmbaren Salze,
worin bedeuten:
B eine Gruppe -S-, -SO- oder $-SO_2-$,
$R_1$ und $R_2$, welche gleich oder verschieden sind, jeweils Wasserstoff, einen Methyl- oder Ethylrest oder ein Halogen,
A einen linearen oder verzweigten $C_2$-$C_5$-Alkylenrest oder den 2-Hydroxypropylenrest, worin das Hydroxy gegebenenfalls durch einen $C_1$-$C_4$-Alkylrest substituiert ist,
Am eine Gruppe:

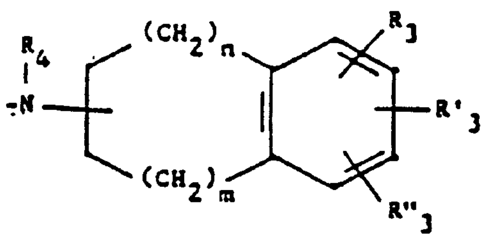 oder 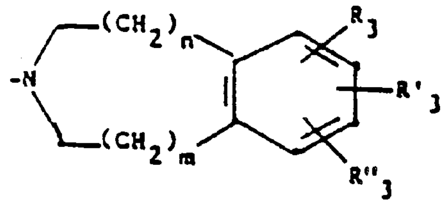

(D) (E)

worin $R_3$, $R'_3$ und $R''_3$, welche gleich oder verschieden sind, jeweils Wasserstoff, ein Halogenatom, eine $C_1$-$C_4$-Alkylgruppe oder eine $C_1$-$C_4$-Alkoxygruppe,

$R_4$ Wasserstoff oder einen $C_1$-$C_8$-Alkylrest,

n und m, welche gleich oder verschieden sind, jeweils 0, 1, 2 oder 3 bedeuten,

Cy eine Gruppe der Formel:

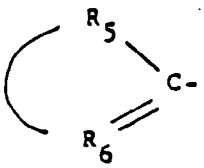 oder 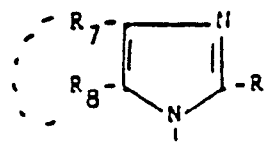

(F) (G)

worin bedeuten:

R Wasserstoff, einen $C_1$-$C_8$-Alkylrest, einen $C_3$-$C_6$-Cycloalkylrest, einen Benzylrest oder Phenylrest, welcher gegebenenfalls durch einen oder mehrere Substituenten, welche gleich oder verschieden sein können, gewählt aus Halogenatomen oder $C_1$-$C_4$-Alkylgruppen, $C_1$-$C_4$-Alkoxygruppen oder Nitro, substituiert ist,

$R_5$ und $R_6$ sind mit dem Kohlenstoffatom, an welches sie gebunden sind, verknüpft zur Bildung:

- einer mono- oder dicyclischen, gegebenenfalls aromatischen, carbocyclischen Gruppe mit 5 bis 10 Kohlenstoffatomen, welche gegebenenfalls durch eine R-Gruppe in $\alpha$-Stellung zur Methingruppe substituiert ist,
- einer gegebenenfalls aromatischen, heterocyclischen, 5-gliedrigen Gruppe, wobei die Heteroatome oder Heterogruppen aus den Gruppen O, S, N,

$$-\overset{|}{N}-R_9;$$

O und N; O und

$$-\overset{|}{N}-R_9;$$

S und

$$\overset{|}{N};$$

S und

$$-\overset{|}{N}-R_9;$$

N und N; N und

$$-\overset{|}{N}-R_9;$$

gewählt sind, die heterocyclische Gruppe gegebenenfalls durch eine R-Gruppe, welche wie obenstehend definiert ist, in α-Stellung zur Methingruppe und gegebenenfalls durch eine oder zwei aus $C_1$-$C_4$-Alkylgruppen und Phenyl gewählte Gruppen substituiert ist,

- einer gegebenenfalls aromatischen, mono- oder dicyclischen, 6- bis 10-gliedrigen heterocyclischen Gruppe, wobei die Heteroatome oder Heterogruppen aus den Gruppen O, S, N,

$$-\overset{|}{N}-R_9;$$

O und N; O und

$$-\overset{|}{N}-R_9;$$

S und N; S und

$$-\overset{|}{N}-R_9;$$

N und N; N und

$$-\overset{|}{N}-R_9,$$

gewählt sind, die heterocyclische Gruppe gegebenenfalls durch eine R-Gruppe, welche wie obenstehend definiert ist, in α-Stellung zur Methingruppe substituiert ist,

$R_7$ und $R_8$, welche gleich oder verschieden sind, jeweils Wasserstoff, einen $C_1$-$C_4$-Niederalkylrest oder einen Phenylrest, oder sie bilden, wenn sie mit den Kohlenstoffatomen, an welche sie gebunden sind, verknüpft sind, einen gegebenenfalls aromatischen, 6-gliedrigen Carbocyclus,

$R_9$ Wasserstoff, eine $C_1$-$C_4$-Alkylgruppe, Phenyl, Benzyl oder Halogenbenzyl.

2. Aminoalkoxyphenylderivate nach Anspruch 1, worin Cy eine Phenyl-, Cyclohexenyl-, Indenyl-, Naphthyl-, Dihydronaphthyl-, Pyridyl-, Dihydropyridyl-, Furyl-, Dihydrofuryl-, Thienyl-, Dihydrothienyl-, Pyrrolyl-, Dihydropyrrolyl-, Pyrazolyl-, Imidazolyl-, Pyrimidyl-, Pyrazinyl-, Pyridazinyl-, Oxazolyl-, Isoxazolyl-, Thiazolyl-, Benzofuryl-, Benzothienyl-, Indolyl-, Benzimidazolyl-, Benzoxazolyl-, Chinolinyl-, Benzisoxazolyl-, Cinnolinyl-, Chinoxalinyl-, Chinazolinyl-, Indolizinyl-, Thienopyridyl-, Tetrahydrothienopyrldyl-, Pyrrolopyridyl-, Pyrazolopyridyl-, Pyrrolopyridazinyl- oder Imidazolpyridyl-Gruppe bedeutet.

3. Aminoalkoxyphenylderivate nach Anspruch 1, worin Cy eine Indolizinyl-, Benzofuryl-, Benzothienyl-, Indolyl-, Oxazolyl-, Pyrazolyl-, Phenyl-, Pyrazolo[1,5-a]pyridyl- oder Imidazo[1,2-a]pyridyl-Gruppe deutet.

4. Aminoalkoxyphenylderivate nach Anspruch 1, worin B die Gruppe $-SO_2-$ bedeutet.

5. Aminoalkoxyphenylderivate nach Anspruch 1, worin $R_1$ und $R_2$ jeweils Wasserstoff bedeuten.

6. Aminoalkoxyphenylderivate nach Anspruch 1, worin $R_3$, $R'_3$ und $R''_3$ Wasserstoff oder Methoxy bedeuten.

7. Aminoalkoxyphenylderivate nach Anspruch 1, worin R eine Isopropyloder Cyclopropyl-Gruppe bedeutet.

8. Aminoalkoxyphenylderivate nach Anspruch 1, worin das pharmazeutisch annehmbare Salz das Oxalat oder das Chlorhydrat ist.

9. Aminoalkoxyphenylderivate nach Anspruch 1, gewählt aus:
1-{4-[3-(6,7-Dimethoxy-1,2,3,4-tetrahydro-2-N-isochinolinyl)-propoxy]-benzolsulfonyl}-2-isopropyl-indolizin,
2-Ethyl-3-{4-[3-{6,7-dimethoxy-1,2,3,4-tetrahydro-2-N-isochinolinyl)-propo-xy]-benzolsulfonyl}-benzothlophen,
4-{4-[3-(6,7-Dimethoxy-1,2,3,4-tetrahydro-2-N-isochinolinyl)-propoxy]-benzolsulfonyl]-5-isopropyl-oxazol,
4-{4-[3-(6,7-Dimethoxy-1,2,3,4-tetrahydro-2-N-isochinolinyl)-propoxy]-benzolsulfonyl}-5-ethyl-1-methyl-pyrazol,
4-{4-[3-(6,7-Dimethoxy-1,2,3,4-tetrahydro-2-N-isochinolinyl)-propoxy]-benzolsulfonyl}-5-isopropyl-1-methyl-pyrazol,
3-{4-[3-(6,7-Dimethoxy-1,2,3,4-tetrahydro-2-N-isochinolinyl)-propoxy]-benzolsulfonyl}-2-isopropyl-1-methyl-indol,
1-{4-[4-(6,7-Dimethoxy-1,2,3,4-tetrahydro-2-N-isochinolinyl)-butoxy]-benzolsulfonyl}-2-isopropyl-indolizin,

2-Isopropyl-3-{4-[3-(6,7-dimethoxy-1,2,3,4-tetrahydro-2-N-isochinolinyl)-propoxy]-benzolsulfonyl}-benzofuran,
2-Ethyl-3-{4-[3-(6,7-dimethoxy-1,2,3,4-tetrahydro-2-N-isochinolinyl)-propoxy]-benzolsulfonyl}-benzofuran.
{4-[2-(6,7-Dimethoxy-1,2,3,4-tetrahydro-N-isochinolinyl)-propoxy]-phenyl}-(2-isopropyl-phenyl)-sulfon,
3-{4-[N-(5,6,7,8,9-Pentahydro-9-benzocycloheptenyl)-3-N-methylamino-propoxy]-benzolsulfonyl}-2-isopropyl-1-methyl-indol,
3-{4-[N-Methyl-N-(1,2,3,4-tetrahydro-1-naphthyl)-3-amino-propoxy]benzolsulfonyl}-2-isopropy]-1-methyl-indol,
1-{4-[N-Methyl-N-(6,7-dimethoxy-1,2,3,4-tetrahydro-1-naphthyl)-3-aminopropoxy]-benzolsulfonyl}-2-isopropyl-indolizin,
3-{4-[N-Methyl-N-(1,2,3,4-tetrahydro-1-naphthyl)-3-amino-propoxy]-benzolsuflonyl}-2-isopropyl-pyrazolo[1,5-a]-pyridin,
und deren pharmazeutisch annehmbaren Salzen.

10. Verfahren zur Herstellung der Aminoalkoxyphenylderivate nach Anspruch 1, worin A wie in Anspruch 1 definiert ist und B eine Gruppe -S- oder $-SO_2-$ bedeutet, **dadurch gekennzeichnet,** daß man in Gegenwart eines Säureakzeptors in einem polaren oder nichtpolaren Lösungsmittel bei einer Temperatur, welche zwischen Umgebungstemperatur und der Rückflußtemperatur liegt, ein 4-Alkoxyphenylderivat der allgemeinen Formel:

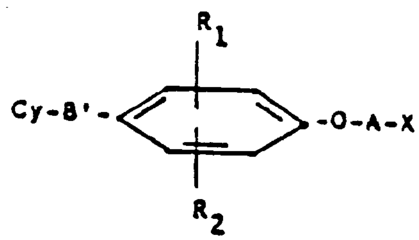

(2)

worin B' eine Gruppe -S- oder $-SO_2-$ bedeutet; Cy, $R_1$ und $R_2$ die in Anspruch 1 angegebenen Bedeutungen haben; A die vorangehende Bedeutung hat, und X ein Halogenatom oder eine Alkylsulfonyloxygruppe mit 1 bis 4 Kohlenstoffatomen oder eine Arylsulfonyloxygruppe mit 6 bis 10 Kohlenstoffatomen bedeutet, mit einem Amin der allgemeinen Formel:

H - Am (3)

worin Am die in Anspruch 1 angegebene Bedeutung hat, kondensiert zur Bildung des erwünschten Derivats, welches, falls erwünscht, mit einer organischen oder anorganischen Säure zur Bildung eines pharmazeutisch annehmbaren Salzes des Derivats umgesetzt wird:

11. Verfahren zur Herstellung der Aminoalkoxyphenylderivate nach Anspruch 1, worin A wie in Anspruch 1 definiert ist und B eine Gruppe -S- oder $-SO_2-$ bedeutet, **dadurch gekennzeichnet,** daß man in Gegenwart eines basischen Mittels ein 4-Hydroxyphenylderivat der allgemeinen Formel:

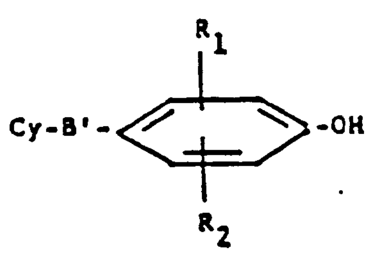

(4)

worin B' eine Gruppe -S- oder $-SO_2-$ bedeutet und Cy, $R_1$ und $R_2$ die in Anspruch 1 angegebenen Bedeutungen haben, mit einer Verbindung der allgemeinen Formel:

X - A - Am (52)

worin X ein Halogenatom oder eine Alkylsulfonyloxygruppe mit 1 bis 4 Kohlenstoffatomen oder Arylsulfonyloxygruppe mit 6 bis 10 Kohlenstoffatomen bedeutet, A wie obenstehend definiert ist und Am die in Anspruch 1 angegebene Bedeutung hat, umsetzt, wobei die Umsetzung unter Rückfluß und in einem geeigneten Lösungsmittel stattfindet, um das erwünschte Derivat zu erhalten, welches, falls erwünscht, mit

einer geeigneten organischen oder anorganischen Säure zur Bildung eines pharmazeutisch annehmbaren Salzes umgesetzt werden kann.

**12.** Verfahren zur Herstellung der Aminoalkoxyphenylderivate nach Anspruch 1, worin A einen gegebenenfalls substituierten 2-Hydroxypropylenrest bedeutet und B eine Gruppe -S- oder -$SO_2$- bedeutet, **dadurch gekennzeichnet,** daß man unter Rückfluß ein Oxiranylmethoxyderivat der allgemeinen Formel:

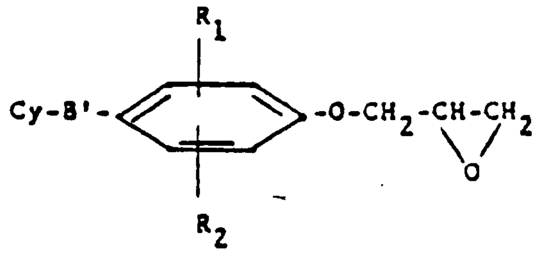

(57)

worin B' eine Gruppe -S- oder -$SO_2$- bedeutet und Cy, $R_1$ und $R_2$ die in Anspruch 1 angegebenen Bedeutungen haben, mit einem Amin der allgemeinen Formel:

H - Am (3)

worin Am die in Anspruch 1 angegebene Bedeutung hat, behandelt, nämlich in einem polaren Lösungsmittel oder in einem Überschuß des Amins, zur Bereitstellung:

- des erwünschten Derivats in Form der freien Base, worin A einen 2-Hydroxypropylenrest bedeutet,
- eines Aminoalkoxyphenylderivats, welches mit einem Alkylhalogenid mit 1 bis 4 Kohlenstoffatomen in Gegenwart einer starken Base umgesetzt werden kann, wodurch das erwünschte Derivat in Form der freien Base erhalten wird, worin A einen 2-Hydroxypropylenrest bedeutet, wobei das Hydroxy durch ein Alkyl mit 1 bis 4 Kohlenstoffatomen substituiert ist,

  wobei das so erhaltene Aminoalkoxyphenylderivat, falls erwünscht, der Umsetzung mit einer organischen oder anorganischen Säure zur Bildung eines pharmazeutisch annehmbaren Salzes unterzogen werden kann.

**13.** Verfahren zur Herstellung der Aminoalkoxyphenylderivate nach Anspruch 1, worin B eine Gruppe -SO- bedeutet, **dadurch gekennzeichnet,** daß man ein Sulfid der allgemeinen Formel:

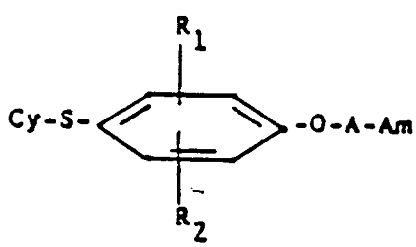

worin Cy, $R_1$, $R_2$, A und Am die in Anspruch 1 angegebenen Bedeutungen haben, mit einem Oxidationsmittel behandelt, wobei das Sulfid in Form der freien Base oder des Salzes vorliegt, um das erwünschte Derivat in Form der freien Base oder des Salzes zu bilden, und daß man das so erhaltene Salz mit einem basischen Mittel zur Bereitstellung des erwünschten Derivats in Form der freien Base behandelt, wobei die so erhaltene freie Base, falls erwünscht, der Umsetzung mit einer organischen oder anorganischen Säure zur Bildung eines pharmazeutisch annehmbaren Salzes des Derivats unterzogen wird.

**14.** Verfahren nach Anspruch 13, **dadurch gekennzeichnet,** daß das Oxidationsmittel Natriumperiodat, Kaliumpermanganat oder 3-Chlorperbenzoesäure ist.

**15.** Verfahren zur Herstellung pharmazeutischer oder tierärztlicher Zusammensetzungen, **dadurch gekennzeichnet,** daß man mindestens ein Aminoalkoxyphenylderviat nach einem der Ansprüche 1 bis 9 mit einem pharmazeutischen Vehikel oder einem geeigneten Träger vermischt.

**16.** Verfahren zur Herstellung pharmazeutischer oder tierärztlicher Zusammensetzungen nach Anspruch 15

zur Behandlung pathologischer Syndrome des kardiovaskulären Systems, enthaltend 50 mg bis 500 mg des Wirkstoffs.

**17.** Verfahren zur Herstellung pharmazeutischer oder tierärztlicher Zusammensetzungen nach Anspruch 15 zur Herstellung eines Arzneimittels zur Behandlung pathologischer Okularaffektionen.

## Claims

### Claims for the following Contracting States : AT, CH, DE, DK, FR, GB, IT, LI, LU, NL, SE,

**1.** Aminoalkoxyphenyl derivatives corresponding to the general formula:

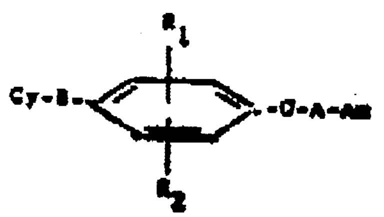

(I)

as well as their pharmaceutically acceptable salts, in which:

B represents a group -S-, -SO- or $-SO_2-$,

$R_1$ and $R_2$, which are identical or different, each represent hydrogen, the methyl or ethyl radical or a halogen,

A represents a linear or branched $C_2$-$C_5$ alkylene radical or the 2-hydroxypropylene radical in which the hydroxyl is optionally substituted with a $C_1$-$C_4$ alkyl radical,

Am represents a group:

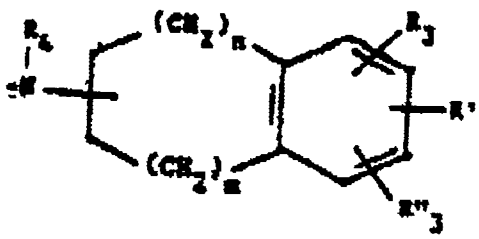

(D),

or

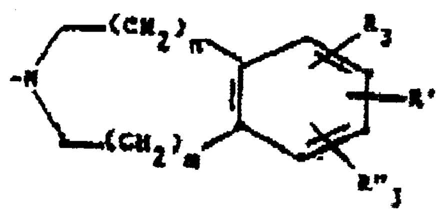

(E)

in which: $R_3$, $R'_3$ and $R''_3$, which are identical or different, each represent hydrogen, a halogen atom, a $C_1$-$C_4$ alkyl group or a $C_1$-$C_4$ alkoxy group,

$R_4$ represents hydrogen or a $C_1$-$C_8$ alkyl radical,

n and m, which may be identical or different, each representent 0, 1, 2 or 3,

Cy represents a group of formula:

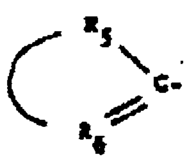

or

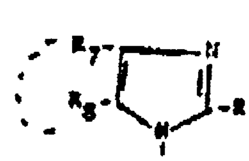

R represents hydrogen, a $C_1$-$C_8$ alkyl radical, a $C_3$-$C_6$ cycloalkyl radical, a benzyl radical or a phenyl radical which is optionally substituted with one or more substituents, which may be identical or different, chosen from halogen atoms or from $C_1$-$C_4$ alkyl, $C_1$-$C_4$ alkoxy or nitro groups,

$R_5$ and $R_6$ are taken together, with the carbon atom to which they are attached, to form:

- a mono- or dicyclic carbocyclic group which is optionally aromatic, having from 5 to 10 carbon atoms and optionally substituted with a group R in the α position with respect to the methyne group,

- a 5-membered heterocyclic group which is optionally aromatic, the heteroatoms or heterogroups being chosen from groups O, S, N,

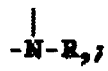

O and N; O and

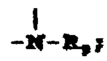

S and

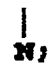

S and

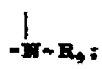

N and N; N and

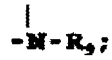

the heterocyclic group being optionally substituted with a group R as defined above in the α position with respect to the methyne group, and optionally substituted with one or two groups chosen from $C_1$-$C_4$ alkyl and phenyl groups,
- a mono- or dicyclic heterocyclic group having from 6 to 10 members and being optionally aromatic, the heteroatoms or heterogroups being chosen from groups O, S, N,

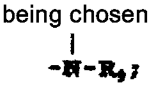

O and N; O and

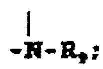

S and N; S and

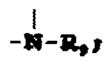

N and N; N and

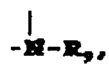

the heterocyclic group being optionally substituted with a group R as defined above in the α position with respect to the methyne group,

$R_7$ and $R_8$, which are identical or different, each represent hydrogen, a $C_1$-$C_4$ lower alkyl radical or a phenyl radical, or when they are taken together, with the carbon atoms to which they are attached, represent a 6-membered carbocycle which is optionally aromatic,

$R_9$ represents hydrogen or a $C_1$-$C_4$ alkyl, phenyl, benzyl or halobenzyl group.

**2.** Aminoalkoxyphenyl derivatives according to Claim 1, in which Cy represents a phenyl, cyclohexenyl, indenyl, naphthyl, dihydronaphthyl, pyridyl, dihydropyridyl, furyl, dihydrofuryl, thienyl, dihydrothienyl, pyrrolyl, dihydropyrrolyl, pyrazolyl, imidazolyl, pyrimidyl, pyrazinyl, pyridazinyl, oxazolyl, isoxazolyl, thiazolyl, benzofuryl, benzothienyl, indolyl, benzimidazolyl, benzoxazolyl, quinolyl, benzisoxazolyl, cinnolinyl, quinoxalinyl, quinazolinyl, indolixinyl, thienopyridyl, tetrahydrothienopyridyl, pyrrolopyridyl, pyrazolopyridyl, pyrolopyridazinyl or imidazopyridyl, group.

**3.** Aminoalkoxyphenyl derivatives according to Claim 1, in which Cy represents an indolizinyl, benzofuryl, benzothienyl, indolyl, oxazolyl, pyrazolyl, phenyl, pyrazolo[1,5-a]pyridyl or imidazo[1,2-a]pyridyl group.

4. Aminoalkoxyphenyl derivatives according to Claim 1, in wich B represents a group $-SO_2-$.

5. Aminoalkoxyphenyl derivatives according to Claim 1, in which $R_1$ and $R_2$ each represent hydrogen.

6. Aminoalkoxyphenyl derivatives according to Claim 1, in which $R_3$, $R'_3$ and $R''_3$ representent hydrogen or methoxy.

7. Aminoalkoxyphenyl derivatives according to Claim 1, in which R represents the isopropyl or cyclopropyl group.

8. Aminoalkoxyphenyl derivatives according to Claim 1, in which the pharmaceutically acceptable salt is the oxalate or the hydrochloride.

9. Aminoalkoxyphenyl derivatives according to Claim 1, chosen from:
1-{4-[3-(6,7-dimethoxy-1,2,3,4-tetrahydro-2-N-isoquinolyl)propoxyl]benzenesulphonyl}-2-isopropylindolizine,
2-ethyl-3-{4-[3-(6,7-dimethoxy-1,2,3,4-tetrahydro-2-N-isoquinolyl)propoxy]benzenesulphonyl}benzothiophene,
4-{4-[3-(6,7-dimethoxy-1,2,3,4-tetrahydro-2-N-isoquinolyl)propoxy]benzenesulphonyl}-5-isopropyloxazole,
4-{4-[3-(6,7-dimethoxy-1,2,3,4-tetrahydro-2-N-isoquinolyl)propoxy]benzenesulphonyl}-5-ethyl-1-methylpyrazole,
4-{4-[3-(6,7-dimethoxy-1,2,3,4-tetrahydro-2-N-isoquinolyl)propoxy]benzenesulphonyl}-5-isopropyl-1-methylpyrazole,
3-{4-[3-(6,7-dimethoxy-1,2,3,4-tetrahydro-2-N-isoquinolyl)propoxy]benzenesulphonyl}-2-isopropyl-1-methylindole,
1-{4-[4-(6,7-dimethoxy-1,2,3,4-tetrahydro-2-N-isoquinolyl)butoxy]benzenesulphonyl}-2-isopropylindolizine,
2-isopropyl-4-[3-(6,7-dimethoxy-1,2,3,4-tetrahydro-2-N-isoquinolyl)propoxy]-3-benzenesulphonylbenzofuran,
2-ethyl-4-[3-(6,7-dimethoxy-1,2,3,4-tetrahydro-2-N-isoquinolyl)propoxy]-3-benzenesulphonylbenzofuran,
4-[2-(6,7-dimethoxy-1,2,3,4-tetrahydro-N-isoquinolyl)propoxy]phenyl (2-isopropylphenyl) sulphone,
4-[N-(5,6,7,8,9-pentahydro-9-benzocycloheptenyl)-3-N-methylaminopropoxy]-3-benzenesulphonyl-2-isopropyl-1-methylindole,
4-[N-methyl-3-N-(1,2,3,4-tetrahydro-1-naphthyl)aminopropoxy]-3-benzenesulphonyl-2-isopropyl-1-methylindole,
4-[N-methyl-3-N-(6,7-dimethoxy-1,2,3,4-tetrahydro-1-naphthyl)aminopropoxy]-1-benzenesulphonyl-2-isopropylindolizine,
4-[N-methyl-3-N-(1,2,3,4-tetrahydro-1-naphthyl)aminopropoxy]-3-benzenesulphonyl-2-isopropylpyrazolo[1,5-a]-pyridine
and their pharmaceutically acceptable salts.

10. Process for the preparation of aminoalkoxyphenyl derivatives according to Claim 1 in which A is as defined in Claim 1 and B represents a group -S- or $-SO_2-$, characterized in that a 4-alkoxyphenyl derivative of general formula:

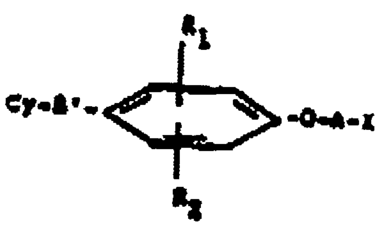

(2)

in which B represents a group -S- or $-SO_2-$: Cy, $R_1$ and $R_2$ have the same meanings as in Claim 1; A has the same meaning as above and X represents a halogen atom or an alkylsulphonyloxy group having 1 to 4 carbon atoms or an arylsulphonyloxy group having 6 to 10 carbon atoms is condensed, in the presence of an acid acceptor, in a polar or nonpolar solvent and at a temperature between room temperature and the reflux temperature, with an amine of general formula:

H - Am (3)

in which Am has the same meaning as in Claim 1, to form the desired derivative which is reacted, if so desired, with an organic or inorganic acid to form a pharmaceutically acceptable salt of this derivative.

**11.** Process for the preparation of aminoalkoxyphenyl derivatives according to Claim 1, in which A is as defined in Claim 1 and B represents a group -S- or $-SO_2-$, characterized in that a 4-hydroxyphenyl derivative of general formula:

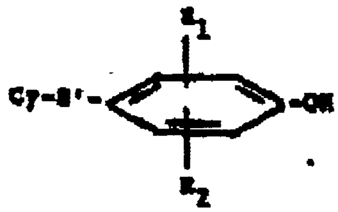

(4)

in which B' represents a group -S- or $-SO_2-$ and Cy, $R_1$ and $R_2$ have the same meanings as in Claim 1 is reacted, in the presence of a basic agent, with a compound of general formula

X - A - Am (52)

in which X represents a halogen atom or an alkylsulphonyloxy group having from 1 to 4 carbon atoms or an arylsulphonyloxy group having 6 to 10 carbon atoms, A is as defined above and Am has the same value as in Claim 1, the reaction taking place at reflux and in a suitable solvent to obtain the desired derivative, which it is possible, if so desired, to react with a suitable organic or inorganic acid in order to form a pharmaceutically acceptable salt.

**12.** Process for the preparation of aminoalkoxyphenyl derivatives according to Claim 1, in which A represents the 2-hydroxypropylene radical which is optionally substituted and B represents a group -S- or $-SO_2-$, characterized in that an oxiranylmethoxy derivative of general formula:

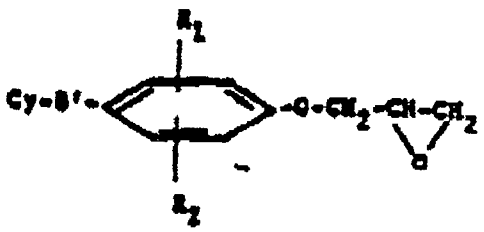

(57)

in which B' represents a group -S- or $-SO_2-$ and Cy, $R_1$ and $R_2$ have the same meanings as in Claim 1, is treated at reflux with an amine of general formula:

H - Am (3)

in which Am has the same meaning as in Claim 1, in a polar solvent or in an excess of the said amine in order to give:

- the desired derivative, in free base form, in which A represents a 2-hydroxypropylene radical
- an aminoalkoxyphenyl derivative which can be reacted with an alkyl halide having from 1 to 4 carbon atoms and in the presence of a strong base, which gives the desired derivative in free base form, in which A represents a 2-hydroxypropylene radical in which the hydroxyl is substituted with an alkyl having from 1 to 4 carbon atoms,

  the aminoalkoxyphenyl derivative thus obtained being able, if so desired, to be reacted with an organic or inorganic acid in order to form a pharmaceutically acceptable salt.

**13.** Process for the preparation of aminoalkoxyphenyl derivatives according to claim 1, in which B represents a group -SO-, characterized in that a sulphide of general formula:

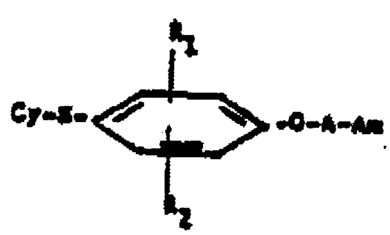

in which Cy, $R_1$, $R_2$, A and Am have the same meanings as in Claim 1, this sulphide being in free base or salt form, is treated with an oxidizing agent in order to form the desired derivative in free base or salt form, and in that the salt thus obtained is treated with a basic agent in order to give the desired derivative in free base form, the free base thus obtained being, if so desired, reacted with an organic or inorganic acid in order to form a pharmaceutically acceptable salt of this derivative.

**14.** Process according to Claim 13, characterized in that the oxidizing agent is sodium periodate, potassium permanganate or 3-chloroperbenzoic acid.

**15.** Pharmaceutical or veterinary compositions containing, as active principle, at least one aminoalkoxyphenyl derivative according to one of Claims 1 to 9, in combination with a suitable pharmaceutical vehicle or excipient.

**16.** Pharmaceutical or veterinary compositions according to claim 15, for the treatment of pathological syndromes of the cardiovascular system, containing from 50 mg to 500 mg of active principle.

**17.** Use of at least one aminoalkoxyphenyl derivative according to any one of claims 1 to 9 for the production of a medicament.

**18.** Use of at least one aminoalkoxyphenyl derivative according to any one of claims 1 to 9 for the production of a medicament intended for the treatment of pathological syndromes of the cardiovascular system.

**19.** Use of at least one aminoalkoxyphenyl derivative according to any one of claims 1 to 9 for the production of a medicament intended for the treatment of ocular pathological complaints.

**Claims for the following Contracting State : ES**

**1.** Process for the preparation of aminoalkoxyphenyl derivatives corresponding to the general formula:

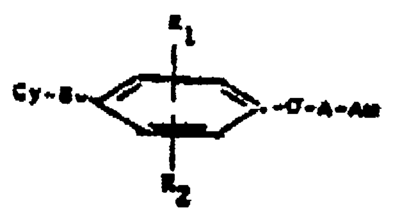

(I)

as well as their pharmaceutically acceptable salts, in which:

B represents a group -S-, -SO- or $-SO_2-$,

$R_1$ and $R_2$, which are identical or different, each represent hydrogen, the methyl or ethyl radical or a halogen,

A represents a linear or branched $C_2$-$C_5$ alkylene radical or the 2-hydroxypropylene radical in which the hydroxyl is optionally substituted with a $C_1$-$C_4$ alkyl radical,

Am represents a group:

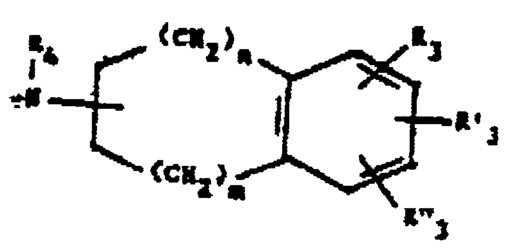

(D).

or

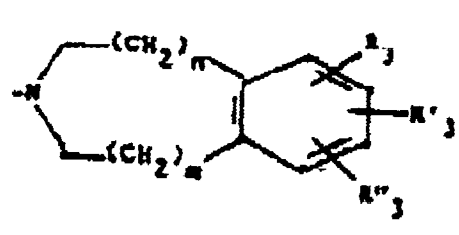

(E)

in which: $R_3$, $R'_3$ and $R''_3$, which are identical or different, each represent hydrogen, a halogen atom, a $C_1$-$C_4$ alkyl group or a $C_1$-$C_4$ alkoxy group,

$R_4$ represents hydrogen or a $C_1$-$C_8$ alkyl radical,

n and m, which may be identical or different, each representent 0, 1, 2 or 3,
Cy represents a group of formula:

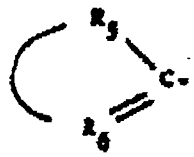

or

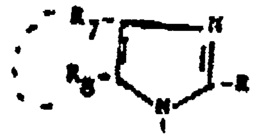

R represents hydrogen, a $C_1$-$C_8$ alkyl radical, a $C_3$-$C_6$ cycloalkyl radical, a benzyl radical or a phenyl radical which is optionally substituted with one or more substituents, which may be identical or different, chosen from halogen atoms or from $C_1$-$C_4$ alkyl, $C_1$-$C_4$ alkoxy or nitro groups,
$R_5$ and $R_6$ are taken together, with the carbon atom to which they are attached, to form:

- a mono- or dicyclic carbocyclic group which is optionally aromatic, having from 5 to 10 carbon atoms and optionally substituted with a group R in the α position with respect to the methyne group,
- a 5-membered heterocyclic group which is optionally aromatic, the heteroatoms or heterogroups being chosen from groups O, S, N,

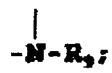

O and N; O and

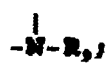

S and

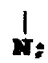

S and

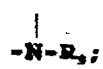

N and N; -N and

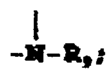

the heterocyclic group being optionally substituted with a group R as defined above in the α position with respect to the methyne group, and optionally substituted with one or two groups chosen from $C_1$-$C_4$ alkyl and phenyl groups,

- a mono- or dicyclic heterocyclic group having from 6 to 10 members and being optionally aromatic, the heteroatoms or heterogroups being chosen from groups O, S, N,

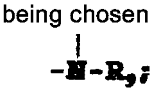

O and N; O and

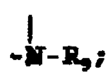

S and N; S and

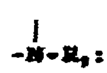

N and N; N and

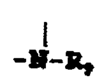

groups, the heterocyclic group being optionally substituted with a group R as defined above in the α position with respect to the methyne group,

$R_7$ and $R_8$, which are identical or different, each represent hydrogen, a $C_1$-$C_4$ lower alkyl radical or a phenyl radical, or when they are taken together, with the carbon atoms to which they are attached, represent a 6-membered carbocycle which is optionally aromatic,

$R_9$ represents hydrogen or a $C_1$-$C_4$ alkyl, phenyl, benzyl or halobenzyl group, characterized in that

A/ when A is as defined previously and B represents a group -S- or -$SO_2$-, a 4-alkoxyphenyl derivative of general formula:

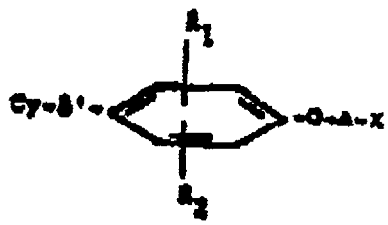

(2)

in which B represents a group -S- or -$SO_2$-: Cy, $R_1$ and $R_2$ have the same meanings as previously; A has the same meaning as above and X represents a halogen atom or an alkylsulphonyloxy group having 1 to 4 carbon atoms or an arylsulphonyloxy group having 6 to 10 carbon atoms is condensed, in the presence of an acid acceptor, in a polar or nonpolar solvent and at a temperature between room temperature and the reflux temperature, with an amine of general formula:

H - Am (3)

in which Am has the same meaning as previously,

- or a 4-hydroxyphenyl derivative of general formula:

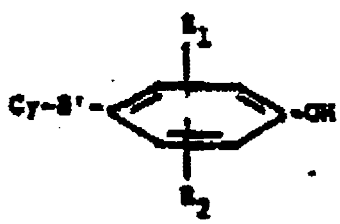

(4)

in which B' represents a group -S- or -$SO_2$- and Cy, $R_1$ and $R_2$ have the same meanings as previously is reacted, in the presence of a basic agent, with a compound of general formula

X - A - Am (52)

in which X represents a halogen atom or an alkylsulphonyloxy group having from 1 to 4 carbon atoms or an arylsulphonyloxy group having 6 to 10 carbon atoms, A is as defined above and Am has the same value as previously, the reaction taking place at reflux and in a suitable solvent;

B/ when A represents the 2-hydroxypropylene radical which may be optionally substituted and B represents a group -S- or -$SO_2$-, an oxiranylmethoxy derivative of general formula:

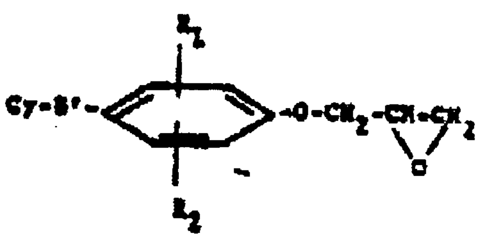

(57)

in which B' represents a group -S- or -$SO_2$- and Cy, $R_1$ and $R_2$ have the same meanings as previously, is treated at reflux with an amine of general formula:

H - Am (3)

in which Am has the same meaning as in previously, in a polar solvent or in an excess of the said amine in order to give:

- the desired derivative, in free base form, in which A represents a 2-hydroxypropylene radical
- an aminoalkoxyphenyl derivative which can be reacted with an alkyl halide having from 1 to 4 carbon

atoms and in the presence of a strong base, which gives the desired derivative in free base form, in which A represents a 2-hydroxypropylene radical in which the hydroxyl is substituted with an alkyl having from 1 to 4 carbon atoms, and

C/ when B represents a group -SO-, a sulphide of general formula:

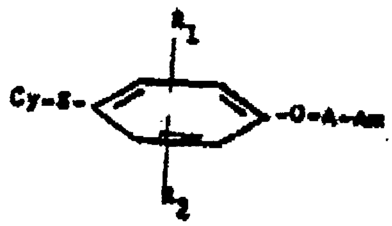

in which Cy, $R_1$, $R_2$, A and Am have the same meanings as previously, this sulphide being in free base or salt form, is treated with an oxidizing agent in order to form the desired derivative in free base or salt form, and the salt thus obtained is treated with a basic agent in order to give the desired derivative in free base form, the aminoalkoxyphenyl derivative thus obtained being, if so desired, reacted with an organic or inorganic acid in order to form a pharmaceutically acceptable salt of this derivative.

2. Process according to Claim 1, characterized in that the oxidizing agent is sodium periodate, potassium permanganate or 3-chloroperbenzoic acid.

3. Process according to Claim 1, characterized in that aminoalkoxyphenyl derivatives of general formula (1) are prepared in which Cy represents a phenyl, cyclohexenyl, indenyl, naphthyl, dihydronaphthyl, pyridyl, dihydropyridyl, furyl, dihydrofuryl, thienyl, dihydrothienyl, pyrrolyl, dihydropyrrolyl, pyrazolyl, imidazolyl, pyrimidyl, pyrazinyl, pyridazinyl, oxazolyl, isoxazolyl, thiazolyl, benzofuryl, benzothienyl, indolyl, benzimidazolyl, benzoxazolyl, quinolyl, benzisoxazolyl, cinnolinyl, quinoxalinyl, quinazolinyl, indolizinyl, thienopyridyl, tetrahydrothienopyridyl, pyrrolopyridyl, pyrazolopyridyl, pyrolopyridazinyl or imidazopyridyl group.

4. Process according to Claim 1, characterized in that aminoalkoxyphenyl derivatives of general formula (1) are prepared in which Cy represents an indolizinyl, benzofuryl, benzothienyl, indolyl, oxazolyl, pyrazolyl, phenyl, pyrazolo[1,5-a]pyridyl or imidazo[1,2-a]pyridyl group.

5. Process according to Claim 1, characterized in that aminoalkoxyphenyl derivatives of general formula (1) are prepared in which B represents a group $-SO_2-$.

6. Process according to Claim 1, characterized in that aminoalkoxyphenyl derivatives of general formula (1) are prepared in which $R_1$ and $R_2$ each represent hydrogen.

7. Process according to Claim 1, characterized in that aminoalkoxyphenyl derivatives of general formula (1) are prepared in which $R_3$, $R'_3$ and $R''_3$ represent hydrogen or methoxy.

8. Process according to Claim 1, characterized in that aminoalkoxyphenyl derivatives of general formula (1) are prepared in which R represents the isopropyl or cyclopropyl group.

9. Process according to Claim 1, characterized in that aminoalkoxyphenyl derivatives of general formula (1) are prepared in which the pharmaceutically acceptable salt is the oxalate or the hydrochloride.

10. Process according to Claim 1, characterized in that aminoalkoxylphenyl derivatives are prepared which are chosen from:
1-{4-[3-(6,7-dimethoxy-1,2,3,4-tetrahydro-2-N-isoquinolyl)propoxy]benzenesulphonyl}-2-isopropylindolizine,
2-ethyl-3-{4-[3-(6,7-dimethoxy-1,2,3,4-tetrahydro-2-N-isoquinolyl)propoxy]benzenesulphonyl}benzothiophene,
4-{4-[3-(6,7-dimethoxy-1,2,3,4-tetrahydro-2-N-isoquinolyl)propoxy]benzenesulphonyl}-5-isopropyloxazole,
4-{4-[3-(6,7-dimethoxy-1,2,3,4-tetrahydro-2-N-isoquinolyl)propoxy]benzenesulphonyl}-5-ethyl-1-methylpyrazole,
4-{4-[3-(6,7-dimethoxy-1,2,3,4-tetrahydro-2-N-isoquinolyl)propoxy]benzenesulphonyl}-5-isopropyl-1-methylpyrazole,

3-{4-[3-(6,7-dimethoxy-1,2,3,4-tetrahydro-2-N-isoquinolyl)propoxy]benzenesulphonyl}-2-isopropyl-1-methylindole,
1-{4-[4-(6,7-dimethoxy-1,2,3,4-tetrahydro-2-N-isoquinolyl)butoxy]benzenesulphonyl}-2-isopropylindolizine,
2-isopropyl-4-[3-(6,7-dimethoxy-1,2,3,4-tetrahydro-2-N-isoquinolyl)propoxy]-3-benzenesulphonylbenzofuran,
2-ethyl-4-[3-(6,7-dimethoxy-1,2,3,4-tetrahydro-2-N-isoquinolyl)propoxy]-3-benzenesulphonylbenzofuran,
4-[2-(6,7-dimethoxy-1,2,3,4-tetrahydro-N-isoquinolyl)propoxy]phenyl (2-isopropylphenyl) sulphone,
4-[N-(5,6,7,8,9-pentahydro-9-benzocycloheptenyl)-3-N-methylaminopropoxy]-3-benzenesulphonyl-2-isopropyl-1-methylindole,
4-[N-methyl-3-N-(1,2,3,4-tetrahydro-1-naphthyl)aminopropoxy]-3-benzenesulphonyl-2-isopropyl-1-methylindole,
4-[N-methyl-3-N-(6,7-dimethoxy-1,2,3,4-tetrahydro-1-naphthyl)aminopropoxy]-1-benzenesulphonyl-2-isopropylindolizine,
4-[N-methyl-3-N-(1,2,3,4-tetrahydro-1-naphthyl)aminopropoxy]-3-benzenesulphonyl-2-isopropylpyrazolo[1,5-a]-pyridine
and their pharmaceutically acceptable salts.

**11.** Process for the preparation of a pharmaceutical or veterinary composition, as active principle, at least one aminoalkoxyphenyl derivative prepared according to one of Claims 1 to 10 is added to a suitable pharmaceutical vehicle or excipient.

**12.** Process for the preparation of a pharmaceutical or veterinary composition according to Claim 11 for the treatment of pathological syndromes of the cardiovascular system, containing from 50 mg to 500 mg of active principle.

**13.** Process for the preparation of a pharmaceutical or veterinary composition according to Claim 11 for the treatment of ocular pathological complaints.

**Claims for the following Contracting State : GR**

**1.** Aminoalkoxyphenyl derivatives corresponding to the general formula:

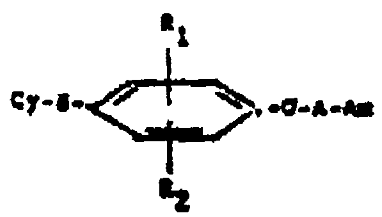

(I)

as well as their pharmaceutically acceptable salts, in which:

B represents a group -S-, -SO- or -$SO_2$-,

$R_1$ and $R_2$, which are identical or different, each represent hydrogen, the methyl or ethyl radical or a halogen,

A represents a linear or branched $C_2$-$C_5$ alkylene radical or the 2-hydroxypropylene radical in which the hydroxyl is optionally substituted with a $C_1$-$C_4$ alkyl radical,

Am represents a group:

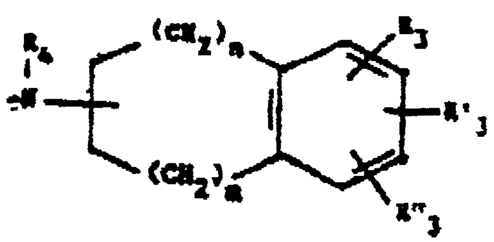

(D),

or

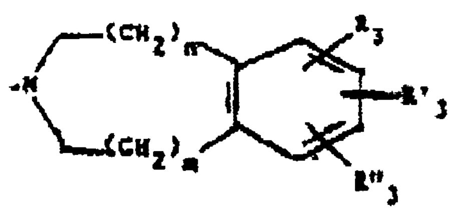

(E)

in which: $R_3$, $R'_3$ and $R''_3$, which are identical or different, each represent hydrogen, a halogen atom, a $C_1$-$C_4$ alkyl group or a $C_1$-$C_4$ alkoxy group,

$R_4$ represents hydrogen or a $C_1$-$C_8$ alkyl radical,

n and m, which may be identical or different, each representent 0, 1, 2 or 3,

Cy represents a group of formula:

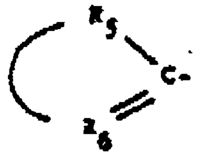

or

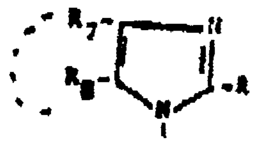

R represents hydrogen, a $C_1$-$C_8$ alkyl radical, a $C_3$-$C_6$ cycloalkyl radical, a benzyl radical or a phenyl radical which is optionally substituted with one or more substituents, which may be identical or different, chosen from halogen atoms or from $C_1$-$C_4$ alkyl, $C_1$-$C_4$ alkoxy or nitro groups,

$R_5$ and $R_6$ are taken together, with the carbon atom to which they are attached, to form:

- a mono- or dicyclic carbocyclic group which is optionally aromatic, having from 5 to 10 carbon atoms and optionally substituted with a group R in the $\alpha$ position with respect to the methyne group,
- a 5-membered heterocyclic group which is optionally aromatic, the heteroatoms or heterogroups being chosen from groups O, S, N,

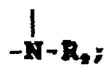

O and N; O and

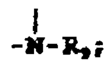

S and

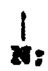

S and

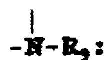

N and N: N and

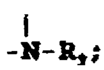

the heterocyclic group being optionally substituted with a group R as defined above in the $\alpha$ position with respect to the methyne group, and optionally substituted with one or two groups chosen from $C_1$-$C_4$ alkyl and phenyl groups,

- a mono- or dicyclic heterocyclic group having from 6 to 10 members and being optionally aromatic, the heteroatoms or heterogroups being chosen from groups O, S, N,

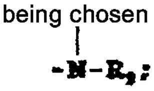

O and N; O and

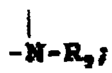

S and N: S and

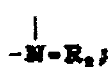

N and N; N and

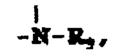

the heterocyclic group being optionally substituted with a group R as defined above in the α position with respect to the methyne group,

$R_7$ and $R_8$, which are identical or different, each represent hydrogen, a $C_1$-$C_4$ lower alkyl radical or a phenyl radical, or when they are taken together, with the carbon atoms to which they are attached, represent a 6-membered carbocycle which is optionally aromatic,

$R_9$ represents hydrogen or a $C_1$-$C_4$ alkyl, phenyl, benzyl or halobenzyl group.

**2.** Aminoalkoxyphenyl derivatives according to Claim 1, in which Cy represents a phenyl, cyclohexenyl, indenyl, naphthyl, dihydronaphthyl, pyridyl, dihydropyridyl, furyl, dihydrofuryl, thienyl, dihydrothienyl, pyrrolyl, dihydropyrrolyl, pyrazolyl, imidazolyl, pyrimidyl, pyrazinyl, pyridazinyl, oxazolyl, isoxazolyl, thiazolyl, benzofuryl, benzothienyl, indolyl, benzimidazolyl, benzoxazolyl, quinolyl, benzisoxazolyl, cinnolinyl, quinoxalinyl, quinazolinyl, indolizinyl, thienopyridyl, tetrahydrothienopyridyl, pyrrolopyridyl, pyrazolopyridyl, pyrolopyridazinyl or imidazopyridyl, group.

**3.** Aminoalkoxyphenyl derivatives according to Claim 1, in which Cy represents an indolizinyl, benzofuryl, benzothienyl, indolyl, oxazolyl, pyrazolyl, phenyl, pyrazolo[1,5-a]pyridyl or imidazo[1,2-a]pyridyl group.

**4.** Aminoalkoxyphenyl derivatives according to Claim 1, in wich B represents a group $-SO_2-$.

**5.** Aminoalkoxyphenyl derivatives according to Claim 1, in which $R_1$ and $R_2$ each represent hydrogen.

**6.** Aminoalkoxyphenyl derivatives according to Claim 1, in which $R_3$, $R'_3$ and $R''_3$ representent hydrogen or methoxy.

**7.** Aminoalkoxyphenyl derivatives according to Claim 1, in which R represents the isopropyl or cyclopropyl group.

**8.** Aminoalkoxyphenyl derivatives according to Claim 1, in which the pharmaceutically acceptable salt is the oxalate or the hydrochloride.

**9.** Aminoalkoxyphenyl derivatives according to Claim 1, chosen from:
1-{4-[3-(6,7-dimethoxy-1,2,3,4-tetrahydro-2-N-isoquinolyl)propoxyl]benzenesulphonyl}-2-isopropylindolizine,
2-ethyl-3-{4-[3-(6,7-dimethoxy-1,2,3,4-tetrahydro-2-N-isoquinolyl)propoxy]benzenesulphonyl}benzothiophene,
4-{4-[3-(6,7-dimethoxy-1,2,3,4-tetrahydro-2-N-isoquinolyl)propoxy]benzenesulphonyl}-5-isopropyloxazole,
4-{4-[3-(6,7-dimethoxy-1,2,3,4-tetrahydro-2-N-isoquinolyl)propoxy]benzenesulphonyl}-5-ethyl-1-methylpyrazole,
4-{4-[3-(6,7-dimethoxy-1,2,3,4-tetrahydro-2-N-isoquinolyl)propoxy]benzenesulphonyl}-5-isopropyl-1-methylpyrazole,
3-{4-[3-(6,7-dimethoxy-1,2,3,4-tetrahydro-2-N-isoquinolyl)propoxy]benzenesulphonyl}-2-isopropyl-1-methylindole,
1-{4-[4-(6,7-dimethoxy-1,2,3,4-tetrahydro-2-N-isoquinolyl)butoxy]benzenesulphonyl}-2-isopropylindolizine,
2-isopropyl-4-[3-(6,7-dimethoxy-1,2,3,4-tetrahydro-2-N-isoquinolyl)propoxy]-3-benzenesulphonylbenzofuran,
2-ethyl-4-[3-(6,7-dimethoxy-1,2,3,4-tetrahydro-2-N-isoquinolyl)propoxy]-3-benzenesulphonylbenzofuran,
4-[2-(6,7-dimethoxy-1,2,3,4-tetrahydro-N-isoquinolyl)propoxy]phenyl (2-isopropylphenyl) sulphone,
4-[N-(5,6,7,8,9-pentahydro-9-benzocycloheptenyl)-3-N-methylaminopropoxy]-3-benzenesulphonyl-2-isopropyl-1-methylindole,
4-[N-methyl-3-N-(1,2,3,4-tetrahydro-1-naphthyl)aminopropoxy]-3-benzenesulphonyl-2-isopropyl-1-methylindole,
4-[N-methyl-3-N-(6,7-dimethoxy-1,2,3,4-tetrahydro-1-naphthyl)aminopropoxy]-1-benzenesulphonyl-2-isopropylindolizine,
4-[N-methyl-3-N-(1,2,3,4-tetrahydro-1-naphthyl)aminopropoxy]-3-benzenesulphonyl-2-isopropylpyrazolo[1,5-a]-pyridine
and their pharmaceutically acceptable salts.

**10.** Process for the preparation of aminoalkoxyphenyl derivatives according to Claim 1 in which A is as defined

in Claim 1 and B represents a group -S- or $-SO_2-$, characterized in that a 4-alkoxyphenyl derivative of general formula:

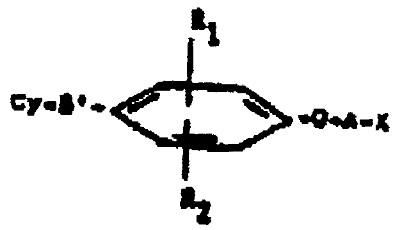

(2)

in which B represents a group -S- or $-SO_2-$: Cy, $R_1$ and $R_2$ have the same meanings as in Claim 1; A has the same meaning as above and X represents a halogen atom or an alkylsulphonyloxy group having 1 to 4 carbon atoms or an arylsulphonyloxy group having 6 to 10 carbon atoms is condensed, in the presence of an acid acceptor, in a polar or nonpolar solvent and at a temperature between room temperature and the reflux temperature, with an amine of general formula:

H - Am (3)

in which Am has the same meaning as in Claim 1, to form the desired derivative which is reacted, if so desired, with an organic or inorganic acid to form a pharmaceutically acceptable salt of this derivative.

**11.** Process for the preparation of aminoalkoxyphenyl derivatives according to Claim 1, in which A is as defined in Claim 1 and B represents a group -S- or $-SO_2-$, characterized in that a 4-hydroxyphenyl derivative of general formula:

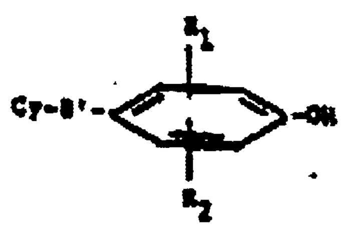

(4)

in which B' represents a group -S- or $-SO_2-$ and Cy, $R_1$ and $R_2$ have the same meanings as in Claim 1 is reacted, in the presence of a basic agent, with a compound of general formula

X - A - Am (52)

in which X represents a halogen atom or an alkylsulphonyloxy group having from 1 to 4 carbon atoms or an arylsulphonyloxy group having 6 to 10 carbon atoms, A is as defined above and Am has the same value as in Claim 1, the reaction taking place at reflux and in a suitable solvent to obtain the desired derivative, which it is possible, if no desired, to react with a suitable organic or inorganic acid in order to form a pharmaceutically acceptable salt.

**12.** Process for the preparation of aminoalkoxyphenyl derivatives according to Claim 1, in which A represents the 2-hydroxypropylene radical which is optionally substituted and B represents a group -S- or $-SO_2-$, characterized in that an oxiranylmethoxy derivative of general formula:

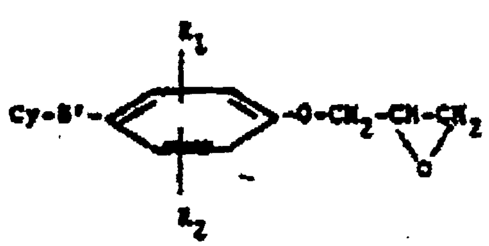

(57)

in which B' represents a group -S- or $-SO_2-$ and Cy, $R_1$ and $R_2$ have the same meanings as in Claim 1, is treated at reflux with an amine of general formula:

H - Am (3)

in which Am has the same meaning as in Claim 1, in a polar solvent or in an excess of the said amine in order to give:

- the desired derivative, in free base form, in which A represents a 2-hydroxypropylene radical
- an aminoalkoxyphenyl derivative which can be reacted with an alkyl halide having from 1 to 4 carbon atoms and in the presence of a strong base, which gives the desired derivative in free base form, in which A represents a 2-hydroxypropylene radical in which the hydroxyl is substituted with an alkyl having from 1 to 4 carbon atoms,

  the aminoalkoxyphenyl derivative thus obtained being able, if so desired, to be reacted with an organic or inorganic acid in order to form a pharmaceutically acceptable salt.

**13.** Process for the preparation of aminoalkoxyphenyl derivatives according to claim 1, in which B represents a group -SO-, characterized in that a sulphide of general formula:

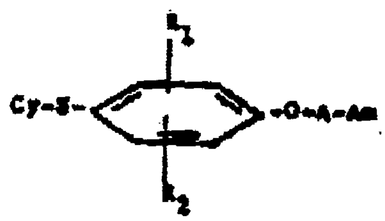

in which Cy, $R_1$, $R_2$, A and Am have the same meanings as in Claim 1, this sulphide being in free base or salt form, is treated with an oxidizing agent in order to form the desired derivative in free base or salt form, and in that the salt thus obtained is treated with a basic agent in order to give the desired derivative in free base form, the free base thus obtained being, if so desired, reacted with an organic or inorganic acid in order to form a pharmaceutically acceptable salt of this derivative.

**14.** Process according to Claim 13, characterized in that the oxidizing agent is sodium periodate, potassium permanganate or 3-chloroperbenzoic acid.

**15.** Process for the preparation of pharmaceutical or veterinary compositions, characterized in that at least one aminoalkoxyphenyl derivative according to one of Claims 1 to 9 is admixed with a suitable pharmaceutical vehicle or excipient.

**16.** Process for the preparation of pharmaceutical or veterinary compositions according to Claim 15, for the treatment of pathological syndromes of the cardiovascular system, containing from 50 mg to 500 mg of active principle.

**17.** Process for the preparation of pharmaceutical or veterinary compositions according to Claim 15 for the production of a medicament intended for the treatment of ocular pathological complaints.